# EUROPEAN PATENT APPLICATION

(11) **EP 4 516 790 A1**
(43) Date of publication of application: **05.03.2025**
(21) Application number: 24196992.2
(22) Date of filing: 28.08.2024
(51) Int. Cl.: C07D 405/14, A61P 35/00, A61K 31/4155

(54) **COVALENT TARGETING OF RHOA FOR CANCER THERAPY**

(30) Priority: 28.08.2023 US 202363579246 P
(71) Applicant: The University Of Hong Kong, Pokfulam, Hong Kong (CN); Centre for Oncology and Immunology Limited, New Territories, Hong Kong (HK)
(72) Inventor: CHUNG, Yik Sham Clive, Hong Kong (CN); LI, Ying Ki Jason, Hong Kong (CN); KOO, Tin Yan, Hong Kong (CN); YAN, Hoi Ning Helen, Hong Kong (CN)
(74) Representative: HGF

(57) **Abstract**

RhoA inhibitors that can covalently bind RhoA are described herein. The disclosed RhoA inhibitors show improved inhibitory efficacy (i.e., an IC₅₀ value down to about 400 nM). Further, the disclosed RhoA inhibitors can specifically bind RhoA over other GTPases, such as other Ras GTPases (e.g., K-Ras and N-Ras) and particularly other Rho GTPases (e.g., Cdc42 and Rac1). The combination of high RhoA inhibitory efficacy and high RhoA specificity of the disclosed RhoA inhibitors allow these compounds to show high cytotoxicity and anti-metastatic effects in cancer cells, with negligible toxicity in normal cells, in *vitro* and in *vivo.* Pharmaceutical compositions containing the RhoA inhibitors and methods of using the RhoA inhibitors or pharmaceutical composition thereof for treating cancer are also described.

## Description

### CROSS REFERENCE TO RELATED APPLICATION

This application claims priority to U.S. Provisional Patent Application No. 63/579,246 filed August 28, 2023, the disclosure of which is incorporated herein by reference.

### FIELD OF THE INVENTION

This invention is generally in the field of synthetic RhoA inhibitors and methods of use thereof.

### BACKGROUND OF THE INVENTION

Upregulation of RhoA has been reported in a number of malignancies, including melanoma, gastric cancer, breast cancer, and colorectal cancer (CRC), most of which are known to have poor prognosis and aggressive infiltration. RhoA plays a role in governing cellular structure by interplaying with downstream effectors like mDia1 and ROCK. It has been reported that RhoA contributes to both amoeboid and mesenchymal mobility in 3D migration and invasion, indicating its importance in cancers to infiltrate nearby tissues and escaping anoikis using the Rho/ROCK pathway. All of these promote cancer aggressiveness. Furthermore, RhoA is reported to be hyperactive in colorectal cancer and regulates cell proliferation, invasion and cell cycle, particularly G1/S transition. As a result, RhoA inhibition should be a promising strategy for cancer therapy.

Despite the therapeutic interest RhoA possess, RhoA was thought to be undruggable due to its smooth surface and globular structure, making it difficult for small molecules to bind. Alternatively, attempts were made to circumvent RhoA but target its downstream proteins, for example ROCK inhibitors Y27632 and Fasudil, with the latter approved for treating post-surgery cerebral vasospasm in Japan. Yet, ROCK inhibitors cannot completely abolish oncogenic signals from RhoA, such as RhoA-mDia1 that mediates chromosomal instability and hence drives tumor development. Therefore, the interest in drugging RhoA for cancer therapy persists.

In 2014, Shang et al screened a small molecule named Rhosin by performing *in vitro* docking. It possesses two aromatic ring moieties which are believed to sit in a shallow groove on RhoA surface. Rhosin was selected for its ability to impair RhoA binding with its guanine nucleotide exchange factor (GEF), LARG, by targeting a GEF recognition site around a critical residue Trp58, thus interfering with GEF-mediated GDP-to-GTP exchange and rendering RhoA inactive. Despite some studies reported anticancer effects of Rhosin *in vivo,* up to now, Rhosin does not proceed beyond animal studies into clinical trial, partly because of its low anticancer efficacy with working concentration of ≥30 µM and suboptimal pharmacokinetic profiles.

In contrast to non-covalent drug lead like Rhosin, covalent ligands can provide an alternative solution without the reliance of high affinity scaffold for shallow binding pockets. Recently, Sun et al reported a covalent ligand, known as DC-Rhoin, that targets C107 of RhoA. They proposed that C107 has a nearby shallow groove which can act as an allosteric site for interfering RhoA-LARG interaction. Moreover, C107 is unique in Rho family proteins but not in other Ras GTPases thus can increase the drug specificity. Therefore, DC-Rhoin targets Rho GTPases, such as RhoA, Cdc42 and Rac1, but not Ras GTPases like K-Ras and N-Ras at its working concentration. However, Rac1 and Cdc42 play important roles in governing essential cellular processes, such as formation of lamellipodia and filopodia. As a result, the alteration ofRac1 and Cdc42 signals by DC-Rhoin leads to toxicity in cancer therapy.

There remains a need to develop compounds that bind RhoA with high efficacy and high specificity over other GTPases, such as Rac1 and Cdc42.

Therefore, it is the object of the present invention to provide compounds that bind RhoA with high specificity.

It is a further object of the present invention to provide compounds that bind and inhibit RhoA with high efficacy.

It is a further object of the present invention to provide compounds for cancer therapy with low toxicity.

It is a further object of the present invention to provide methods of using the compounds for cancer therapy.

### SUMMARY OF THE INVENTION

RhoA inhibitors (also referred to herein as "RhoA covalent inhibitors" or "compounds") are described herein. The disclosed RhoA inhibitors can covalently bind RhoA, and thereby achieve improved inhibitory efficacy (i.e., an IC₅₀ value down to about 400 nM). Further, the disclosed RhoA inhibitors can specifically bind RhoA over other GTPases, such as other Ras GTPases (e.g., K-Ras and N-Ras) and particularly other Rho GTPases (e.g., Cdc42 and Rac1). The combination of high RhoA inhibitory efficacy and high RhoA specificity of the disclosed RhoA inhibitors allow these compounds to show high cytotoxicity in cancer cells, with negligible toxicity in normal cells, in *vitro* and in *vivo.* For example, as demonstrated by the data in the Examples below, exemplary compound CL16 showed high cytotoxicity in CRC cells, with correlation with the RhoA expression/activity in the cancer cells, while it is much less toxic for normal colon fibroblast which has lower RhoA level. Further, CL16 administered in tumor-bearing mice inhibited tumor growth *in vivo,* with no observable toxicity was found from mice treated with the exemplary compound CL16, as shown by the body weight measurement and immunohistochemistry on the tissue sections of different organs. The superior efficacy and specificity of the disclosed RhoA inhibitors also allow them to outperform the reported RhoA inhibitor, Rhosin. For example, as demonstrated by the data in the Examples below, exemplary compound CL16 inhibited the growth of CRC spheroids, with better potency than the reported RhoA inhibitor, Rhosin. Further, since the binding site of DC-Rhoin, i.e., Cys107, is conserved in Rac1 and Cdc42, DC-Rhoin alters Rac1 and Cdc42 signals, which leads to cytotoxicity.

Without being bound to any theories, it is believed that the superior efficacy and specificity of the disclosed RhoA inhibitors arises from covalent binding of these compounds onto Cys16, which locates near the nucleotide-binding pocket of RhoA. Upon binding, the disclosed RhoA inhibitors resided next to the nucleotide-binding pocket in RhoA, which results in significant decreases in the GDP-to-GTP self-exchange and the LARG-mediated exchange, and thereby leads to their high inhibitory efficacy. Further, the binding site of the disclosed RhoA inhibitors, i.e., Cys16, is unique to RhoA compared to other GTPases (such as Rac1 and Cdc42), and thereby leads to their high binding specificity.

In some forms, the disclosed RhoA inhibitors can have the structure of Formula I: 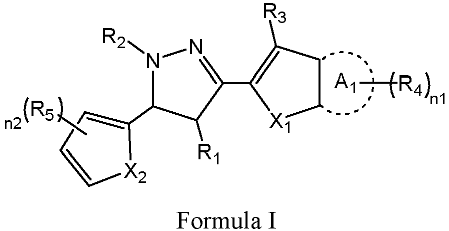 wherein: (i) X₁ and X₂ can be independently C(R₆)₂, O, S, or NR₆ and R₆ is hydrogen, a substituted or unsubstituted alkyl, a substituted or unsubstituted alkenyl, a substituted or unsubstituted alkynyl, an alkoxyl, or a carbonyl; (ii) A₁ can be a C₅-C₆ aromatic ring or a C₄-C₅ heteroaromatic ring; (iii) n1 can be an integer from 0 to 4, from 0 to 3, from 0 to 2, such as 0, 1, or 2; (iv) n2 can be an integer from 0 to 3, from 0 to 2, or 0 or 1; (v) R₁, R₃, and R₅ can be independently hydrogen, a substituted or unsubstituted alkyl, or a substituted or unsubstituted aryl; (vi) R₂ is a cysteine-reactive group (such as haloacetamide, acrylamide, maleimide, vinyl sulfone, epoxide, etc.), which allows chemical reaction with cysteines on RhoA; (vii) R₄ can be hydrogen, an alkoxyl, a hydroxyl, a halogen, a substituted or unsubstituted alkynyl, a substituted or unsubstituted aryl, a carbonyl, an amino, or a haloalkyl; and (viii) the substituents, when present, can be independently unsubstituted alkyl, unsubstituted aryl, unsubstituted heteroaryl, unsubstituted polyaryl, unsubstituted heteropolyaryl, unsubstituted alkylaryl, unsubstituted cyclic, unsubstituted heterocyclic, halide, amino, amido, thiol, hydroxyl, cyano, nitro, carbonyl, or alkoxyl.

In some forms, the disclosed RhoA inhibitors can have the structure of Formula II: 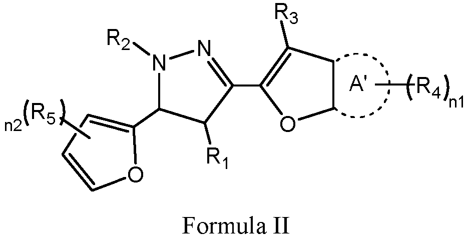 wherein: A', n1, n2, and R₁-R₅ can be as defined above for Formula I.

In some forms, the disclosed RhoA inhibitors can have the structure of Formula III: 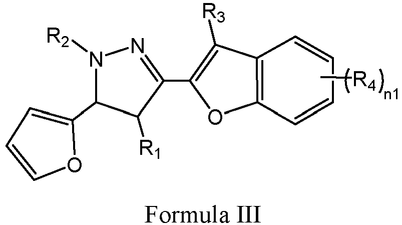 wherein: n1 and R₁-R₄ can be as defined above for Formula I.

In some forms, for any of the formulae described herein, n1 can be 0 or 1.

In some forms, for any of the formulae described herein, R₁ can be hydrogen.

In some forms, for any of the formulae described herein, R₃ can be hydrogen or a substituted or unsubstituted aryl, wherein the substituents, when present, are independently an unsubstituted alkyl, an unsubstituted aryl, or an unsubstituted alkyl aryl.

In some forms, for any of the formulae described herein, R₂ can be 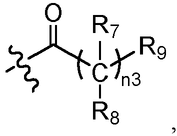 where n3 can be an integer from 0 to 6, from 0 to 5, from 0 to 4, from 0 to 3, from 0 to 2, such as 0, 1, or 2; R₇ and R₈ can be independently hydrogen, an unsubstituted alkyl, or halogen; and R₉ can be hydrogen or halogen.

In some forms, for any of the formulae described herein, R₄ can be hydrogen.

In some forms, for any of the formulae described herein, R₄ can be 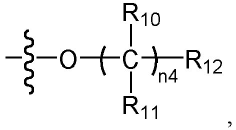 where n4 can be an integer from 0 to 6, from 0 to 5, from 0 to 4, from 0 to 3, from 0 to 2, such as 0, 1, or 2; R₁₀ and R₁₁ can be independently hydrogen or halogen; and R₁₂ can be hydrogen, halogen, amino, an unsubstituted alkynyl, aldehyde, or carboxylic acid. In some forms, R₁₀ and R₁₁ can be hydrogen, and R₁₂ can be an unsubstituted alkynyl. In these forms, R₄ can function as a probe that allows further functionalization with and/or binding to a target, such as detection moieties (e.g., a fluorophore), for biological study (e.g., RhoA inhibitor binding and function *in vitro* and/or *in vivo*). An exemplary compound is CL-alkyne shown below.

In some forms, the RhoA inhibitor is CL16 or CL16-alkynyl.

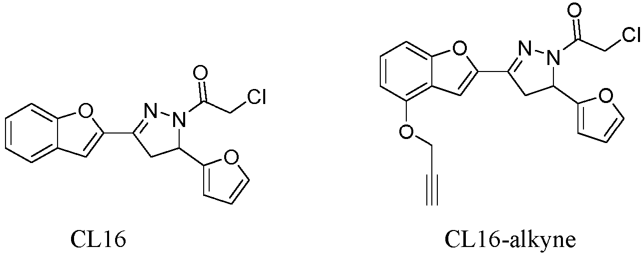

Pharmaceutical compositions containing one or more of the RhoA inhibitors and optionally one or more pharmaceutically acceptable excipients are disclosed. The pharmaceutical compositions can be formulated for administration via a variety of routes, such as oral administration, intranasal administration, intramuscular administration, intravenous administration, intraperitoneal administration, or subcutaneous administration, or a combination thereof. Optionally, the pharmaceutical composition further contains one or more additional active agent(s), such as one or more additional anticancer agent(s).

Methods of using the RhoA inhibitors or pharmaceutical compositions containing the RhoA inhibitors for treating cancer are also disclosed. Generally, the method for treating cancer includes a step of administering the RhoA inhibitors or pharmaceutical composition thereof to a subject in need thereof. In some forms, the cancer is a solid cancer. Typically, following the administration step, an effective amount of the RhoA inhibitors is administered to the subject, such that cancer cells are killed and/or growth or proliferation of the cancer cells are reduced or prevented, and thereby ameliorate one or more symptoms associated with the cancer in the subject, such as reduce the tumor volume and/or tumor weight. For example, following the administration step or all of the administration steps (if the administration occurs more than one time), an effective amount of the RhoA inhibitors is administered to the subject, such that the tumor volume and/or tumor weight in the subject is at least 10%, at least 20%, at least 30%, at least 40%, or at least 50% smaller/less than a control that is administered with the pharmaceutically acceptable excipient(s) only. Optionally, the anticancer effect is achieved without observable toxicity to the subject, as indicated by body vital measurements (e.g., body weight), standard hematology markers, and/or blood biochemical parameters compared to the control. In some forms, the effective amount of RhoA inhibitors administered to the subject is from about 0.1 µg to about 1000 µg, from about 0.1 µg to about 500 µg, from about 0.1 µg to about 200 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject. Optionally, the method further includes administering an additional active agent prior to, during, and/or subsequent to the administration of the pharmaceutical composition containing the RhoA inhibitors, optionally wherein the active agent is an anticancer agent.

### BRIEF DESCRIPTION OF THE DRAWINGS

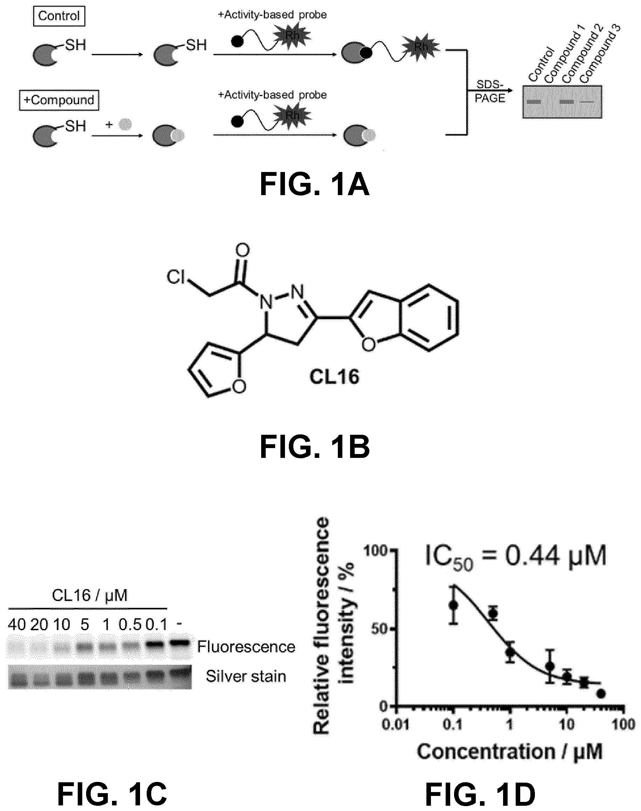

**Figure 1A** is a schematic illustrating the process of gel-based ABPP experiment. **Figure 1B** shows the chemical structure of compounds CL16. **Figure 1C** and **Figure 1D** show the gel results from dose-dependent experiments **(****Figure 1C****)** showing strong *in vitro* binding of CL16 onto RhoA, and a relative fluorescence intensity-concentration plot showing the IC₅₀ of the competitive binding at 0.44 µM **(****Figure 1D****).**

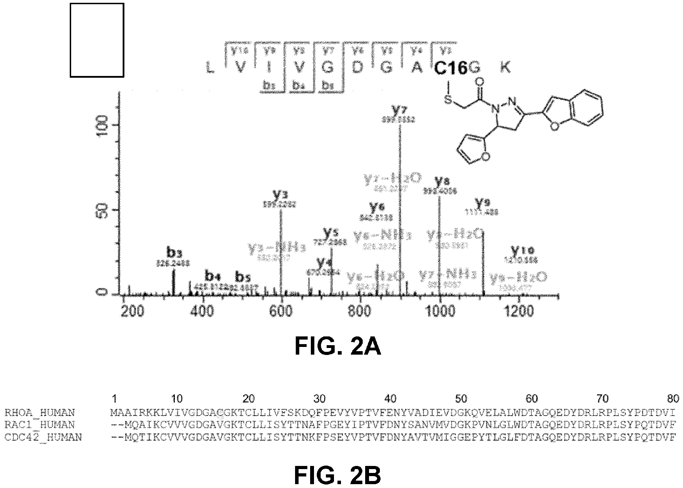

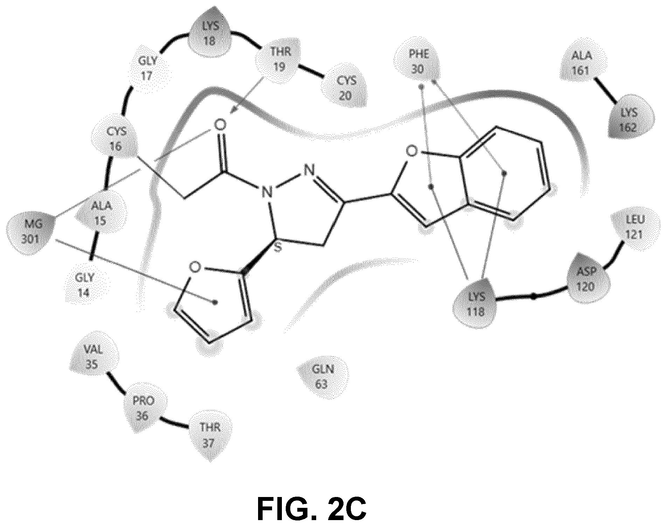

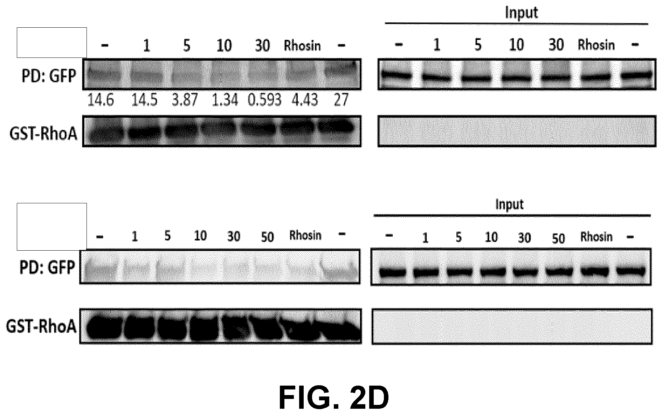

**Figure 2A** shows the results of LC-MS/MS experiment on HCT116 cells treated with CL16, revealing the covalent binding of CL16 onto Cys16 of RhoA. **Figure 2B** shows the amino acid sequence alignment of human RHOA, RAC1 and CDC42. Cys16 is unique for RhoA as compared to RAC1 and CDC42. **Figure 2C** shows the covalent docking of CL16 onto RhoA (PDB: 1CC0). CL16 resided next to the nucleotide-binding pocket of RhoA through covalent targeting of Cys16. The ligand interaction map indicated interactions of CL16 with the amino acid residues and the Mg²⁺ cofactor ion. **Figure 2D** shows the results from co-immunoprecipitation experiments, showing the disruption of RhoA and ARHGEF1/LARG interactions upon incubation with CL16.

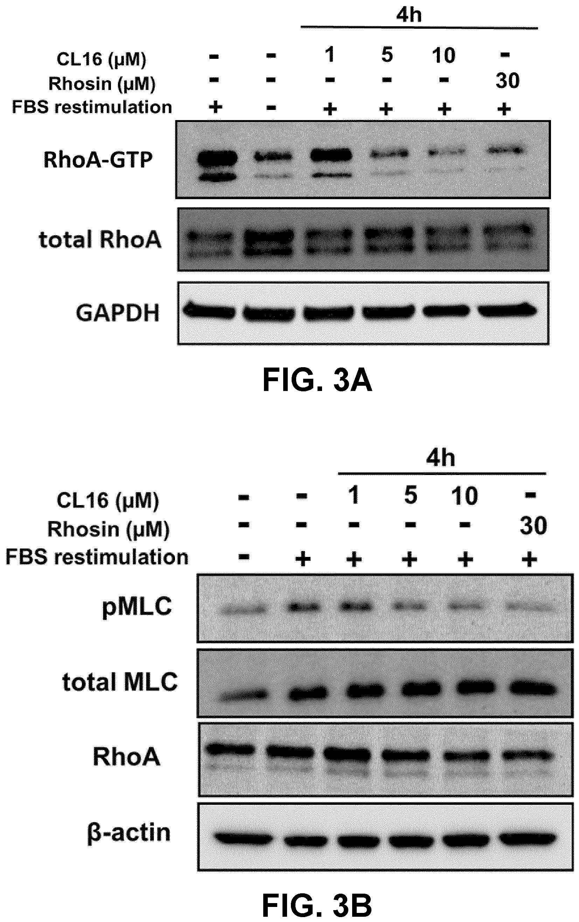

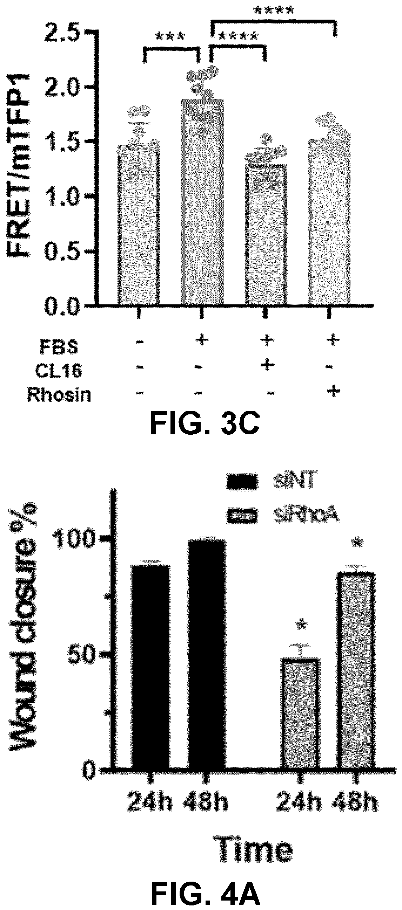

**Figure 3A** shows the western blotting results, showing the prohibition of RhoA activation in HT29 cells in the serum starvation-restimulation experiment upon incubation with CL16. **Figure 3B** shows the results from western blotting. An inhibition of RhoA downstream signal by CL16 was found in HT29 cells in serum starvation-restimulation experiment, as revealed by a decrease in phosphorylation levels of MLC. **Figure 3C** is a graph showing prohibition of RhoA activity by CL16 in live cells with serum restimulation, as demonstrated by FRET-RhoA biosensor. Quantified data were shown in average±SD from *n* = 25 cells from 3 different biological replicates/group. ****P*<0.001 and *****P*<0.0001.

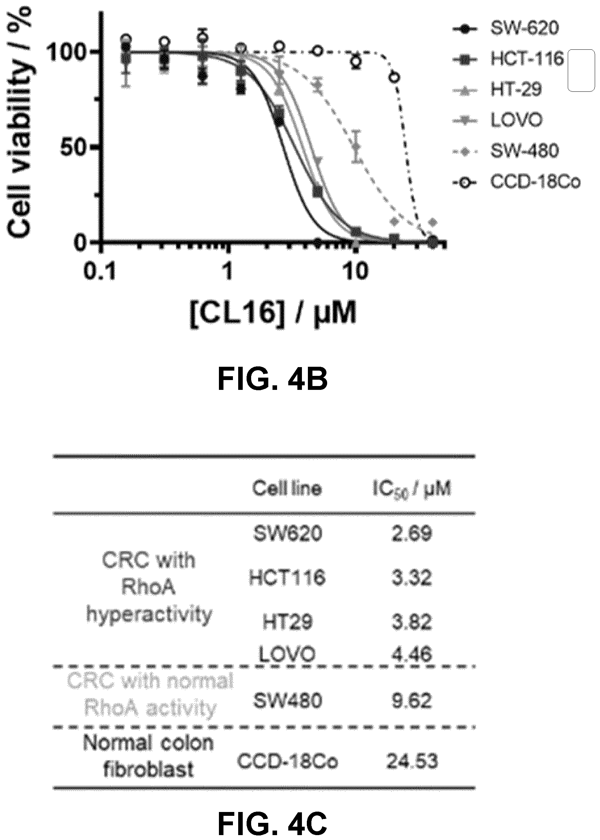

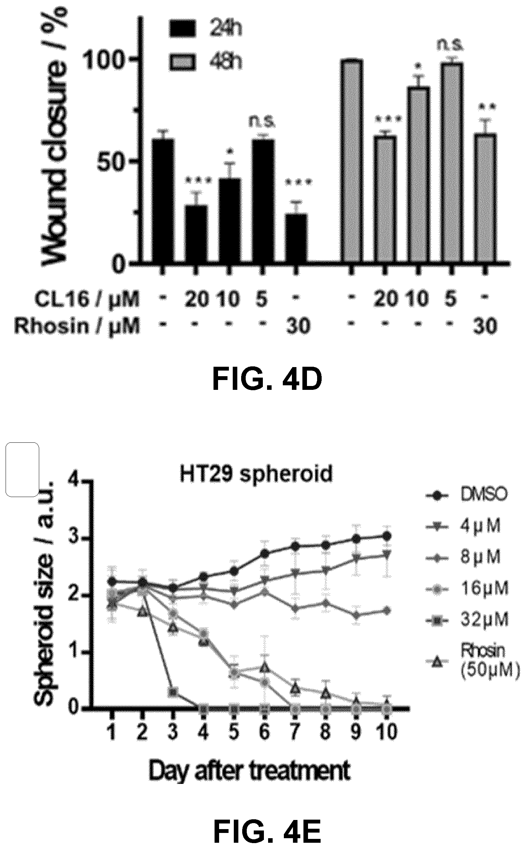

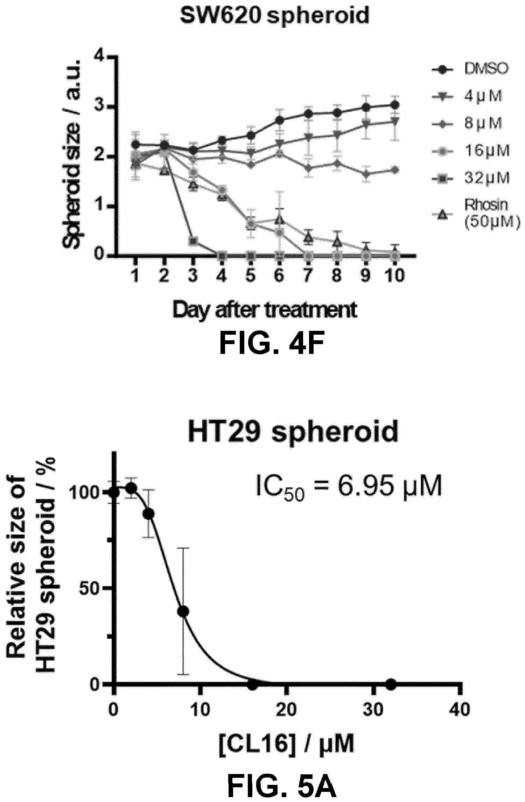

**Figure 4A** is a bar graph showing decreases in colony formation and cell migration of HT29 cells with genetic knockdown of RhoA by siRNA, using wound healing assay. **Figure 4B** is a graph showing cell viability of CRCs and normal colon fibroblast CCD-18Co cells treated with CL16 for 48 h using MTT assay; *n* = 3 different biological replicates/group. **Figure 4C** shows a Table presenting the IC₅₀ values of CL16 against different cell lines. **Figure 4D** is a bar graph showing wound healing assay on HT29 cells incubated with solvent vehicle, CL 16 or Rhosin; *n* = 3 different biological replicates/group. **Figures 4E-4F** are graphs showing the inhibition of HT29 **(****Figure 4E****)** and SW620 **(****Figure 4F****)** spheroid growth, by the treatment with solvent vehicle, CL16, or Rhosin; *n* = 3 different biological replicates/group. Quantified data were shown in average±SD. **P*<0.05, ***P*<0.01 and ****P*<0.001.

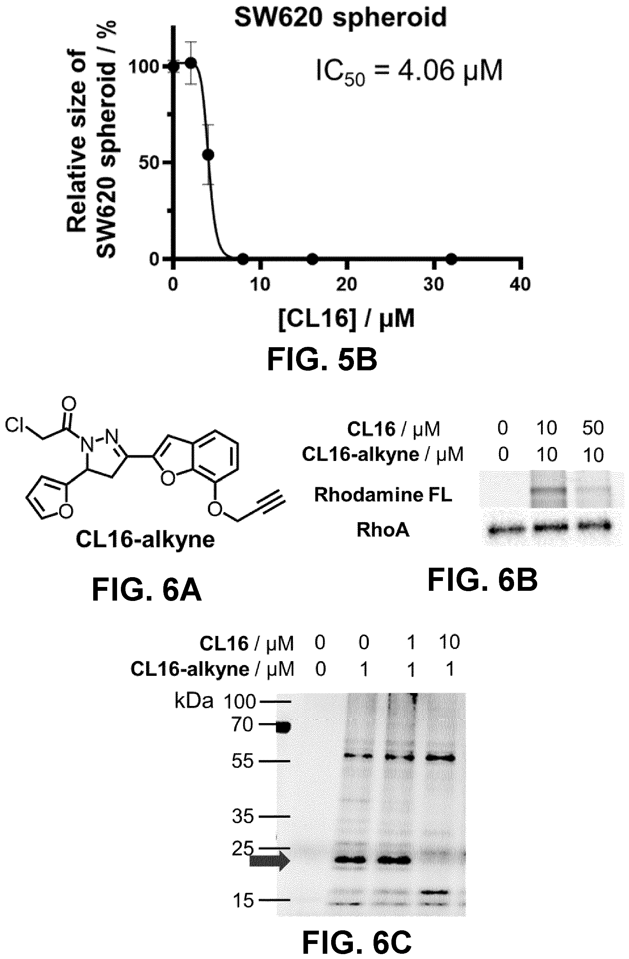

**Figures 5A-5B** are graphs showing the anticancer effects of CL16 in HT29 **(****Figure 5A****)** and SW620 **(****Figure 5B****)** spheroids. HT29 spheroid or SW620 spheroid were treated with CL16 at the indicated concentrations for 10 days. The spheroids were then imaged by microscope using 10x objective and the size was determined by ImageJ.
**Figure 6A** shows the chemical structure of the molecular probe for CL16, CL16-alkyne. **Figure 6B** shows the results of validation of covalent binding of CL16 onto RhoA. HCT116 cells transiently expressing FLAG-RhoA were pre-treated with solvent vehicle or CL16 for 2 h, followed by the incubation with CL16-alkyne in complete medium for another 2 h. The cells were then lysed and subjected to immunoprecipitation by anti-FLAG beads. The enriched proteins were reacted with rhodamine azide through coppercatalyzed azide-alkyne cycloaddition (CuAAC), boiled with sampling buffer and separated by SDS-PAGE. The protein bands were then visualized by their in-gel fluorescence or blotted for RhoA. **Figure 6C** shows the results of protein target of CL 16. HCT 116 cells were pre-treated with solvent vehicle or CL16 for 2 h, followed by the incubation with CL16-alkyne in complete medium for another 2 h. The cells were then lysed, reacted with rhodamine azide through CuAAC, boiled with sampling buffer, separated by SDS-PAGE and imaged. The arrow is pointing the primary protein target of CL16 at ~22 kDa which matches with the molecular weight of RhoA.

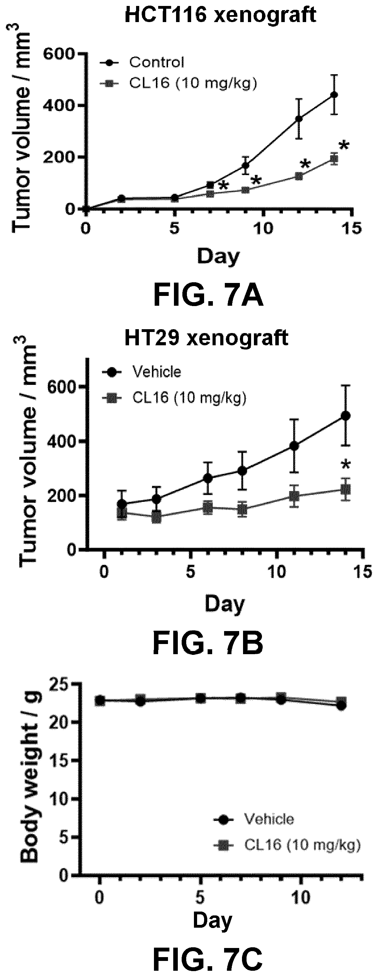

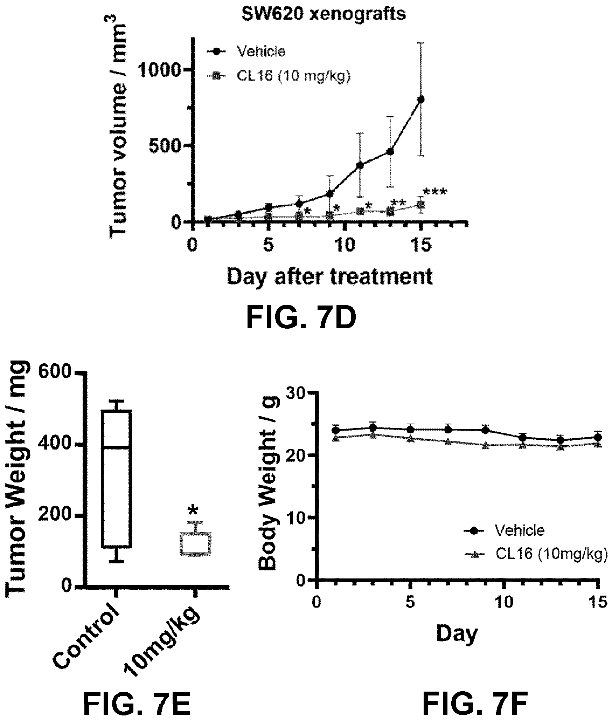

**Figure 7A** is a graph showing inhibition of tumor growth by CL16 in mouse HCT 116 xenografts through intraperitoneal injection at 10mg/kg (*n*=6 per group). **Figure 7B** is a graph showing inhibition of tumor growth by CL16 in mouse HT29 xenografts through intraperitoneal injection at 10mg/kg (*n*=7). **Figure 7C** is a graph showing the body weight of mice with HT29 xenografts throughout the treatment with CL16. **Figure 7D** is a graph showing inhibition of tumor growth by CL16 in mouse SW620 xenografts through intraperitoneal injection at 10mg/kg (*n*=6 per group). **Figure 7E** is a bar graph showing the tumor weight of harvested tumors from the treated mice with SW620 xenografts. **Figure 7F** is a graph showing the body weight of mice with SW620 xenografts throughout the treatment with CL16. Statistical analysis using a two-tailed Student's t-test. **P*<0.05.

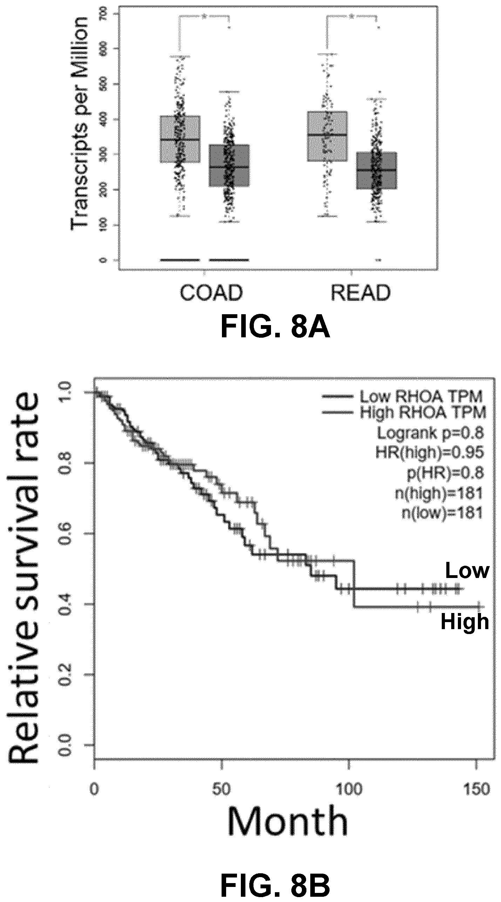

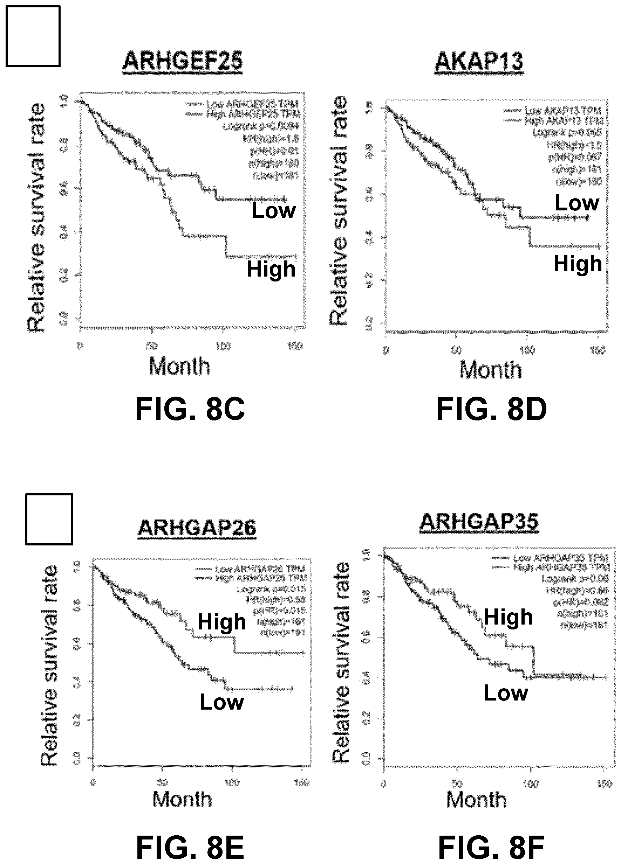

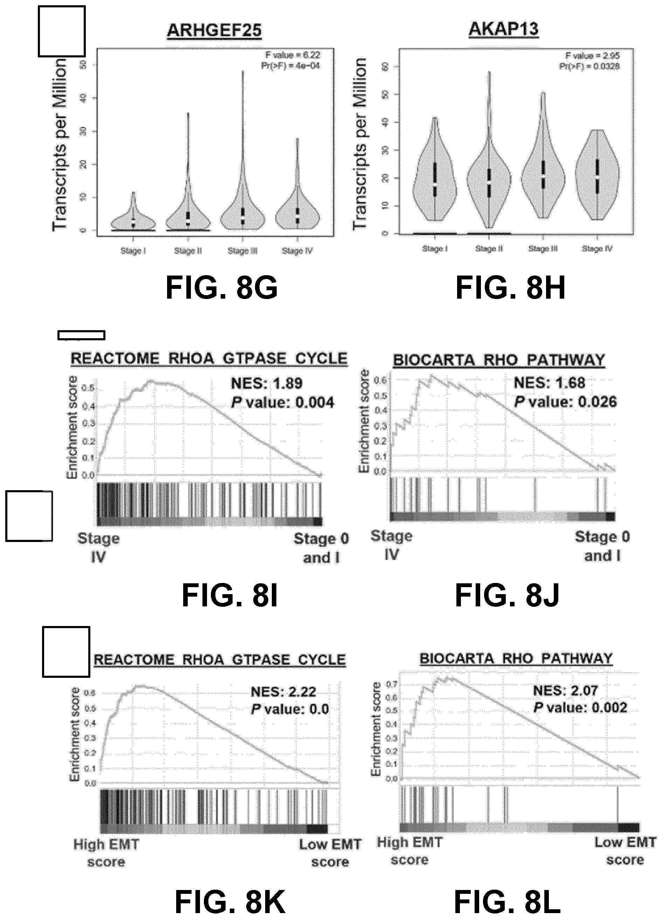

**Figure 8A** is a graph showing the analysis of RhoA expression levels in tumor (red) and normal tissues (grey), respectively, in patients from the colon adenocarcinoma (COAD) and rectum adenocarcinoma (READ) dataset in The Cancer Genome Atlas (TCGA) and Genotype-Tissue Expression (GTEx). **Figure 8B** is a graph showing the correlations of expression levels of RhoA with survival rates of patients in the TCGA and GTEx dataset. **Figures 8C** and **8D** are graphs showing the analysis of the expressions of GEFs and GAPs and their correlations with survival rates of patients in the TCGA and GTEx dataset for AARHGEF 25 **(****Figure 8C****)** and AKAP 14 **(****Figure 8D). Figures 8E** and **8F** are graphs showing the analysis of the expressions of GEFs and GAPs and their correlations with survival rates of patients in the TCGA and GTEx dataset for ARGAP26 **(****Figure 8E****)** and ARHGAP35 **(****Figure 8F****).** **Figures 8G** and **8H** are graphs showing the analysis of expression of GEFs in different tumor stages of patients in the TCGA and GTEx dataset for AARHGEF 25 **(****Figure 8G****)** and AKAP 13 **(****Figure 8H). Figures 8I** and **8J** are graphs showing the gene set enrichment analysis (GESA) of RhoA-related pathway in COAD and READ patients with early stage (0 and I) and late stage (IV) tumors in the TCGA dataset. NES: normalized enrichment score. **Figures 8K** and **8L** are graphs showing the GESA of RhoA-related pathway in COAD and READ patients in the TCGA dataset with high epithelial mesenchymal transition (EMT) score (mesenchymal-like) and low EMT score (epithelial-like).

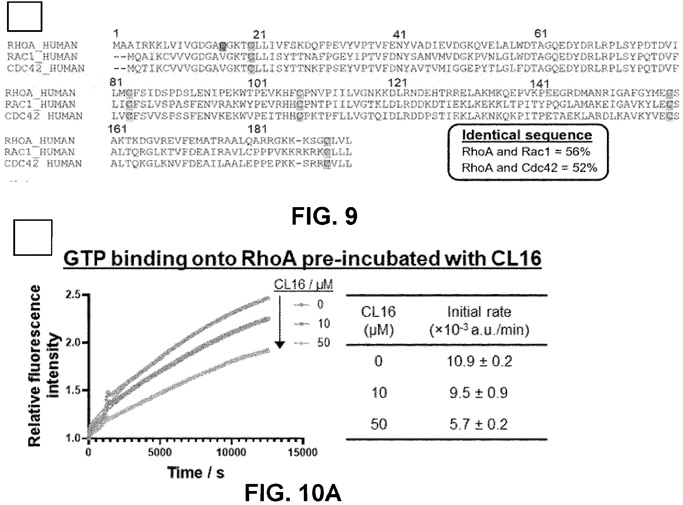

**Figure 9** shows the amino acid sequence alignment of human RhoA, Rac1 and Cdc42. Cys16 on RhoA is highlighted in yellow, while other conserved cysteines are highlighted in blue.

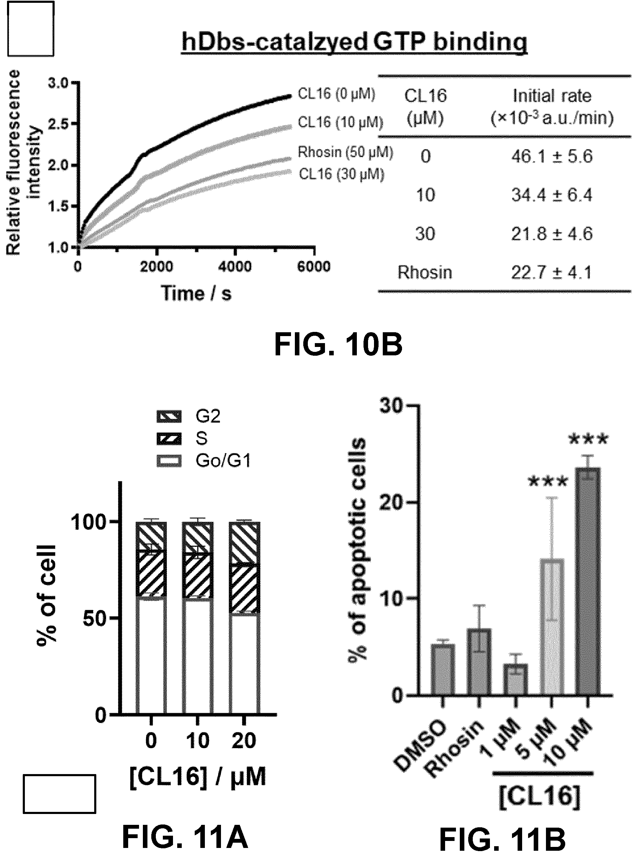

**Figure 10** is a graph showing GTP binding assay of RhoA (0.75 µg), CL16 and GTP-MANT (5.6 µM) in the buffer solution (20 mM Tris, 50 mM NaCl, 10 mM MgCl₂, pH 7.5).
**Figure 11A** is a graph showing flow cytometry analysis of the percentage of HCT116 cells in the Gₒ/G₁, S and G₂/M phase after incubation with CL16 for 24 h. **Figure 11B** is a graph showing the results from the apoptosis assay on CL16-treated HCT116 cells using Caspase-3/7 Green Detection Reagent.

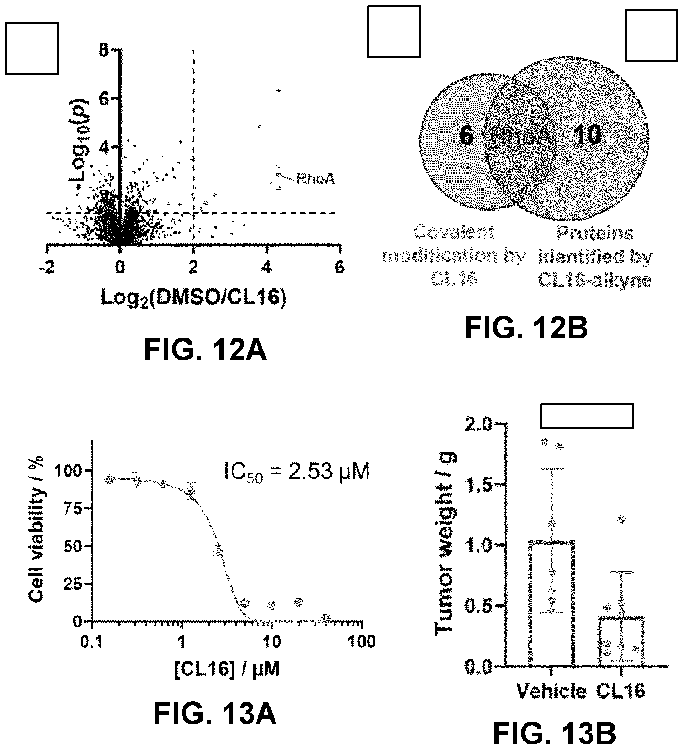

**Figure 12A** is a volcano plot of the MS experiment on **HCT116** cells probed by CL16-alkyne. **Figure 12B** is a graph showing the comparison of the proteins in HCT116 cells modified by CL16 and the proteins in HCT116 cells probed by CL16-alkyne.

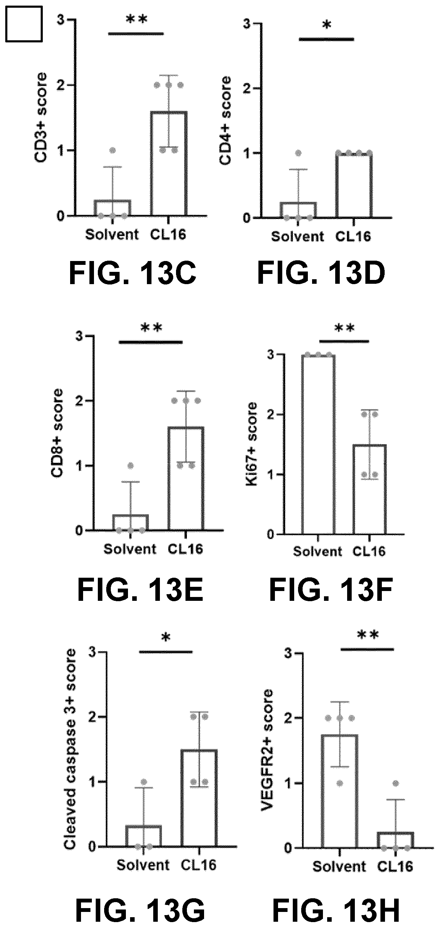

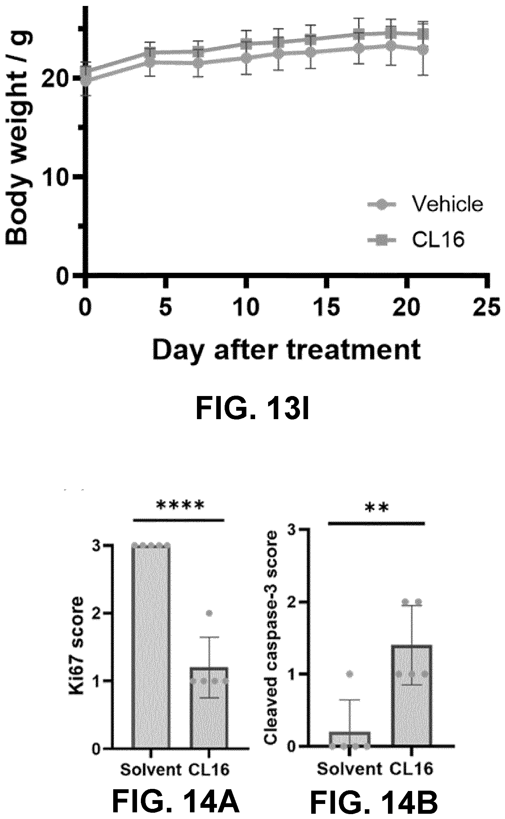

**Figure 13A** is a graph showing the cell viability, which was examined by MTT assay. SL4 cells were treated with solvent vehicle or CL16 in complete medium for 48 h. **Figure 13B** is a graph showing the weight of tumors from the mice treated with vehicle (n = 7) or CL16 (n = 8) at the end point of the investigation of CL16 treatment. **Figures 13C-****13H** are graphs showing the scores of the immunohistochemistry staining of CD3 **(****Figure 13C****),** CD4 **(****Figure 13D****),** CD8 **(****Figure 13E****),** Ki67 **(****Figure 13F****),** cleaved caspase-3 **(****Figure 13G****)** and VEGFR2 **(****Figure 13H****)** on tumor tissues from the vehicle- or CL16-treated mice, **Figure 13I** is a graph showing the changes in the body weight of mice with orthotopic SL4 CRC model treated with solvent vehicle or CL16 through i.p. injection.

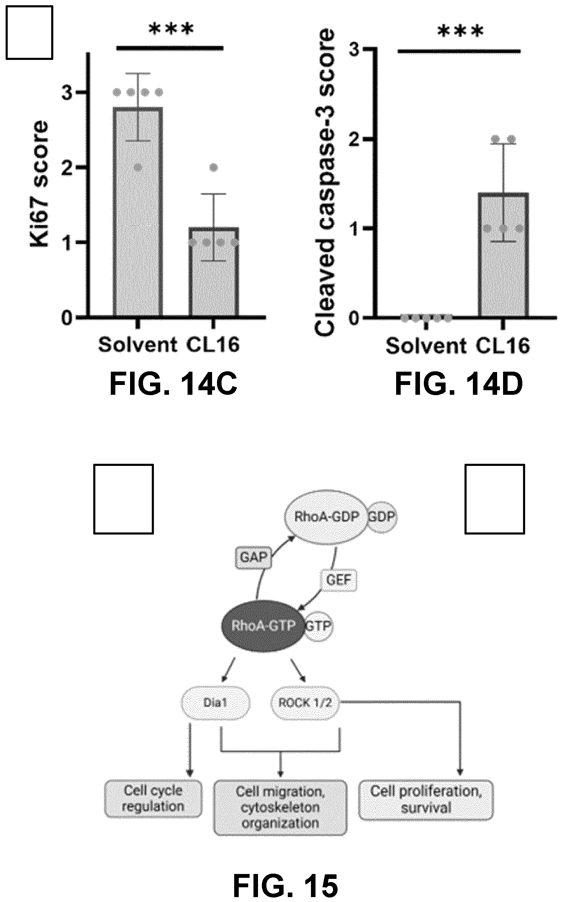

**Figures 14A** and **14B** are graphs showing immunohistochemistry staining scores for the images of H&E and immunohistochemistry staining of Ki67 **(****Figure 14A****)** and cleaved caspase-3 staining **(****Figure 14B****)** of tumor tissues from the vehicle- or CL16-treated mice. **Figure 14C** and **14D** are graphs showing immunohistochemistry staining scores for the images of H&E, Ki67 **(****Figure 14C****)** and cleaved caspase-3 **(****Figure 14D****)** staining of tumor tissues from the vehicle- or CL16-treated mice with HT29 xenografts.
**Figure 15** is a schematic cartoon illustrating RhoA biology and its regulation by guanine exchange factors (GEFs) and GTPase activating proteins (GAPs).

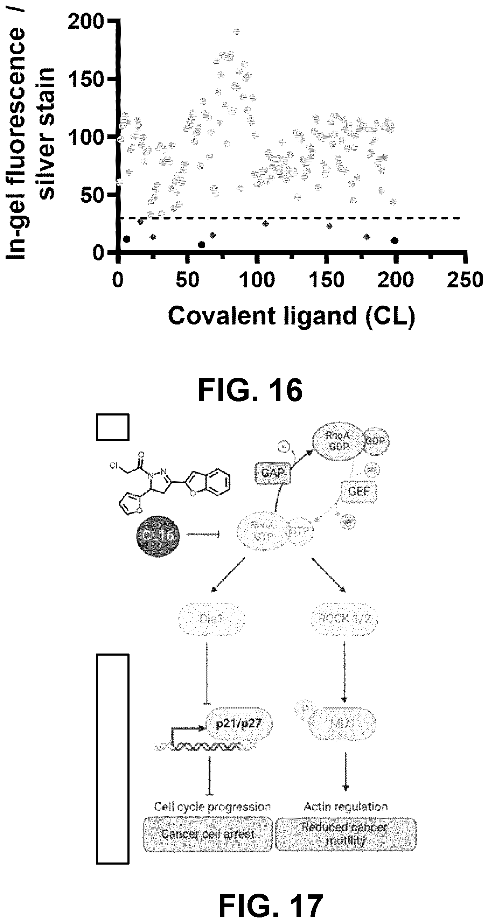

**Figure 16** is a graph showing the analysis of the 1^{st} screening result. Covalent ligands, that show lower than 30% of the intensity ratio and retain at least 70% intensity in the silver stain as compared to the DMSO control, are labeled in red and considered as lead compounds.
**Figure 17** is a schematic cartoon illustrating the mechanism of action of CL16 to mediate anticancer and antimetastatic effects by RhoA inhibition.

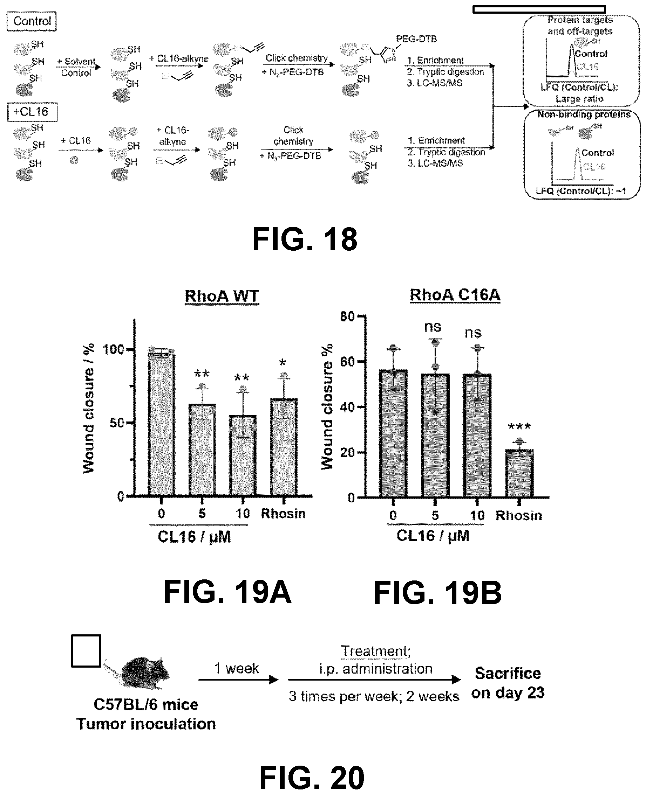

**Figure 18** is a schematic workflow illustrating the MS experiment using CL16-alkyne for the identification of protein targets of CL16 in HCT 116 cells.
**Figures 19A** and **19B** are graphs showing the results of the wound healing assay on HT29 cells expressing RhoA WT **(****Figure 19A****)** or C16A protein **(****Figure 19B****)** incubated with solvent vehicle, CL16 or Rhosin in complete medium for 48 h.
**Figure 20** is a schematic cartoon illustrating the workflow of the establishment of orthotopic mouse CRC model and the investigation of CL16 treatment.

### DETAILED DESCRIPTION OF THE INVENTION

It is to be understood that the disclosed compounds, compositions, and methods are not limited to specific synthetic methods, specific analytical techniques, or to particular reagents unless otherwise specified, and, as such, may vary. It is also to be understood that the terminology used herein is for the purpose of describing particular forms and embodiments only and is not intended to be limiting.

"Substituted," as used herein, refers to all permissible substituents of the compounds or functional groups described herein. In the broadest sense, the permissible substituents include acyclic and cyclic, branched and unbranched, carbocyclic and heterocyclic, aromatic and nonaromatic substituents of organic compounds. Illustrative substituents include, but are not limited to, halogens, hydroxyl groups, or any other organic groupings containing any number of carbon atoms, preferably 1-14 carbon atoms, and optionally include one or more heteroatoms such as oxygen, sulfur, or nitrogen grouping in linear, branched, or cyclic structural formats. Representative substituents include a substituted or unsubstituted alkyl, a substituted or unsubstituted alkenyl, a substituted or unsubstituted alkynyl, a substituted or unsubstituted heterocyclyl, a substituted or unsubstituted phenyl, a substituted or unsubstituted aryl, a substituted or unsubstituted heteroaryl, a substituted or unsubstituted polyaryl, a substituted or unsubstituted polyheteroaryl, a substituted or unsubstituted aralkyl, a halogen, a hydroxyl, an alkoxy, a phenoxy, an aroxy, a silyl, a thiol, an alkylthio, a substituted alkylthio, a phenylthio, an arylthio, a cyano, an isocyano, a nitro, a substituted or unsubstituted carbonyl, a carboxyl, an amino, an amido, an oxo, a sulfinyl, a sulfonyl, a sulfonic acid, a phosphonium, a phosphanyl, a phosphoryl, a phosphonyl, an amino acid. Such a substituted or unsubstituted alkyl, a substituted or unsubstituted alkenyl, a substituted or unsubstituted alkynyl, a substituted or unsubstituted heterocyclyl, a substituted or unsubstituted phenyl, a substituted or unsubstituted aryl, a substituted or unsubstituted heteroaryl, a substituted or unsubstituted polyaryl, a substituted or unsubstituted polyheteroaryl, a substituted or unsubstituted aralkyl, a halogen, a hydroxyl, an alkoxy, a phenoxy, an aroxy, a silyl, a thiol, an alkylthio, a substituted alkylthio, a phenylthio, an arylthio, a cyano, an isocyano, a nitro, a substituted or unsubstituted carbonyl, a carboxyl, an amino, an amido, an oxo, a sulfinyl, a sulfonyl, a sulfonic acid, a phosphonium, a phosphanyl, a phosphoryl, a phosphonyl, and an amino acid can be further substituted.

Heteroatoms such as nitrogen may have hydrogen substituents and/or any permissible substituents of organic compounds described herein which satisfy the valences of the heteroatoms. It is understood that "substitution" or "substituted" includes the implicit proviso that such substitution is in accordance with permitted valence of the substituted atom and the substituent, and that the substitution results in a stable compound, *i.e.,* a compound that does not spontaneously undergo transformation such as by rearrangement, cyclization, elimination, etc.

"Alkyl," as used herein, refers to the radical of saturated aliphatic groups, including straight-chain alkyl groups, branched-chain alkyl, and cycloalkyl (alicyclic). In some forms, a straight chain or branched chain alkyl has 30 or fewer carbon atoms in its backbone (e.g., C₁-C₃₀ for straight chains, C₃-C₃₀ for branched chains), 20 or fewer, 15 or fewer, or 10 or fewer. Alkyl includes methyl, ethyl, n-propyl, isopropyl, n-butyl, isobutyl, t-butyl, pentyl, hexyl, heptyl, octyl, decyl, tetradecyl, hexadecyl, eicosyl, tetracosyl and the like. Likewise, a cycloalkyl is a non-aromatic carbon-based ring composed of at least three carbon atoms, such as a nonaromatic monocyclic or nonaromatic polycyclic ring containing 3-30 carbon atoms, 3-20 carbon atoms, or 3-10 carbon atoms in their ring structure, and have 5, 6 or 7 carbons in the ring structure. Cycloalkyls containing a polycyclic ring system can have two or more non-aromatic rings in which two or more carbons are common to two adjoining rings (i.e., "fused cycloalkyl rings"). Examples of cycloalkyl groups include, but are not limited to, cyclopropyl, cyclobutyl, cyclopentyl, cyclohexyl, cycloheptyl, cyclooctanyl, etc.

"Substituted alkyl" refers to alkyl moieties having one or more substituents replacing a hydrogen on one or more carbons of the hydrocarbon backbone. Such substituents can be any substituents described above, e.g., halogen (such as fluorine, chlorine, bromine, or iodine), hydroxyl, carbonyl (such as a carboxyl, alkoxycarbonyl, formyl, or an acyl), thiocarbonyl (such as a thioester, a thioacetate, or a thioformate), aryl, alkoxyl, aralkyl, phosphonium, phosphanyl, phosphonyl, phosphoryl, phosphate, phosphonate, a phosphinate, amino, amido, amidine, imine, cyano, nitro, azido, oxo, sulfhydryl, thiol, alkylthio, silyl, sulfinyl, sulfate, sulfonate, sulfamoyl, sulfonamido, sulfonyl, heterocyclyl, an aromatic or heteroaromatic moiety. -NRR', wherein R and R' are independently hydrogen, alkyl, or aryl, and wherein the nitrogen atom is optionally quaternized; -SR, wherein R is a phosphonyl, a sulfinyl, a silyl a hydrogen, an alkyl, or an aryl; -CN; -NO₂; -COOH; carboxylate; -COR, -COOR, or -CON(R)₂, wherein R is hydrogen, alkyl, or aryl; imino, silyl, ether, haloalkyl (such as -CF3, -CH₂-CF₃, -CCl₃); -CN; -NCOCOCH₂CH_{2;} -NCOCOCHCH; and -NCS; and combinations thereof.

It will be understood by those skilled in the art that the moieties substituted on the hydrocarbon chain can themselves be substituted, if appropriate. For instance, the substituents of a substituted alkyl may include halogen, hydroxy, nitro, thiols, amino, aralkyl, azido, imino, amido, phosphonium, phosphanyl, phosphoryl (including phosphonate and phosphinate), oxo, sulfonyl (including sulfate, sulfonamido, sulfamoyl and sulfonate), and silyl groups, as well as ethers, alkylthios, carbonyls (including ketones, aldehydes, carboxylates, and esters), haloalkyls, -CN and the like. Cycloalkyls can be substituted in the same manner.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths.

"Heteroalkyl," as used herein, refers to straight or branched chain, or cyclic carbon-containing alkyl radicals, or combinations thereof, containing at least one heteroatom on the carbon backbone. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkyl group" is a cycloalkyl group as defined above where at least one of the carbon atoms of the ring is substituted with a heteroatom such as, but not limited to, nitrogen, oxygen, sulphur, or phosphorus.

The term "alkenyl" as used herein is a hydrocarbon group of from 2 to 24 carbon atoms and structural formula containing at least one carbon-carbon double bond. Alkenyl groups include straight-chain alkenyl groups, branched-chain alkenyl, and cycloalkenyl. A cycloalkenyl is a non-aromatic carbon-based ring composed of at least three carbon atoms and at least one carbon-carbon double bond, such as a nonaromatic monocyclic or nonaromatic polycyclic ring containing 3-30 carbon atoms and at least one carbon-carbon double bond, 3-20 carbon atoms and at least one carbon-carbon double bond, or 3-10 carbon atoms and at least one carbon-carbon double bond in their ring structure, and have 5, 6 or 7 carbons and at least one carbon-carbon double bond in the ring structure. Cycloalkenyls containing a polycyclic ring system can have two or more non-aromatic rings in which two or more carbons are common to two adjoining rings (i.e., "fused cycloalkenyl rings") and contain at least one carbon-carbon double bond. Asymmetric structures such as (AB)C=C(C'D) are intended to include both the *E* and *Z* isomers. This may be presumed in structural formulae herein wherein an asymmetric alkene is present, or it may be explicitly indicated by the bond symbol C. The term "alkenyl" as used throughout the specification, examples, and claims is intended to include both "unsubstituted alkenyls" and "substituted alkenyls," the latter of which refers to alkenyl moieties having one or more substituents replacing a hydrogen on one or more carbons of the hydrocarbon backbone. The term "alkenyl" also includes "heteroalkenyl."

The term "substituted alkenyl" refers to alkenyl moieties having one or more substituents replacing one or more hydrogen atoms on one or more carbons of the hydrocarbon backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, oxo, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

"Heteroalkenyl," as used herein, refers to straight or branched chain, or cyclic carbon-containing alkenyl radicals, or combinations thereof, containing at least one heteroatom. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkenyl group" is a cycloalkenyl group where at least one of the carbon atoms of the ring is substituted with a heteroatom such as, but not limited to, nitrogen, oxygen, sulphur, or phosphorus.

The term "alkynyl group" as used herein is a hydrocarbon group of 2 to 24 carbon atoms and a structural formula containing at least one carbon-carbon triple bond. Alkynyl groups include straight-chain alkynyl groups, branched-chain alkynyl, and cycloalkynyl. A cycloalkynyl is a non-aromatic carbon-based ring composed of at least three carbon atoms and at least one carbon-carbon triple bond, such as a nonaromatic monocyclic or nonaromatic polycyclic ring containing 3-30 carbon atoms and at least one carbon-carbon triple bond, 3-20 carbon atoms and at least one carbon-carbon triple bond, or 3-10 carbon atoms and at least one carbon-carbon triple bond in their ring structure, and have 5, 6 or 7 carbons and at least one carbon-carbon triple bond in the ring structure. Cycloalkynyls containing a polycyclic ring system can have two or more non-aromatic rings in which two or more carbons are common to two adjoining rings (i.e., "fused cycloalkynyl rings") and contain at least one carbon-carbon triple bond. Asymmetric structures such as (AB)C ≡C(C"D) are intended to include both the *E* and *Z* isomers. This may be presumed in structural formulae herein wherein an asymmetric alkyne is present, or it may be explicitly indicated by the bond symbol C. The term "alkynyl" as used throughout the specification, examples, and claims is intended to include both "unsubstituted alkynyls" and "substituted alkynyls," the latter of which refers to alkynyl moieties having one or more substituents replacing a hydrogen on one or more carbons of the hydrocarbon backbone. The term "alkynyl" also includes "heteroalkynyl."

The term "substituted alkynyl" refers to alkynyl moieties having one or more substituents replacing one or more hydrogen atoms on one or more carbons of the hydrocarbon backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

"Heteroalkynyl," as used herein, refers to straight or branched chain, or cyclic carbon-containing alkynyl radicals, or combinations thereof, containing at least one heteroatom. Suitable heteroatoms include, but are not limited to, O, N, Si, P and S, wherein the nitrogen, phosphorous and sulfur atoms are optionally oxidized, and the nitrogen heteroatom is optionally quaternized. For example, the term "heterocycloalkynyl group" is a cycloalkynyl group where at least one of the carbon atoms of the ring is substituted with a heteroatom such as, but not limited to, nitrogen, oxygen, sulphur, or phosphorus.

"Aryl," as used herein, refers to C₄-C₂₆-membered aromatic rings or fused ring systems containing one aromatic ring and optionally one or more non-aromatic rings. Examples of aryl groups are benzene, tetralin, indane, etc.

The term "substituted aryl" refers to an aryl group, wherein one or more hydrogen atoms on one or more aromatic rings are substituted with one or more substituents including, but not limited to, halogen, azide, alkyl, aralkyl, alkenyl, alkynyl, cycloalkyl, hydroxyl, alkoxy, carbonyl (such as a ketone, aldehyde, carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (such as CF₃, -CH₂-CF₃, -CCl₃), -CN, aryl, heteroaryl, and combinations thereof.

"Heterocyclo" and "heterocyclyl" are used interchangeably, and refer to a cyclic radical attached via a ring carbon or nitrogen atom of a monocyclic ring or polycyclic ring system containing 3-30 ring atoms, 3-20 ring atoms, 3-10 ring atoms, or 5-6 ring atoms, where the polycyclic ring system contains one or more non-aromatic rings and optionally one or more aromatic rings, where at least one non-aromatic ring contains carbon and one to four heteroatoms each selected from the group consisting of non-peroxide oxygen, sulfur, and N(Y) wherein Y is absent or is H, O, C₁-C₁₀ alkyl, phenyl or benzyl, and optionally containing 1-3 double bonds and optionally substituted with one or more substituents. Heterocyclyl are distinguished from heteroaryl by definition. Heterocycles can be a heterocycloalkyl, a heterocycloalkenyl, a heterocycloalkynyl, etc, such as piperazinyl, piperidinyl, piperidonyl, 4-piperidonyl, dihydrofuro[2,3-*b*]tetrahydrofuran, morpholinyl, piperazinyl, piperidinyl, piperidonyl, 4-piperidonyl, piperonyl, pyranyl, 2H-pyrrolyl, 4*H*-quinolizinyl, quinuclidinyl, tetrahydrofuranyl, 6*H*-1,2,5-thiadiazinyl. Heterocyclic groups can optionally be substituted with one or more substituents as defined above for alkyl and aryl.

The term "heteroaryl" refers to C₃-C₂₆-membered aromatic rings or fused ring systems containing one aromatic ring and one or more non-aromatic rings, in which one or more carbon atoms on the aromatic ring structure have been substituted with a heteroatom. Suitable heteroatoms include, but are not limited to, oxygen, sulfur, and nitrogen. Examples of heteroaryl groups pyrrole, furan, thiophene, imidazole, oxazole, thiazole, triazole, tetrazole, pyrazole, pyridine, pyrazine, pyridazine and pyrimidine, and the like. Examples of heteroaryl rings include, but are not limited to, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, furanyl, furazanyl, imidazolidinyl, imidazolinyl, imidazolyl, 1H-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, pteridinyl, purinyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, quinoxalinyl, tetrahydroisoquinolinyl, tetrahydroquinolinyl, tetrazolyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl and xanthenyl. One or more of the rings can be substituted as defined below for "substituted heteroaryl."

The term "substituted heteroaryl" refers to a heteroaryl group in which one or more hydrogen atoms on one or more heteroaromatic rings are substituted with one or more substituents including, but not limited to, halogen, azide, alkyl, aralkyl, alkenyl, alkynyl, cycloalkyl, hydroxyl, alkoxy, carbonyl (such as a ketone, aldehyde, carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (such as CF₃, -CH₂-CF₃, -CCl₃), -CN, aryl, heteroaryl, and combinations thereof.

The term "polyaryl" refers to a fused ring system that includes two or more aromatic rings and optionally one or more non-aromatic rings. Examples of polyaryl groups are naphthalene, anthracene, phenanthrene, chrysene, pyrene, corannulene, coronene, etc. When a fused ring system containing two or more aromatic rings and optionally one or more non-aromatic rings, in which one or more carbon atoms on one or more aromatic ring structures have been substituted with a heteroatom, the fused ring system can be referred to as a "heteropolyaryl" or "polyheteroaryl". The terms "heteropolyaryl" and "polyheteroaryl" are used interchangeably herein.

The term "substituted polyaryl" refers to a polyaryl in which one or more of the aryls are substituted, with one or more substituents including, but not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (or quarternized amino), amido, amidine, imine, cyano, nitro, azido, sulfhydryl, alkylthio, sulfate, sulfonate, sulfamoyl, sulfoxide, sulfonamido, sulfonyl, heterocyclyl, alkylaryl, haloalkyl, -CN, aryl, heteroaryl, and combinations thereof. When a polyheteroaryl is involved, the chemical moiety can be referred to as a "substituted polyheteroaryl."

The term "cyclic," "cyclic ring" or "cyclic group" refers to a substituted or unsubstituted monocyclic ring or a substituted or unsubstituted polycyclic ring (such as those formed from single or fused ring systems), such as a substituted or unsubstituted cycloalkyl, a substituted or unsubstituted cycloalkenyl, a substituted or unsubstituted cycloalkynyl, or a substituted or unsubstituted heterocyclyl, that have from three to 30 carbon atoms, as geometric constraints permit. The substituted cycloalkyls, cycloalkenyls, cycloalkynyls, and heterocyclyls are substituted as defined above for the alkyls, alkenyls, alkynyls, and heterocyclyls, respectively.

The term "aralkyl" as used herein is an aryl group or a heteroaryl group having an alkyl, alkynyl, or alkenyl group as defined above attached to the aromatic group, such as an aryl, a heteroaryl, a polyaryl, or a polyheteroaryl. An example of an aralkyl group is a benzyl group.

The terms "alkoxyl" or "alkoxy," "aroxy" or "aryloxy," generally describe compounds represented by the formula -OR^{v}, wherein R^{v} includes, but is not limited to, a substituted or unsubstituted alkyl, a substituted or unsubstituted alkenyl, a substituted or unsubstituted alkynyl, a substituted or unsubstituted cycloalkyl, a substituted or unsubstituted heterocyclyl, a substituted or unsubstituted cycloalkenyl, a substituted or unsubstituted heterocycloalkenyl, a substituted or unsubstituted aryl, a substituted or unsubstituted heteroaryl, a substituted or unsubstituted polyaryl, a substituted or unsubstituted polyheteroaryl, a substituted or unsubstituted arylalkyl, a substituted or unsubstituted heteroalkyl, a substituted or unsubstituted alkylaryl, a substituted or unsubstituted alkylheteroaryl, a substituted or unsubstituted aralkyl, a substituted or unsubstituted carbonyl, a phosphonium, a phosphanyl, a phosphonyl, a sulfinyl, a silyl, a thiol, an amido, and an amino. Exemplary alkoxyl groups include methoxy, ethoxy, propyloxy, tert-butoxy and the like. A "lower alkoxy" group is an alkoxy group containing from one to six carbon atoms. An "ether" is two functional groups covalently linked by an oxygen as defined below. Accordingly, the substituent of an alkyl that renders that alkyl an ether is or resembles an alkoxyl, such as can be represented by one of -O-alkyl, -O-alkenyl, -O-alkynyl, -O-arakyl, -O-aryl, -O-heteroaryl, -O-polyaryl, -O-polyheteroaryl, -O-heterocyclyl, etc.

The term "substituted alkoxy" refers to an alkoxy group having one or more substituents replacing one or more hydrogen atoms on one or more carbons of the alkoxy backbone. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, oxo, sulfate, sulfonate, sulfamoyl, sulfonamido, sulfonyl, heterocyclyl, alkylaryl, haloalkyl, -CN, aryl, heteroaryl, and combinations thereof.

The term "ether" as used herein is represented by the formula A²OA¹, where A² and A¹ can be, independently, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl, a substituted or unsubstituted polyaryl, a substituted or unsubstituted polyheteroaryl, a phosphonium, a phosphanyl, a phosphonyl, a sulfinyl, a silyl, a thiol, a substituted or unsubstituted carbonyl, an alkoxy, an amido, or an amino, described above.

The term "polyether" as used herein is represented by the formula: 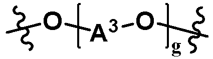 where A³ can be, independently, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl, a substituted or unsubstituted polyaryl, a substituted or unsubstituted polyheteroaryl, a phosphonium, a phosphanyl, a substituted or unsubstituted carbonyl, an alkoxy, an amido, or an amino, described above; g can be a positive integer from 1 to 30.

The term "phenoxy" is art recognized and refers to a compound of the formula -OR^{v} wherein R^{v} is C₆H₅ (*i.e.,* -O-C₆H₅). One of skill in the art recognizes that a phenoxy is a species of the aroxy genus.

The term "substituted phenoxy" refers to a phenoxy group, as defined above, having one or more substituents replacing one or more hydrogen atoms on one or more carbons of the phenyl ring. Such substituents include, but are not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, and combinations thereof.

The terms "aroxy" and "aryloxy," as used interchangeably herein, are represented by -O-aryl or -O-heteroaryl, wherein aryl and heteroaryl are as defined herein.

The terms "substituted aroxy" and "substituted aryloxy," as used interchangeably herein, represent -O-aryl or -O-heteroaryl, having one or more substituents replacing one or more hydrogen atoms on one or more ring atoms of the aryl and heteroaryl, as defined herein. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The term "amino" as used herein includes the group 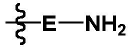 (primary amino), 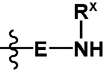 (secondary amino), 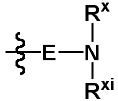 (tertiary amino), and 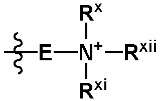 (quaternary amino), wherein, E is absent, or E is substituted or unsubstituted alkyl, substituted or unsubstituted alkenyl, substituted or unsubstituted alkynyl, substituted or unsubstituted aralkyl, substituted or unsubstituted aryl, substituted or unsubstituted heteroaryl, a substituted or unsubstituted polyaryl, a substituted or unsubstituted polyheteroaryl, substituted or unsubstituted heterocyclyl, wherein independently of E, R^{x}, R^{xi}, and R^{xii} each independently represent a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. The term "quaternary amino" also includes the groups where the nitrogen, R^{x}, R^{xi}, and R^{xii} with the N⁺ to which they are attached complete a heterocyclyl or heteroaryl having from 3 to 14 atoms in the ring structure. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The terms "amide" or "amido" are used interchangeably, refer to both "unsubstituted amido" and "substituted amido" and are represented by the general formula: 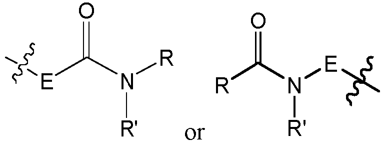 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted aryl, substituted or unsubstituted heteroaryl, a substituted or unsubstituted polyaryl, a substituted or unsubstituted polyheteroaryl, or a substituted or unsubstituted heterocyclyl, wherein independently of E, R and R' each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or Rand R' taken together with the N atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. In some forms, when E is oxygen, a carbamate is formed. It is understood by those of ordinary skill in the art, that the E groups listed above are divalent (e.g., methylene, ethane-1,2-diyl, ethene-1,2-diyl, 1,4-phenylene, cyclohexane-1,2-diyl).

"Carbonyl," as used herein, is art-recognized and includes such moieties as can be represented by the general formula: 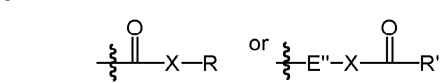 wherein X is a bond, or represents an oxygen or a sulfur, and R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R", or a pharmaceutical acceptable salt; E" is absent, or E" is a substituted or unsubstituted alkyl, a substituted or unsubstituted alkenyl, a substituted or unsubstituted alkynyl, a substituted or unsubstituted aralkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl; R' represents a hydrogen, a substituted or unsubstituted alkyl, a substituted or unsubstituted alkenyl, a substituted or unsubstituted alkynyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R"; R" represents a hydroxyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphonium, phosphanyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, and combinations thereof. It is understood by those of ordinary skill in the art, that the E" groups listed above are divalent (e.g., methylene, ethane-1,2-diyl, ethene-1,2-diyl, 1,4-phenylene, cyclohexane-1,2-diyl). Where X is oxygen and R is defined as above, the moiety is also referred to as a carboxyl group. When X is oxygen and R is hydrogen, the formula represents a "carboxylic acid." Where X is oxygen and R' is hydrogen, the formula represents a "formate." Where X is oxygen and R or R' is not hydrogen, the formula represents an "ester." In general, where the oxygen atom of the above formula is replaced by a sulfur atom, the formula represents a "thiocarbonyl" group. Where X is sulfur and R or R' is not hydrogen, the formula represents a "thioester." Where X is sulfur and R is hydrogen, the formula represents a "thiocarboxylic acid." Where X is sulfur and R' is hydrogen, the formula represents a "thioformate." Where X is a bond and R is not hydrogen, the above formula represents a "ketone." Where X is a bond and R is hydrogen, the above formula represents an "aldehyde."

The term "phosphanyl" is represented by the formula 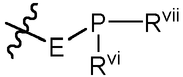 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted cycloalkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R^{vi} and R^{vii} each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi} and R^{vii} taken together with the P atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphonium" is represented by the formula 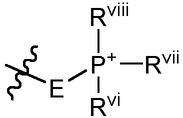 wherein, E is absent, or E is a substituted or unsubstituted alkyl, a substituted or unsubstituted alkenyl, a substituted or unsubstituted alkynyl, a substituted or unsubstituted aralkyl, a substituted or unsubstituted cycloalkyl, a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R^{vi}, R^{vii}, and R^{viii} each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi}, R^{vii}, and R^{viii} taken together with the P⁺ atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphonyl" is represented by the formula 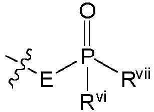 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, oxygen, alkoxy, aroxy, or substituted alkoxy or substituted aroxy, wherein, independently of E, R^{vi} and R^{vii} are independently a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a sulfinyl, a silyl, a thiol, an amido, an amino, or -(CH₂)ₘ-R‴, or R^{vi} and R^{vii} taken together with the P atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "phosphoryl" defines a phosphonyl in which E is absent, oxygen, alkoxy, aroxy, substituted alkoxy or substituted aroxy, as defined above, and independently of E, R^{vi} and R^{vii} are independently hydroxyl, alkoxy, aroxy, substituted alkoxy or substituted aroxy, as defined above. When E is oxygen, the phosphoryl cannot be attached to another chemical species, such as to form an oxygen-oxygen bond, or other unstable bonds, as understood by one of ordinary skill in the art. When E, R^{vi} and R^{vii} are substituted, the substituents include, but are not limited to, halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfinyl" is represented by the formula 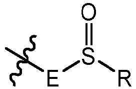 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted heterocyclyl, a substituted or unsubstituted polyaryl, a substituted or unsubstituted polyheteroaryl, wherein independently of E, R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, a phosphonyl, a silyl, a thiol, an amido, an amino, or -(CH₂)ₘ-R‴, or E and R taken together with the S atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonyl" is represented by the formula 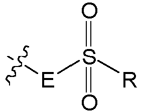 wherein E is absent, or E is a substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted arylalkyl, etc.), a substituted or unsubstituted aryl, a substituted or unsubstituted heteroaryl, a substituted or unsubstituted heterocyclyl, a substituted or unsubstituted polyaryl, a substituted or unsubstituted polyheteroaryl, wherein independently of E, R represents a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R‴, or E and R taken together with the S atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonic acid" refers to a sulfonyl, as defined above, wherein R is hydroxyl, and E is absent, or E is substituted or unsubstituted cycloalkyl, substituted or unsubstituted heterocyclyl, substituted or unsubstituted alkylaryl, substituted or unsubstituted arylalkyl, substituted or unsubstituted aryl, a substituted or unsubstituted polyaryl, a substituted or unsubstituted polyheteroaryl, or substituted or unsubstituted heteroaryl. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfate" refers to a sulfonyl, as defined above, wherein E is absent, oxygen, alkoxy, aroxy, substituted alkoxy or substituted aroxy, as defined above, and R is independently hydroxyl, alkoxy, aroxy, substituted alkoxy or substituted aroxy, as defined above. When E is oxygen, the sulfate cannot be attached to another chemical species, such as to form an oxygen-oxygen bond, or other unstable bonds, as understood by one of ordinary skill in the art. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfonate" refers to a sulfonyl, as defined above, wherein E is oxygen, alkoxy, aroxy, substituted alkoxy or substituted aroxy, as defined above, and R is independently hydrogen, substituted or unsubstituted alkyl, substituted or unsubstituted alkenyl, substituted or unsubstituted alkynyl, substituted or unsubstituted amino, substituted or unsubstituted cycloalkyl, substituted or unsubstituted heterocyclyl, substituted or unsubstituted aralkyl, substituted or unsubstituted alkylaryl, substituted or unsubstituted arylalkyl, substituted or unsubstituted aryl, substituted or unsubstituted heteroaryl, a substituted or unsubstituted polyaryl, a substituted or unsubstituted polyheteroaryl, -(CH₂)ₘ-R‴, R‴ represents a hydroxy group, substituted or unsubstituted carbonyl group, an aryl, a cycloalkyl ring, a cycloalkenyl ring, a heterocycle, an amido, an amino, or a polycycle; and m is zero or an integer ranging from 1 to 8. When E is oxygen, sulfonate cannot be attached to another chemical species, such as to form an oxygen-oxygen bond, or other unstable bonds, as understood by one of ordinary skill in the art. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "sulfamoyl" refers to a sulfonamide or sulfonamide represented by the formula 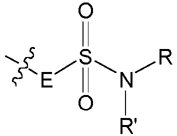 wherein E is absent, or E is substituted or unsubstituted alkyl, a substituted or unsubstituted alkenyl, a substituted or unsubstituted alkynyl, a substituted or unsubstituted aralkyl (e.g., a substituted or unsubstituted alkylaryl, a substituted or unsubstituted cycloalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, wherein independently of E, R and R' each independently represent a hydrogen, a substituted or unsubstituted alkyl, a substituted or unsubstituted alkenyl, a substituted or unsubstituted alkynyl, a substituted or unsubstituted carbonyl, a substituted or unsubstituted heterocyclyl, a substituted or unsubstituted aralkyl (e.g. a substituted or unsubstituted alkylaryl, a substituted or unsubstituted arylalkyl, etc.), a substituted or unsubstituted aryl, a substituted or unsubstituted heteroaryl, a substituted or unsubstituted polyaryl, a substituted or unsubstituted polyheteroaryl, a substituted or unsubstituted heterocyclyl, a hydroxyl, an alkoxy, a phosphonium, a phosphanyl, an amido, an amino, or -(CH₂)ₘ-R‴, or Rand R' taken together with the N atom to which they are attached complete a heterocycle having from 3 to 14 atoms in the ring structure; R‴ represents a hydroxyl group, a substituted or unsubstituted carbonyl group, a substituted or unsubstituted aryl, a substituted or unsubstituted cycloalkyl, a substituted or unsubstituted cycloalkenyl, a substituted or unsubstituted heterocyclyl, a substituted or unsubstituted aryl, a substituted or unsubstituted heteroaryl, a substituted or unsubstituted polyaryl, a substituted or unsubstituted polyheteroaryl, an alkoxy, a phosphonium, a phosphanyl, an amido, or an amino; and m is zero or an integer ranging from 1 to 8. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof. It is understood by those of ordinary skill in the art, that the E groups listed above are divalent (e.g., methylene, ethane-1,2-diyl, ethene-1,2-diyl, 1,4-phenylene, cyclohexane-1,2-diyl).

The term "silyl group" as used herein is represented by the formula -SiRR'R," where R, R', and R" can be, independently, a hydrogen, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl (e.g. a substituted or unsubstituted alkylaryl, a substituted or unsubstituted arylalkyl, etc.), a substituted or unsubstituted polyaryl, a substituted or unsubstituted polyheteroaryl, a substituted or unsubstituted carbonyl, a phosphonium, a phosphanyl, a phosphonyl, a sulfinyl, a thiol, an amido, an amino, an alkoxy, or an oxo, described above. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The terms "thiol" are used interchangeably and are represented by -SR, where R can be a hydrogen, a substituted or unsubstituted alkyl, a substituted or unsubstituted alkenyl, a substituted or unsubstituted alkynyl, a substituted or unsubstituted heterocyclyl, a substituted or unsubstituted aryl, a substituted or unsubstituted heteroaryl, a substituted or unsubstituted aralkyl (e.g. a substituted or unsubstituted alkylaryl, a substituted or unsubstituted arylalkyl, etc.), a substituted or unsubstituted polyaryl, a substituted or unsubstituted polyheteroaryl, a substituted or unsubstituted carbonyl, a phosphonium, a phosphanyl, an amido, an amino, an alkoxy, an oxo, a phosphonyl, a sulfinyl, or a silyl, described above. Such substituents can be any substituents described above, e.g., halogen, azide, alkyl, aralkyl, alkenyl, alkynyl, cycloalkyl, hydroxyl, carbonyl (such as a carboxyl, alkoxycarbonyl, formyl, or an acyl), silyl, ether, ester, thiocarbonyl (such as a thioester, a thioacetate, or a thioformate), alkoxyl, phosphoryl, phosphate, phosphonate, phosphinate, amino (e.g. quarternized amino), amido, amidine, imine, cyano, nitro, azido, sulfhydryl, alkylthio, sulfate, sulfonate, sulfamoyl, sulfonamido, sulfonyl, heterocyclyl, alkylaryl, haloalkyl, -CN, aryl, heteroaryl, polyaryl, polyheteroaryl, and combinations thereof.

The disclosed compounds and substituent groups, can, independently, possess two or more of the groups listed above. For example, if the compound or substituent group is a straight chain alkyl group, one of the hydrogen atoms of the alkyl group can be substituted with a hydroxyl group, an alkoxy group, etc. Depending upon the groups that are selected, a first group can be incorporated within second group or, alternatively, the first group can be pendant (i.e., attached) to the second group. For example, with the phrase "an alkyl group comprising an ester group," the ester group can be incorporated within the backbone of the alkyl group. Alternatively, the ester can be attached to the backbone of the alkyl group. The nature of the group(s) that is (are) selected will determine if the first group is embedded or attached to the second group.

The compounds and substituents can be substituted, independently, with the substituents described above in the definition of "substituted."

The numerical ranges disclose individually each possible number that such a range could reasonably encompass, as well as any sub-ranges and combinations of sub-ranges encompassed therein. For example, in a given range carbon range of C₃-C₉, the range also discloses C₃, C₄, C₅, C₆, C₇, C₈, and C₉, as well as any subrange between these numbers (for example, C₄ -C₆), and any possible combination of ranges possible between these values. In yet another example, a given temperature range may be from about 25 °C to 30 °C, where the range also discloses temperatures that can be selected independently from about 25, 26, 27, 28, 29, and 30 °C, as well as any range between these numbers (for example, 26 to 28 °C), and any possible combination of ranges between these values.

Use of the term "about" is intended to describe values either above or below the stated value, which the term "about" modifies, to be within a range of approximately +/-10%. When the term "about" is used before a range of numbers (*i.e*., about 1-5) or before a series of numbers (*i.e*., about 1, 2, 3, 4, etc.) it is intended to modify both ends of the range of numbers and/or each of the numbers recited in the entire series, unless specified otherwise.

The disclosed compounds and substituent groups, can, independently, possess two or more of the groups listed above. For example, if the compound or substituent group is a straight chain alkyl group, one of the hydrogen atoms of the alkyl group can be substituted with a hydroxyl group, an alkoxy group, etc. Depending upon the groups that are selected, a first group can be incorporated within second group or, alternatively, the first group can be pendant (i.e., attached) to the second group. For example, with the phrase "an alkyl group comprising an ester group," the ester group can be incorporated within the backbone of the alkyl group. Alternatively, the ester can be attached to the backbone of the alkyl group. The nature of the group(s) that is (are) selected will determine if the first group is embedded or attached to the second group.

The compounds and substituents can be substituted with, independently, with the substituents described above in the definition of "substituted."

### II. Compositions

RhoA covalent inhibitors (also referred to herein as "RhoA inhibitors" or "compounds") have been developed. The disclosed RhoA inhibitors are suitable for use in cancer therapy, owning to their high RhoA inhibitory efficacy and high RhoA specificity.

The disclosed RhoA inhibitors can covalently bind RhoA, and thereby achieve improved inhibitory efficacy (i.e., down to about 400 nM). Further, the disclosed RhoA inhibitors can specifically bind RhoA over other GTPases, such as other Ras GTPases (e.g., K-Ras and N-Ras) and particularly other Rho GTPases (e.g., Cdc42 and Rac1). This combination of high RhoA inhibitory efficacy and high RhoA specificity of the disclosed RhoA inhibitors allow these compounds to show high cytotoxicity in cancer cells, with negligible toxicity in normal cells, in *vitro* and in *vivo.* Prior to the disclosed RhoA covalent inhibitors, there were no RhoA covalent inhibitors having *in vivo* antitumor effects on mouse colorectal cancer xenograft models with good efficacy.

For example, as demonstrated by the data in the Examples below, exemplary compound CL16 showed high cytotoxicity in CRC cells, with correlation with the RhoA expression/activity in the cancer cells, while it is much less toxic for normal colon fibroblast which has lower RhoA level. Further, CL16 administered in tumor-bearing mice inhibited tumor growth *in vivo,* with no observable toxicity was found from mice treated with the exemplary compound CL16, as shown by the body weight measurement and immunohistochemistry on the tissue sections of different organs.

The superior efficacy and specificity of the disclosed RhoA inhibitors also allow them to outperform the reported RhoA inhibitors, Rhosin and DC-Rhoin. For example, as demonstrated by the data in the Examples below, exemplary compound CL16 inhibited the growth of CRC spheroids, with better potency than the reported RhoA inhibitor, Rhosin. Further, since the binding site of DC-Rhoin, i.e., Cys107, is conserved in Rac1 and Cdc42, DC-Rhoin alters Rac1 and Cdc42 signals, which leads to cytotoxicity. In contrast, the disclosed compounds are relatively non-toxic in normal cells.

Without being bound to any theories, it is believed that the superior efficacy and specificity of the disclosed RhoA inhibitors arises from covalent binding of these compounds onto Cys16, which locates near the nucleotide-binding pocket of RhoA. Upon binding, the disclosed RhoA inhibitors resided next to the nucleotide-binding pocket in RhoA, which results in significant decreases in the GDP-to-GTP self-exchange and the LARG-mediated exchange, and thereby leads to their high inhibitory efficacy. Further, the binding site of the disclosed RhoA inhibitors, i.e., Cys16, is unique to RhoA compared to other GTPases (such as Rac1 and Cdc42), and thereby leads to their high binding specificity.

In some forms, the disclosed RhoA inhibitors can contain a probe, such as an alkyne group, for biological studies of RhoA functions and activities in cancers, as well as studies of the disclosed RhoA inhibitors in cancer therapy. For example, as shown in the Examples below, a molecular probe of CL16, CL16-alkyne, was used to validate the specific binding of CL16 with RhoA.

Pharmaceutical formulations containing the RhoA inhibitors are also disclosed.

### A. RhoA Inhibitors and Probes Thereof

The compounds disclosed herein are covalent RhoA inhibitors that have high RhoA inhibitory efficacy and high RhoA specificity. These are suitable for use in cancer therapy.

In some forms, the disclosed RhoA inhibitors can have the structure of Formula I: 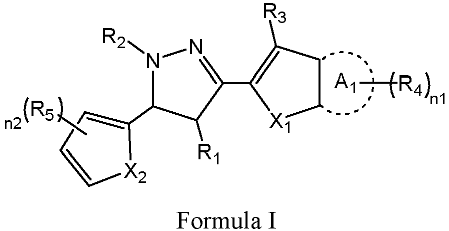 wherein: (i) X₁ and X₂ can be independently C(R₆)₂, O, S, or NR₆ and R₆ is hydrogen, a substituted or unsubstituted alkyl, a substituted or unsubstituted alkenyl, a substituted or unsubstituted alkynyl, an alkoxyl, or a carbonyl; (ii) A₁ can be a C₅-C₆ aromatic ring or a C₄-C₅ heteroaromatic ring; (iii) n1 can be an integer from 0 to 4, from 0 to 3, from 0 to 2, such as 0, 1, or 2; (iv) n2 can be an integer from 0 to 3, from 0 to 2, or 0 or 1; (v) R1, R₃, and R₅ can be independently hydrogen, a substituted or unsubstituted alkyl, or a substituted or unsubstituted aryl; (vi) R₂ is a cysteine-reactive group (such as haloacetamide, acrylamide, maleimide, vinyl sulfone and epoxide), which allows chemical reaction with cysteines on RhoA; (vii) R₄ can be hydrogen, an alkoxyl, a hydroxyl, a halogen, a substituted or unsubstituted alkynyl, a substituted or unsubstituted aryl, a carbonyl, an amino, or a haloalkyl; and (viii) the substituents, when present, can be independently unsubstituted alkyl, unsubstituted aryl, unsubstituted heteroaryl, unsubstituted polyaryl, unsubstituted heteropolyaryl, unsubstituted alkylaryl, unsubstituted cyclic, unsubstituted heterocyclic, halide, amino, amido, thiol, hydroxyl, cyano, nitro, carbonyl, or alkoxyl.

In some forms, the disclosed RhoA inhibitors can have the structure of Formula II: 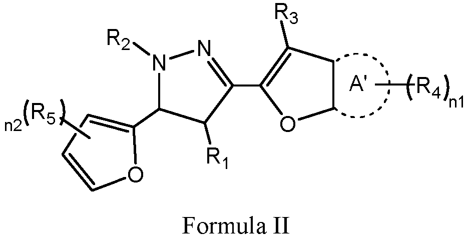 wherein: A', n1, n2, and R₁-R₅ can be as defined above for Formula I.

In some forms, the disclosed RhoA inhibitors can have the structure of Formula III: 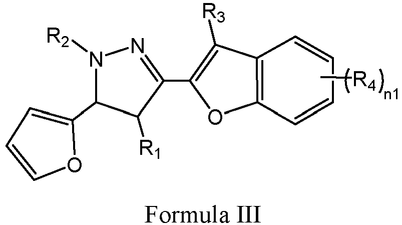 wherein: n1 and R₁-R₄ can be as defined above for Formula I.

In some forms, for any of the formulae described herein, n1 can be 0 or 1.

In some forms, for any of the formulae described herein, R₁ can be hydrogen.

In some forms, for any of the formulae described herein, R₃ can be hydrogen or a substituted or unsubstituted aryl, wherein the substituents, when present, are independently an unsubstituted alkyl, an unsubstituted aryl, or an unsubstituted alkyl aryl.

In some forms, for any of the formulae described herein, R₂ can be 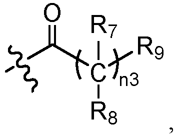 where n3 can be an integer from 0 to 6, from 0 to 5, from 0 to 4, from 0 to 3, from 0 to 2, such as 0, 1, or 2; R₇ and R₈ can be independently hydrogen, an unsubstituted alkyl, or halogen; and R₉ can be hydrogen or halogen.

In some forms, for any of the formulae described herein, R₄ can be hydrogen.

In some forms, for any of the formulae described herein, R₄ can be 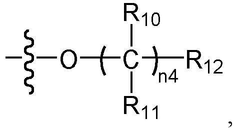 where n4 can be an integer from 0 to 6, from 0 to 5, from 0 to 4, from 0 to 3, from 0 to 2, such as 0, 1, or 2; R₁₀ and R₁₁ can be independently hydrogen or halogen; and R₁₂ can be hydrogen, halogen, amino, an unsubstituted alkynyl, aldehyde, or carboxylic acid. In some forms, R₁₀ and R₁₁ can be hydrogen, and R₁₂ can be an unsubstituted alkynyl. In these forms, R₄ can function as a probe that allows further functionalization with and/or binding to a target, such as detection moieties (e.g., a fluorophore), for biological study (e.g., RhoA inhibitor binding and function *in vitro* and/or *in vivo*). An exemplary compound is CL-alkyne shown below.

For any of the formulae described herein, the alkyl (when present) can be a linear alkyl, a branched alkyl, or a cyclic alkyl (either monocyclic or polycyclic). The terms "cyclic alkyl" and "cycloalkyl" are used interchangeably herein. Exemplary alkyl include a linear C₁-C₃₀ alkyl, a branched C₄-C₃₀ alkyl, a cyclic C₃-C₃₀ alkyl, a linear C₁-C₂₀ alkyl, a branched C₄-C₂₀ alkyl, a cyclic C₃-C₂₀ alkyl, a linear C₁-C₁₀ alkyl, a branched C₄-C₁₀ alkyl, a cyclic C₃-C₁₀ alkyl, a linear C₁-C₆ alkyl, a branched C₄-C₆ alkyl, a cyclic C₃-C₆ alkyl, a linear C₁-C₄ alkyl, cyclic C₃-C₄ alkyl, such as a linear C₁-C₁₀, C₁-C₉, C₁-C₈, C₁-C₇, C₁-C₆, C₁-C₅, C₁-C₄, C₁-C₃, or C₁-C₂ alkyl group, a branched C₃-C₉, C₃-C₉, C₃-C₈, C₃-C₇, C₃-C₆, C₃-C₅, or C₃-C₄ alkyl group, or a cyclic C₃-C₉, C₃-C₉, C₃-C₈, C₃-C₇, C₃-C₆, C₃-C₅, or C₃-C₄ alkyl group. The cyclic alkyl can be a monocyclic or polycyclic alkyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkyl group.

For any of the formulae described herein, the alkenyl (when present) can be a linear alkenyl, a branched alkenyl, or a cyclic alkenyl (either monocyclic or polycyclic). The terms "cyclic alkenyl" and "cycloalkenyl" are used interchangeably herein. Exemplary alkenyl include a linear C₂-C₃₀ alkenyl, a branched C₄-C₃₀ alkenyl, a cyclic C₃-C₃₀ alkenyl, a linear C₂-C₂₀ alkenyl, a branched C₄-C₂₀ alkenyl, a cyclic C₃-C₂₀ alkenyl, a linear C₂-C₁₀ alkenyl, a branched C₄-C₁₀ alkenyl, a cyclic C₃-C₁₀ alkenyl, a linear C₂-C₆ alkenyl, a branched C₄-C₆ alkenyl, a cyclic C₃-C₆ alkenyl, a linear C₂-C₄ alkenyl, cyclic C₃-C₄ alkenyl, such as a linear C₂-C₁₀, C₂-C₉, C₂-C₈, C₂-C₇, C₂-C₆, C₂-C₅, C₂-C₄, C₂-C₃ alkenyl group, a branched C₃-C₉, C₃-C₉, C₃-C₈, C₃-C₇, C₃-C₆, C₃-C₅, C₃-C₄ alkenyl group, or a cyclic C₃-C₉, C₃-C₉, C₃-C₈, C₃-C₇, C₃-C₆, C₃-C₅, C₃-C₄ alkenyl group. The cyclic alkenyl can be a monocyclic or polycyclic alkenyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkenyl group.

For any of the formulae described herein, the alkynyl (when present) can be a linear alkynyl, a branched alkynyl, or a cyclic alkynyl (either monocyclic or polycyclic). The terms "cyclic alkynyl" and "cycloalkynyl" are used interchangeably herein. Exemplary alkynyl include a linear C₂-C₃₀ alkynyl, a branched C₄-C₃₀ alkynyl, a cyclic C₃-C₃₀ alkynyl, a linear C₂-C₂₀ alkynyl, a branched C₄-C₂₀ alkynyl, a cyclic C₃-C₂₀ alkynyl, a linear C₂-C₁₀ alkynyl, a branched C₄-C₁₀ alkynyl, a cyclic C₃-C₁₀ alkynyl, a linear C₂-C₆ alkynyl, a branched C₄-C₆ alkynyl, a cyclic C₃-C₆ alkynyl, a linear C₁-C₄ alkynyl, cyclic C₃-C₄ alkynyl, such as a linear C₂-C₁₀, C₂-C₉, C₂-C₈, C₂-C₇, C₂-C₆, C₂-C₅, C₂-C₄, C₂-C₃ alkynyl group, a branched C₃-C₉, C₃-C₉, C₃-C₈, C₃-C₇, C₃-C₆, C₃-C₅, C₃-C₄ alkynyl group, or a cyclic C₃-C₉, C₃-C₉, C₃-C₈, C₃-C₇, C₃-C₆, C₃-C₅, C₃-C₄ alkynyl group. The cyclic alkynyl can be a monocyclic or polycyclic alkynyl, such as a C₄-C₃₀, C₄-C₂₅, C₄-C₂₀, C₄-C₁₈, C₄-C₁₆, C₄-C₁₅, C₄-C₁₄, C₄-C₁₃, C₄-C₁₂, C₄-C₁₀, C₄-C₉, C₄-C₈, C₄-C₇, C₄-C₆, or C₄-C₅ monocyclic or polycyclic alkynyl group.

For any of the formulae described herein, the aryl (when present) can be a C₄-C₃₀ aryl, a C₄-C₂₀ aryl, a C₄-C₁₂ aryl, a C₄-C₁₁ aryl, a C₄-C₉ aryl, a C₅-C₃₀ aryl, a C₅-C₂₀ aryl, a C₅-C₁₂ aryl, a C₅-C₁₁ aryl, a C₅-C₉ aryl, a C₆-C₂₀ aryl, a C₆-C₁₂ aryl, a C₆-C₁₁ aryl, or a C₆-C₉ aryl. It is understood that the aryl can be a heteroaryl, such as a C₄-C₃₀ heteroaryl, a C₄-C₂₀ heteroaryl, a C₄-C₁₂ heteroaryl, a C₄-C₁₁ heteroaryl, a C₄-C₉ heteroaryl, a C₅-C₃₀ heteroaryl, a C₅-C₂₀ heteroaryl, a C₅-C₁₂ heteroaryl, a C₅-C₁₁ heteroaryl, a C₅-C₉ heteroaryl, a C₆-C₃₀ heteroaryl, a C₆-C₂₀ heteroaryl, a C₆-C₁₂ heteroaryl, a C₆-C₁₁ heteroaryl, or a C₆-C₉ heteroaryl. For any of the formulae described herein, the polyaryl group can be a C₈-C₃₀ polyaryl, a C₈-C₂₀ polyaryl, a C₈-C₁₂ polyaryl, a C₈-C₁₁ polyaryl, a C₁₀-C₃₀ polyaryl, a C₁₀-C₂₀ polyaryl, a C₁₀-C₁₂ polyaryl, a C₁₀-C₁₁ polyaryl, or a C₁₂-C₂₀ polyaryl. It is understood that the aryl can be a polyheteroaryl, such as a C₁₀-C₃₀ polyheteroaryl, a C₁₀-C₂₀ polyheteroaryl, a C₁₀-C₁₂ polyheteroaryl, a C₁₀-C₁₁ polyheteroaryl, or a C₁₂-C₂₀ polyheteroaryl.

Exemplary RhoA inhibitors are presented below.

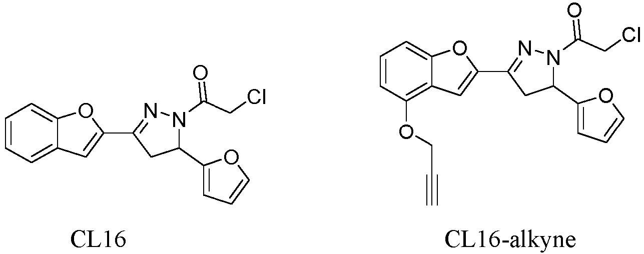

The compounds described herein may be neutral or may be one or more pharmaceutically acceptable salts, crystalline forms, non-crystalline forms, hydrates, or solvates, or a combination thereof. References to the compounds may refer to the neutral molecule, and/or those additional forms thereof collectively and individually from the context. Pharmaceutically acceptable salts of the compounds include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminum, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

The compounds described herein may be in a prodrug form. The term "prodrug" refers to a pharmacologically inactive substance that is converted in the body (e.g., via enzymatic or non-enzymatic action, including pH dependent bioactivation) into a pharmacologically active drug. In certain forms of such prodrugs, the prodrug may contain the drug promoeity linked to an auxophore in a prodrug designed to take advantage of cellular transporters (e.g., wherein the auxophore is a saccharide or disaccharide, or an amino acid or dipeptide). Such a prodrug may exhibit enhanced active transport across cellular membranes in the body; alternatively such a prodrug may inhibit efflux of drug and prodrug via interaction with cellular efflux transporters in the body. Exemplary groups that can convert the disclosed compounds to a prodrug form include, but are not limited to, hydroxyl, carboxyl, amine, phosphate, phosphonate, amidine, guanine and/or carbohydrate. These can be attached to any suitable position of the disclosed compounds to form a prodrug of the compounds.

The *in vitro* and *in vivo* inhibitory activity and anti-cancer effect of the RhoA inhibitors disclosed herein can be evaluated by using methods known in the art. For example, the *in vitro* inhibitory activity of the disclosed RhoA inhibitors can be obtained by measuring cell viability of 2D and 3D cell culture models using MTT assay, wound healing assay, and/or Matrigel invasion assay. For example, the *in vitro* anti-cancer effect of the disclosed RhoA inhibitors can be obtained by measuring tumor volume, tumor size, and/or tumor weight of mouse xenograft models. Specific examples for evaluating the *in vitro* and *in vivo* inhibitory activity of the RhoA inhibitors are described in the Examples below.

### B. Pharmaceutical Composition Containing RhoA Inhibitors

Pharmaceutical compositions that contain one or more of the RhoA inhibitors described herein in a form suitable for *in vitro* or *in vivo* applications (such as administration to a mammal), are disclosed. In some forms, the pharmaceutical composition that contains one or more of the RhoA inhibitors is for treating a cancer in a subject. The pharmaceutical composition may include one or more pharmaceutically acceptable carriers and/or one or more pharmaceutically acceptable excipients. For example, the pharmaceutical composition may be in the form of a liquid, such as a solution or a suspension, and contain a plurality of the disclosed RhoA inhibitors in an aqueous medium and, optionally, one or more suitable excipients for the liquid composition. Optionally, the pharmaceutical composition is in a solid form, and contains a plurality of the disclosed RhoA inhibitors and one or more suitable excipients for a solid composition.

### 1. Carriers and Excipients

Suitable pharmaceutically acceptable carriers and excipients are generally recognized as safe (GRAS), and may be administered to an individual without causing undesirable biological side effects or unwanted interactions.

Representative carriers and excipients include solvents (including buffers), diluents, pH modifying agents, preservatives, antioxidants, suspending agents, wetting agents, viscosity modifiers, tonicity agents, and stabilizing agents, and a combination thereof.

Excipients can be added to a liquid or solid pharmaceutical composition (for *in vivo* or *in vitro* applications) to assist in sterility, stability (e.g. shelf-life), integration, and to adjust and/or maintain pH or isotonicity of the RhoA inhibitors in the pharmaceutical composition, such as diluents, pH modifying agents, preservatives, antioxidants, suspending agents, wetting agents, viscosity modifiers, tonicity agents, and stabilizing agents, and a combination thereof.

RhoA inhibitors for administering to a subject in need thereof, such as a mammal, can be dissolved or suspended in a suitable carrier to form a liquid pharmaceutical formulation, such as sterile saline, phosphate buffered saline (PBS), balanced salt solution (BSS), viscous gel, or other pharmaceutically acceptable carriers for administration. The pharmaceutical formulation may also be a sterile solution, suspension, or emulsion in a nontoxic, parenterally acceptable diluent or solvent.

### 2. Forms for Administration

The disclosed RhoA inhibitors can be formulated into a pharmaceutical composition, in a liquid form or a solid form, as a liquid formulation or a solid formulation for oral administration, mucosal administration (e.g. intravaginal administration, pulmonary administration, and/or administration to other mucosal surfaces), or parenteral administration (e.g. intramuscular administration, intravenous administration, intraperitoneal administration, and subcutaneous administration) to a subject.

### a. Oral Formulations

The pharmaceutical composition containing one or more of the disclosed RhoA inhibitors may be provided in a form suitable for oral administration to a subject, such as a mammal (i.e., an oral composition). Oral administration may involve swallowing, so that the RhoA inhibitors, optionally including additional active agent(s) in the pharmaceutical composition, enter the gastrointestinal tract, or buccal or sublingual administration may be employed by which the RhoA inhibitors enter the blood stream directly from the mouth.

Compositions suitable for oral administration include solid compositions such as tablets, capsules containing particulates, liquids, powders, lozenges (including liquid-filled lozenges), chews, multi- and nano-particulates, gels, solid solutions, liposomes, films, ovules, sprays, and liquid compositions.

Liquid compositions for oral administration include suspensions, solutions, syrups, and elixirs. Such oral compositions may be employed as fillers in soft or hard capsules and can contain one or more suitable carriers and/or excipients, for example, water, ethanol, polyethylene glycol, propylene glycol, chitosan polymers and chitosan derivatives (e.g. N-trimethylene chloride chitosan, chitosan esters, chitosan modified with hydrophilic groups, such as amino groups, carboxyl groups, sulfate groups, etc.), methylcellulose, a suitable oil, one or more emulsifying agents, and/or suspending agents. Liquid compositions for oral administration may also be prepared by the reconstitution of a solid, for example, from a sachet.

Optionally, the RhoA inhibitors are included in a fast-dissolving and/or fast-disintegrating dosage form.

For tablet or capsule dosage forms, in addition to the RhoA inhibitors described herein, tablets generally contain disintegrants, binders, diluents, surface active agents, lubricants, glidants, antioxidants, colourants, flavoring agents, preservatives, or taste masking agents, or a combination thereof.

Examples of suitable disintegrants for forming a table or capsule dosage form containing the RhoA inhibitors include, but are not limited to, sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant can have a concentration in a range from about 1 wt% to about 25 wt%, from about 5 wt% to about 20 wt% of the tablet or capsule dosage form containing the RhoA inhibitors.

Binders are generally used to impart cohesive qualities to a tablet composition containing the RhoA inhibitors. Suitable binders for forming a tablet or capsule formulation containing the RhoA inhibitors include, but are not limited to, microcrystalline cellulose, gelatin, sugars, polyethylene glycol, natural and synthetic gums, polyvinylpyrrolidone, pregelatinised starch, chitosan polymers and chitosan derivatives (e.g. N-trimethylene chloride chitosan, chitosan esters, chitosan modified with hydrophilic groups, such as amino groups, carboxyl groups, sulfate groups, etc.), hydroxypropyl cellulose, and hydroxypropyl methylcellulose.

Suitable diluents for forming a table or capsule composition containing the RhoA inhibitors include, but are not limited to, lactose (as, for example, the monohydrate, spray-dried monohydrate or anhydrous form), chitosan polymers and chitosan derivatives (e.g. N-trimethylene chloride chitosan, chitosan esters, chitosan modified with hydrophilic groups, such as amino groups, carboxyl groups, sulfate groups, etc.), N-sulfonated derivatives of chitosan, quaternarized derivatives of chitosan, carbosyalkylated chitosan, microcrystalline chitosan, mannitol, xylitol, dextrose, sucrose, sorbitol, microcrystalline cellulose, starch and dibasic calcium phosphate dihydrate.

Tablet or capsule composition containing the RhoA inhibitors may also contain surface active agents, such as sodium lauryl sulfate and polysorbate 80, and glidants such as silicon dioxide and talc, in the coating. When present, surface active agents can have a concentration in a range from about 0.2 wt% to 5 wt% of the tablet or capsule formulation.

Tablet or capsule compositions containing the RhoA inhibitors also generally contain lubricants, such as magnesium stearate, calcium stearate, zinc stearate, sodium stearyl fumarate, and mixtures of magnesium stearate with sodium lauryl sulphate. Lubricants can have a concentration in a range from about 0.25 wt% to 10 wt%, from about 0.5 wt% to about 3 wt% of the tablet or capsule composition.

Other possible excipients included in a tablet or capsule formulation containing the RhoA inhibitors include glidants (e.g. Talc or colloidal anhydrous silica at about 0.1 wt% to about 3 wt% of the table or capsule formulation), antioxidants, colourants, flavouring agents, preservatives and taste-masking agents. When present, glidants can have a concentration in a range from about 0.2 wt% to 1 wt% of the tablet or capsule composition.

An exemplary tablet composition contains up to about 80 wt% of the RhoA inhibitors described herein, from about 10 wt% to about 90 wt% binder, from about 0 wt% to about 85 wt% diluent, from about 2 wt% to about 10 wt% disintegrant, and from about 0.25 wt% to about 10 wt % lubricant.

Tablet or capsule blends, including the RhoA inhibitors and one or more suitable excipients, may be compressed directly or by roller to form tablets. Tablet or capsule blends or portions of the blends may alternatively be wet-, dry-, or melt-granulated, melt congealed, or extruded before tableting. The final table or capsule composition may contain one or more layers and may be coated or uncoated; it may even be encapsulated in a particle, such as a polymeric particle or a liposomal particle.

Solid formulations containing the RhoA inhibitors for oral administration may be formulated to be immediate and/or modified release. Modified release formulations include delayed, sustained, pulsed, controlled, targeted and programmed release formulations.

### b. Mucosal Formulation

The disclosed RhoA inhibitors can be formulated for pulmonary or mucosal administration. The administration can include delivery of the composition to the lungs, nasal, oral (sublingual, buccal), vaginal, or rectal mucosa.

For example, the RhoA inhibitors can also be administered intranasally or by oral inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as water, ethanol -water mixture, 1,1,1,2-tetrafluoroethane or 1, 1, 1,2,3,3,3-heptafluoropropane. For intranasal or oral inhalation use, the powder may contain a bioadhesive agent, for example, chitosan or cyclodextrin. The term "aerosol" as used herein refers to any preparation of a fine mist of particles, which can be in solution or a suspension, whether or not it is produced using a propellant. Aerosols can be produced using standard techniques, such as ultrasonication or high-pressure treatment.

The pressurized container, pump, spray, atomizer, or nebulizer contains a solution or suspension of one or more of the RhoA inhibitors including, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Capsules (made, for example, from gelatin or hydroxypropylmethylcellulose), blisters and cartridges for use in an inhaler or insufflator may be formulated to contain a powder mix of the RhoA inhibitors described herein, a suitable powder base such as lactose or starch and a performance modifier such as 1-leucine, mannitol, or magnesium stearate. The lactose may be anhydrous or in the form of a monohydrate, preferably the latter. Other suitable excipients include dextran, glucose, maltose, sorbitol, xylitol, fructose, sucrose, and trehalose.

A suitable solution formulation for use in an atomizer using electrohydrodynamics to produce a fine mist may contain from 1 µg to 20 mg of one or more of the RhoA inhibitors per actuation and the actuation volume may vary from 1 µl to 100 µl. A typical formulation may contain a plurality of the RhoA inhibitors disclosed herein, propylene glycol, sterile water, ethanol and sodium chloride. Alternative solvents that may be used instead of propylene glycol include glycerol and polyethylene glycol.

Suitable flavors, such as menthol and levomenthol, or sweeteners, such as saccharin or saccharin sodium, may be added to those formulations intended for inhaled/intranasal administration.

Formulations for inhaled/intranasal administration may be formulated to be immediate and/or modified release using for example, PGLA. Modified release formulations include delayed, sustained, pulsed, controlled, targeted, and programmed release formulations.

In the case of dry powder inhalers and aerosols, the dosage unit is determined by means of a valve which delivers a metered amount. Units in accordance with the RhoA inhibitors are typically arranged to administer a metered dose or "puff". The overall daily dose will be administered in a single dose or, more usually, as divided doses throughout the day.

In some forms, the RhoA inhibitors can be formulated for pulmonary delivery, such as intranasal administration or oral inhalation. Carriers for pulmonary formulations can be divided into those for dry powder formulations and for administration as solutions. Aerosols for the delivery of therapeutic agents to the respiratory tract are known in the art. For administration via the upper respiratory tract, the RhoA inhibitors can be formulated into an aqueous solution, e.g., water or isotonic saline, buffered or un-buffered, or as an aqueous suspension, for intranasal administration as drops or as a spray. Such aqueous solutions or suspensions may be isotonic relative to nasal secretions and of about the same pH, ranging e.g., from about pH 4.0 to about pH 7.4 or, from pH 6.0 to pH 7.0. Buffers should be physiologically compatible and include, simply by way of example, phosphate buffers. One skilled in the art can readily determine a suitable saline content and pH for an innocuous aqueous solution for nasal and/or upper respiratory administration.

In some forms, the aqueous solution is water, physiologically acceptable aqueous solutions containing salts and/or buffers, such as phosphate buffered saline (PBS), or any other aqueous solution acceptable for administration to an animal or human. Such solutions are well known to a person skilled in the art and include, but are not limited to, distilled water, de-ionized water, pure or ultrapure water, saline, phosphate-buffered saline (PBS). Other suitable aqueous vehicles include, but are not limited to, Ringer's solution and isotonic sodium chloride. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

In some forms, solvents that are low toxicity organic (i.e. nonaqueous) class 3 residual solvents, such as ethanol, acetone, ethyl acetate, tetrahydrofuran, ethyl ether, and propanol may be used for the formulations. The solvent is selected based on its ability to readily aerosolize the formulation. The solvent should not detrimentally react with the RhoA inhibitors. An appropriate solvent should be used that forms a suspension of the RhoA inhibitors. The solvent should be sufficiently volatile to allow formation of an aerosol of the solution or suspension. Additional solvents or aerosolizing agents, such as freons, can be added as desired to increase the volatility of the solution or suspension.

In some forms, the pharmaceutical formulations may contain minor amounts of surfactants or other excipients well known to those of the art. In this context, "minor amounts" means no excipients are present that might affect or mediate penetration of the RhoA inhibitors in tissues and that the excipients that are present in amount that do not adversely affect penetration of the RhoA inhibitors in tissues.

In some forms, the pharmaceutical formulations containing one or more of the RhoA inhibitors can be administered directly to the mucous membranes (including the surface membranes of the nose, lungs and mouth), such that RhoA inhibitors cross the mucosal layer and enters the underlying tissues.

Such formulations for direct application on the mucous membrane generally contain a dermatologically acceptable carrier that is suitable for application to the mucous membrane, has good aesthetic properties, is compatible with the active agents and any other components, and will not cause any untoward safety or toxicity concerns.

The carrier can be in a wide variety of forms. For example, emulsion carriers, including, but not limited to, oil-in-water, water-in-oil, water-in-oil-in-water, and oil-in-water-in-silicone emulsions, are useful herein. These emulsions can cover a broad range of viscosities, e.g., from about 100 cps to about 200,000 cps. These emulsions can also be delivered in the form of sprays using either mechanical pump containers or pressurized aerosol containers using conventional propellants. These carriers can also be delivered in the form of a mousse or a transdermal patch. Other suitable topical carriers include anhydrous liquid solvents such as oils, alcohols, and silicones (e.g., mineral oil, ethanol isopropanol, dimethicone, cyclomethicone, and the like); aqueous-based single phase liquid solvents (e.g., hydro-alcoholic solvent systems, such as a mixture of ethanol and/or isopropanol and water); and thickened versions of these anhydrous and aqueous-based single phase solvents (e.g. where the viscosity of the solvent has been increased to form a solid or semi-solid by the addition of appropriate gums, resins, waxes, polymers, salts, and the like). Examples of topical carrier systems useful in the present formulations are described in the following four references all of which are incorporated herein by reference in their entirety: "Sun Products Formulary" Cosmetics & Toiletries, vol. 105, pp. 122-139 (December 1990); "Sun Products Formulary," Cosmetics & Toiletries, vol. 102, pp. 117-136 (March 1987); U.S. Pat. No. 5,605,894 to Blank et al., and U.S. Pat. No. 5,681,852 to Bissett.

Formulations for direct application on the mucous membrane may be formulated to be immediate and/or modified release. Modified release formulations include delayed, sustained, pulsed, controlled, targeted and programmed release formulations. Thus, the RhoA inhibitors may be formulated as a solid, semi-solid, or thixotropic liquid for administration as an implanted depot providing modified release of the active agents. Examples of such formulations include drug-coated stents.

### c. Parenteral Formulations

Optionally, the pharmaceutical composition containing one or more of the disclosed RhoA inhibitors is in a form suitable for administration directly into the blood stream, into muscle, or into an internal organ. Suitable routes for such parenteral administration include intravenous, intraarterial, intraperitoneal, intrathecal, epidural, intracerebroventricular, intraurethral, intrasternal, intracranial, intramuscular, and subcutaneous delivery. Suitable means for parenteral administration include needle (including microneedle) injectors, needle-free injectors, and infusion techniques.

For example, the pharmaceutical formulation containing one or more of the RhoA inhibitors is in a form suitable for intramuscular administration, intravenous administration, intraperitoneal administration, or subcutaneous administration, or a combination thereof.

Parenteral formulations containing the RhoA inhibitors described herein are typically aqueous solutions which can contain excipients such as salts, carbohydrates and buffering agents (e.g., from about pH 6.5 to about pH 8.0, from about pH 6.5 to about pH 7.4, from about pH 6.5 to about pH 7.0, from about pH 7.0 to pH 8.0, or from about pH 7.0 to about pH 7.4), but, for some applications, they may be more suitably formulated as a sterile aqueous solution or as a dried form to be used in conjunction with a suitable vehicle such as sterile, pyrogen-free water.

The liquid compositions containing the RhoA inhibitors for parenteral administration may be a solution, a suspension, or an emulsion.

The liquid pharmaceutically acceptable carrier forming the parenteral composition containing the RhoA inhibitors can include one or more physiologically compatible buffers, such as a phosphate buffers. One skilled in the art can readily determine a suitable saline content and pH for an aqueous carrier for administration (e.g., from about pH 6.5 to about pH 8.0, from about pH 6.5 to about pH 7.4, from about pH 6.5 to about pH 7.0, from about pH 7.0 to pH 8.0, or from about pH 7.0 to about pH 7.4).

Liquid compositions containing the RhoA inhibitors for parenteral administration may include one or more suspending agents, such as cellulose derivatives, sodium alginate, polyvinylpyrrolidone, gum tragacanth, or lecithin. The liquid compositions may also include one or more preservatives, such as ethyl or n-propyl p-hydroxybenzoate.

Optionally, the liquid composition containing the RhoA inhibitors contains one or more solvents that are low toxicity organic (i.e., nonaqueous) class 3 residual solvents, such as ethanol, acetone, ethyl acetate, tetrahydofuran, ethyl ether, and propanol, and a combination thereof. Any such solvents included in the liquid formulation should not detrimentally react with the RhoA inhibitors or any additional active agents in the liquid composition. Solvents such as freon, alcohol, glycol, polyglycol, or fatty acid, can also be included in the liquid composition containing the RhoA inhibitors as desired to increase the volatility of the solution or suspension.

Liquid compositions containing the RhoA inhibitors for parenteral administration may also contain minor amounts of polymers, surfactants, or other pharmaceutically acceptable excipients known to those in the art. In this context, "minor amounts" means an amount that is sufficiently small to avoid adversely affecting uptake of the RhoA inhibitors by the targeted cells, such as pituitary gonadotrophs.

The preparation of parenteral compositions containing the RhoA inhibitors is typically under sterile conditions, for example, by lyophilisation, which can be accomplished using standard pharmaceutical techniques known to those skilled in the art.

Compositions for parenteral administration containing the RhoA inhibitors may be formulated to provide immediate and/or modified release of the active agent. Modified release formulations include delayed, sustained, pulsed, controlled, targeted and programmed release formulations.

### d. Amount of RhoA inhibitors

Pharmaceutical compositions typically contain an effective amount of the RhoA inhibitors and one or more pharmaceutically acceptable carriers and/or excipients. As used herein, the term "effective amount" means any amount of the RhoA inhibitors that is sufficient to achieve the desired therapeutic, prophylactic, and/or diagnostic effect on a biological sample or in a subject to which it is administered. For example, the pharmaceutical composition contains an effective amount of the RhoA inhibitors to ameliorate one or more symptoms associated with a cancer in a subject.

Depending on the specific condition to be treated and/or the route of administration, such an effective amount of the RhoA inhibitors can be from about 0.01 mg/kg to about 250 mg/kg body weight, from about 0.1 mg/kg to about 250 mg/kg body weight, from about 0.01 mg/kg to about 200 mg/kg body weight, from about 0.1 mg/kg to about 200 mg/kg body weight, from about 0.01 mg/kg to about 150 mg/kg body weight, from about 0.1 mg/kg to about 150 mg/kg body weight, from about 0.01 mg/kg to about 100 mg/kg body weight, from about 0.1 mg/kg to about 100 mg/kg body weight, from about 0.01 mg/kg to about 50 mg/kg body weight, from about 0.1 mg/kg to about 50 mg/kg body weight, from about 0.01 mg/kg to about 10 mg/kg body weight, from about 0.1 mg/kg to about 10 mg/kg body weight, from about 0.2 mg/kg to about 20 mg/kg body weight, or from about 0.5 mg/kg to about 50 mg/kg body weight, of the subject per day, which can be administered as a single daily dose, divided over one or more daily doses.

For example, the effective amount of the RhoA inhibitors is about 0.1 mg/kg, about 0.5 mg/kg, about 1 mg/kg, about 10 mg/kg, about 20 mg/kg, about 30 mg/kg, about 40 mg/kg, about 50 mg/kg, about 60 mg/kg, about 70 mg/kg, about 80 mg/kg, about 90 mg/kg, about 100 mg/kg, about 110 mg/kg, about 120 mg/kg, about 130 mg/kg, about 140 mg/kg, about 150 mg/kg, about 160 mg/kg, about 170 mg/kg, about 180 mg/kg, about 190 mg/kg, about 200 mg/kg, about 210 mg/kg, about 220 mg/kg, about 230 mg/kg, about 240 mg/kg, or about 250 mg/kg, of the subject per day, which can be administered as a single daily dose, divided over one or more daily doses. The amount of the RhoA inhibitors administered, the route of administration, and the further treatment regimen can be determined by the treating clinician or testing technologies, depending on factors such as the age, gender and general condition of the subject, the nature and severity of the disease/symptoms being prevented, treated, or diagnosed, and/or the samples being tested.

In some forms, the pharmaceutical composition is in a unit dosage form, and can be suitably packaged, for example in a box, blister, vial, bottle, sachet, ampoule or in any other suitable single-dose or multi-dose holder or container (which can be properly labeled); optionally with one or more leaflets containing product information and/or instructions for use. Generally, such unit dosages can contain from 0.01 to 1000 mg, such as from 0.01 to 500 mg, from 0.01 to 100 mg, from 0.01 to 50 mg, from 0.01 to 10 mg, from 0.01 to 1 mg, from 0.1 to 500 mg, from 0.1 to 100 mg, from 0.1 to 50 mg, from 0.1 to 10 mg, or from 0.1 to 1 mg, of the disclosed RhoA inhibitors, e.g., about 0.1, 0.2, 0.5, 1, 5, 10, 25, 50, 100, 200, 300 or 400 mg per unit dosage.

### e. Additional Active Agent

In some forms, the pharmaceutical composition containing the RhoA inhibitors also contains one or more additional active agent(s), such as one or more additional anticancer agent(s). For example, one or more additional active agents is formulated into the same pharmaceutical composition containing one or more of the RhoA inhibitors described herein as a single dosage unit or as multiple dosage units for coordinated, combination, or concomitant administration, or into separate pharmaceutical compositions for combinational therapy.

Alternatively, the one or more additional active agents may be formulated as separate pharmaceutical compositions, for example, as a single dosage unit or as multiple dosage units, for co-administration with the pharmaceutical composition containing one or more of the RhoA inhibitors described herein. When formulated into a pharmaceutical composition that is separated from the pharmaceutical composition containing the disclosed RhoA inhibitors, the one or more additional active agents may be formulated in various formulations, for example, injectable formulations, lyophilized formulations, liquid formulations, or oral formulations. The formulation can be selected based upon the suitable administration route.

Examples of anticancer agents that can be used in the pharmaceutical compositions containing the RhoA inhibitors or a separate pharmaceutical composition include, but are not limited to, temozolomide, carmustine, bevacizumab, procarbazine, lomustine, vincristine, gefitinib, erlotinib, cisplatin, carboplatin, oxaliplatin, 5-fluorouracil, gemcitabine, tegafur, raltitrexed, methotrexate, cytosine arabinoside, hydroxyurea, adriamycin, bleomycin, doxorubicin, daunomycin, epirubicin, idarubicin, mitomycin-C, dactinomycin, mithramycin, vinblastine, vindesine, vinorelbine, paclitaxel, taxol, docetaxel, etoposide, teniposide, amsacrine, topotecan, camptothecin, bortezomib, anagrelide, tamoxifen, toremifene, raloxifene, droloxifene, iodoxyfene, fulvestrant, bicalutamide, flutamide, nilutamide, cyproterone, goserelin, leuprorelin, buserelin, megestrol, anastrozole, letrozole, vorozole, exemestane, finasteride, marimastat, trastuzumab, cetuximab, dasatinib, imatinib, combretastatin, thalidomide, azacitidine, azathioprine, capecitabine, chlorambucil, cyclophosphamide, cytarabine, daunorubicin, doxifluridine, epothilone, irinotecan, mechlorethamine, mercaptopurine, mitoxantrone, pemetrexed, tioguanine, valrubicin and/or lenalidomide or combinations thereof such as cyclophosphamide, methotrexate, 5-fluorouracil (CMF); doxorubicin, cyclophosphamide (AC); mustine, vincristine, procarbazine, prednisolone (MOPP); sdriamycin, bleomycin, vinblastine, dacarbazine (ABVD); cyclophosphamide, doxorubicin, vincristine, prednisolone (CHOP); bleomycin, etoposide, cisplatin (BEP); epirubicin, cisplatin, 5-fluorouracil (ECF); epirubicin, cisplatin, capecitabine (ECX); and methotrexate, vincristine, doxorubicin, cisplatin (MVAC); and combinations thereof. Additional anticancer agents are described in U.S. Patent No. 10,393,736, the disclosure of which is incorporated herein by reference in its entirety.

### III. Methods of Use

The disclosed RhoA inhibitors are suitable for use in cancer therapy. In particular, the RhoA inhibitors have high inhibitory efficacy and excellent binding specificity, which allow them to outperform the reported RhoA inhibitors, Rhosin and DC-Rhoin. For example, the RhoA inhibitors disclosed herein can bind strongly onto RhoA *in vitro* with an IC₅₀ value down to about 400 nM. Such a strong binding allows CL16 to inhibit RhoA in cancer cells with high efficacy. For example, the RhoA inhibitory effect is significantly higher than the reported Rhosin. For example, as demonstrated by data in the Examples, a lower RhoA-GTP level was observed in cells treated with 10 µM of CL16 than those treated with 30 µM of Rhosin. For example, in contrast to DC-Rhoin that binds RhoA, Rac1, and Cdc42, the RhoA inhibitors disclosed herein can specifically bind RhoA over other GTPases, such as other Ras GTPases (e.g., K-Ras and N-Ras) and particularly other Rho GTPases (e.g., Cdc42 and Rac1). This allows the disclosed RhoA inhibitors to show high cytotoxicity in cancer cells, with negligible toxicity in normal cells, both *in vitro* and *in vivo.* Prior to the disclosed RhoA covalent inhibitors, there were no RhoA covalent inhibitors having *in vivo* antitumor effects on mouse colorectal cancer xenograft models with good efficacy.

In some forms, the disclosed RhoA inhibitors or pharmaceutical composition containing the RhoA inhibitors can be used for treating a cancer. In some forms, the cancer is a solid cancer (also referred to herein as a "tumor"), such as melanoma, gastric cancer, breast cancer, and colorectal cancer. "Treatment" or "treating" refers to the medical management of a patient with the intent to cure, ameliorate, stabilize, or prevent a disease, pathological condition, or disorder, such as a cancer. This term includes active treatment, that is, treatment directed specifically toward the improvement of a disease, pathological condition, or disorder, such as a cancer; and also includes causal treatment, that is, treatment directed toward removal of the cause of the associated disease, pathological condition, or disorder, such as a cancer. In addition, this term includes palliative treatment, that is, treatment designed for the relief of symptoms rather than the curing of the disease, pathological condition, or disorder, such as a cancer; preventative treatment, that is, treatment directed to minimizing or partially or completely inhibiting the development of the associated disease, pathological condition, or disorder, such as a cancer; and supportive treatment, that is, treatment employed to supplement another specific therapy directed toward the improvement of the associated disease, pathological condition, or disorder, such as a cancer.

In some forms, a method for treating cancer, such as a solid cancer, using a pharmaceutical composition containing the disclosed RhoA inhibitors includes: (i) administering the pharmaceutical composition to a subject in need thereof.

The administration in step (i) can be performed using any suitable technique, such as oral administration, intranasal administration, intramuscular administration, intravenous administration, intraperitoneal administration, or subcutaneous administration, or a combination thereof.

Step (i) (administration of the pharmaceutical composition) of the disclosed method may occur one or more times. When more than one administration step is performed, each administration can be performed regularly every 5 mins, every 10 mins, every 20 mins, every 30 mins, every hour, every 2 hours, every day, every two days, every 3 days, every week, every two weeks, every month, etc.; or irregularly with a time interval of 5 mins, 10 mins, 20 mins, 30 mins, 1 hour, 2 hours, 1 day, 2 days, 3 days, 5 days, 1 week, 2 weeks, etc. The specific number of treatment and time interval for the treatment can be determined by the treating clinician or testing technologies, depending on factors such as the age, gender and general condition of the subject, the nature and severity of the disease/symptoms being prevented, treated, or diagnosed, and/or the samples being tested.

Typically, following the administration step or all of the administration steps, an effective amount of the RhoA inhibitors in the pharmaceutical formulation is administered to the subject, such that cancer cells are killed and/or growth or proliferation of the cancer cells are reduced or prevented, and thereby ameliorate one or more symptoms associated with the cancer in the subject, such as reduce the tumor volume and/or tumor weight.

For example, following the administration step or all of the administration steps (if the administration occurs more than one time), an effective amount of the RhoA inhibitors is administered to the subject, such that the tumor volume in the subject is at least 10%, at least 20%, at least 30%, at least 40%, or at least 50% smaller than a control that is administered with the pharmaceutically acceptable excipient(s) only. For example, following the administration step or all of the administration steps (if the administration occurs more than one time), an effective amount of the RhoA inhibitors is administered to the subject, such that the tumor volume in the subject does not change compared to before administration of the pharmaceutical composition or the increase of tumor volume compared to before administration of the pharmaceutical composition in the subject is less than the increase of tumor volume in a control that is administered with the pharmaceutically acceptable excipient(s) only.

For example, following the administration step or all of the administration steps (if the administration occurs more than one time), an effective amount of the RhoA inhibitors is administered to the subject, such that the tumor weight in the subject is at least 10%, at least 20%, at least 30%, at least 40%, or at least 50% less than a control that is administered with the pharmaceutically acceptable excipient(s) only.

For example, following the administration step or all of the administration steps (if the administration occurs more than one time), an effective amount of the RhoA inhibitors is administered to the subject, such that the tumor volume and the tumor weight in the subject is at least 10%, at least 20%, at least 30%, at least 40%, or at least 50% smaller/less than a control that is administered with the pharmaceutically acceptable excipient(s) only.

For example, following the administration step or all of the administration steps (if the administration occurs more than one time), an effective amount of the RhoA inhibitors is administered to the subject, such that the tumor volume in the subject does not change compared to before administration of the pharmaceutical composition or the increase of tumor volume compared to before administration of the pharmaceutical composition in the subject is less than the increase of tumor volume in a control that is administered with the pharmaceutically acceptable excipient(s) only; and the tumor weight in the subject is at least 10%, at least 20%, at least 30%, at least 40%, or at least 50% less than the control.

For example, following the administration step or all of the administration steps, the effective amount of the RhoA administered to the subject is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 2 µg to about 20 µg, or from about 5 µg to about 50 µg, per g of the subject.

The cancer treatment effect as described above can occur within 3 days, within 5 days, within 7 days, within 10 days, within 2 weeks, within one month, within three months, or within six months following the administration step or all of the administration steps. Optionally, the cancer treatment effect occurs without any observable toxicity to the subject, as indicated by body vital measurements (e.g., body weight), standard hematology markers, and/or blood biochemical parameters compared to the control.

In some forms, the disclosed method can further include administering one or more additional active agent(s), such as one or more additional anticancer agent(s) described above, to the subject before step (i) administering a pharmaceutical composition containing the RhoA inhibitors, simultaneously with step (i), or after step (i), or a combination thereof.

The disclosed compositions and methods can be further understood through the following enumerated paragraphs.
1. A compound having a structure of: 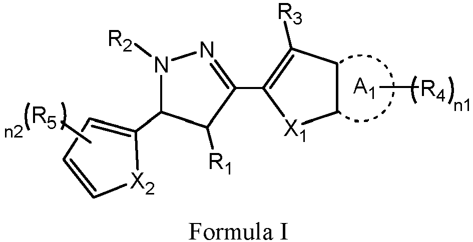 wherein:
   (i) X₁ and X₂ are independently C(R₆)₂, O, S, or NR₆ and R₆ is hydrogen, a substituted or unsubstituted alkyl, a substituted or unsubstituted alkenyl, a substituted or unsubstituted alkynyl, an alkoxyl, or a carbonyl;
   (ii) A₁ is a C₅-C₆ aromatic ring or a C₄-C₅ heteroaromatic ring;
   (iii) n1 is an integer from 0 to 4;
   (iv) n2 is an integer from 0 to 3;
   (v) R₁, R₃, and R₅ are independently hydrogen, a substituted or unsubstituted alkyl, or a substituted or unsubstituted aryl;
   (vi) R₂ is a cysteine-reactive group (such as haloacetamide, acrylamide, maleimide, vinyl sulfone and epoxide), which allows chemical reaction with cysteines on RhoA;
   (vii) R₄ is hydrogen, an alkoxyl, a hydroxyl, a halogen, a substituted or unsubstituted alkynyl, a substituted or unsubstituted aryl, a carbonyl, an amino, or a haloalkyl; and
   (viii) the substituents, when present, are independently unsubstituted alkyl, unsubstituted aryl, unsubstituted heteroaryl, unsubstituted polyaryl, unsubstituted heteropolyaryl, unsubstituted alkylaryl, unsubstituted cyclic, unsubstituted heterocyclic, halide, amino, amido, thiol, hydroxyl, cyano, nitro, carbonyl, or alkoxyl.
2. The compound of paragraph 1, having a structure of: 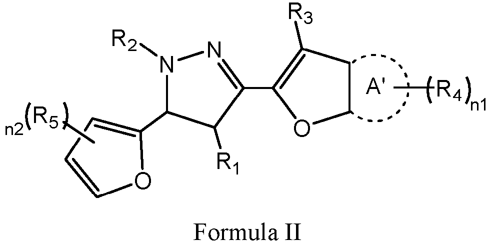
3. The compound of paragraph 1 or 2, having a structure of: 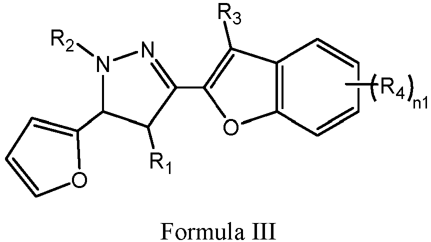
4. The compound of any one of paragraphs 1-3, wherein n₁ is 0 or 1.
5. The compound of any one of paragraphs 1-4, wherein R₁ is hydrogen.
6. The compound of any one of paragraphs 1-5, wherein R₃ is hydrogen or a substituted or unsubstituted aryl, wherein the substituents, when present, are independently an unsubstituted alkyl, an unsubstituted aryl, or an unsubstituted alkyl aryl.
7. The compound of any one of paragraphs 1-6, wherein R₂ is 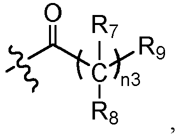 n3 is an integer from 0 to 6, R₇ and R₈ are independently hydrogen, an unsubstituted alkyl, or halogen, and R₉ is hydrogen or halogen.
8. The compound of any one of paragraphs 1-7, wherein R₄ is hydrogen or 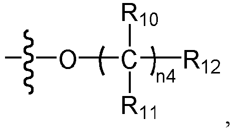 n4 is an integer from 0 to 6, R₁₀ and R₁₁ are independently hydrogen or halogen, and R₁₂ is hydrogen, halogen, amino, an unsubstituted alkynyl, aldehyde, or carboxylic acid.
9. The compound of paragraph 8, wherein R₁₀ and R₁₁ are hydrogen, and R₁₂ is an unsubstituted alkynyl.
10. The compound of any one of paragraphs 1-9, wherein the compound is not 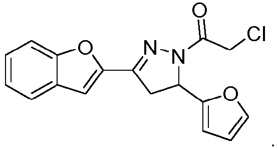
11. The compound of any one of paragraphs 1-10, wherein when X₁ is O, X₂ is S, R₁ and R₃-R₄ are hydrogen, and A₁ is a C₆ aromatic ring, then R₂ is not haloacetamide.
12. The compound of any one of paragraphs 1-9, having the structure of: 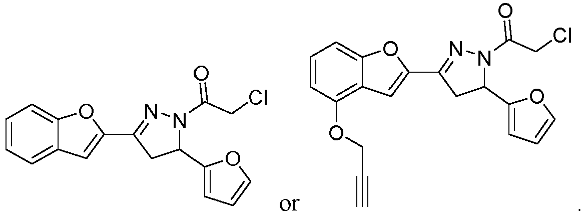
13. The compound of any one of paragraphs 1-12, having the structure of: 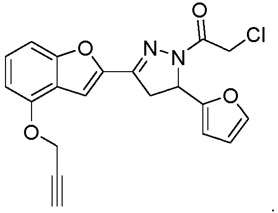
14. The compound of any one of paragraphs 1-13, wherein the compound covalently binds onto RhoA.
15. The compound of paragraph 14, wherein the compound covalently binds onto Cys16 of RhoA.
16. The compound of paragraph 14 or 15, wherein the compound inhibits RhoA activity in living cells.
17. A pharmaceutical composition comprising the compound of any one of paragraphs 1-16, and optionally one or more pharmaceutically acceptable excipients.
18. The pharmaceutical composition of paragraph 17, further comprising a second active agent, optionally wherein the second active agent is an anticancer agent.
19. The pharmaceutical composition of paragraph 17 or 18, wherein the compound in the pharmaceutical composition is in an effective amount to ameliorate one or more symptoms associated with a cancer in a subject.
20. The pharmaceutical composition of any one of claims 17-19, wherein the compound in the pharmaceutical composition is in an amount ranging from about 0.1 µM to about 1 mM, from about 0.1 µM to about 500 µM, from about 0.1 µM to about 200 µM, from about 0.1 µM to about 100 µM, from about 0.1 µM to about 50 µM, or from about 0.1 µM to about 20 µM.
21. A method for treating cancer using the pharmaceutical composition of any one of paragraphs 17-20 comprising:
   (i) administering the pharmaceutical composition to a subject in need thereof, wherein step (i) occurs one or more times.
22. The method of paragraph 21, wherein the pharmaceutical composition is administered by oral administration, intramuscular administration, intravenous administration, intraperitoneal administration, or subcutaneous administration, or a combination thereof.
23. The method of paragraph 21 or 22, wherein in step (i), the dosage of the compound administered is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject.
24. The method of any one of paragraphs 21-23, wherein the cancer is a solid cancer.
25. The method of any one of paragraphs 21-24, wherein the cancer is a colorectal cancer.
26. The method of paragraph 24 or 25, wherein following step (i) or all of step (i) (if step (i) occurs more than one time), the tumor volume and/or tumor weight is at least 10%, at least 20%, at least 30%, at least 40%, or at least 50% smaller/less than a control administered with the pharmaceutically acceptable excipient(s) only.
27. The method of any one of paragraphs 21-26, following step (i) or all of step (i) (if step (i) occurs more than one time), there is no observable toxicity to the subject, as indicated by body vital measurements (e.g., body weight), standard hematology markers, and/or blood biochemical parameters compared to the control.
28. The method of any one of paragraphs 21-27, further comprising administering an active agent prior to, during, and/or subsequent to step (i), optionally wherein the active agent is an anticancer agent.

The present invention will be further understood by reference to the following non-limiting examples.

### Examples

### Example 1. Synthetic RhoA covalent inhibitors that target Cys16 for cancer therapy

The design, synthesis, and biological activities of exemplary RhoA covalent inhibitors that target Cys16 for cancer therapy are provided. A chemoproteomics platform, ABPP, was used to discover RhoA covalent inhibitors. Through covalent targeting of RhoA Cys16, which has never been exploited as a binding site of drug compounds, an exemplary compound, CL16, showed selective binding onto RhoA, but not other Rho family proteins such as Rac1 and Cdc42. LC-MS/MS experiment showed covalent binding of CL16 on Cys16, which is adjacent to the nucleotide-binding pocket of RhoA. This covalent binding allowed CL16 to reside next to the nucleotide-binding pocket as shown in computational docking, and resulted in prohibition of GTP binding onto RhoA. The covalent binding of CL16 on RhoA Cys16 also disrupted RhoA-GEF interactions. The selective covalent binding of CL16 with RhoA resulted in a specific inhibition of RhoA activity through prohibition of GDP-to-GTP exchange *in vitro* and in cancer cells, as demonstrated using FRET-RhoA biosensor imaging, immunofluorescence, and western blotting experiments. For example, as demonstrated by the data below, the selective covalent binding of CL16 with RhoA led to inhibition of RhoA signaling in colorectal cancer (CRC) cells after CL16 treatment, resulting in high cytotoxicity and significant slowdown in cell migration and invasion of CRC with overexpression of RhoA. Particularly, CL16 treatment was shown as effective in shrinking CRC spheroid and decelerating tumor growth *in vivo* with no observable toxicity. These demonstrate the use of the disclosed RhoA covalent inhibitors as anti-cancer and anti-metastatic agents.

The RhoA inhibitory effects of CL16 were found to be more effective than the reported RhoA inhibitor, Rhosin. Further, the good efficacy in RhoA inhibition allowed CL16 to mediate anticancer and anti-metastatic properties in 2D cancer cell culture and 3D spheroid model with hyperactivity of RhoA, with much lower toxicity in normal cells. CL16 also showed potent *in vivo* anti-tumor effects in mouse cancer xenograft models without observable toxicity. It is believed that CL16 is the first RhoA covalent inhibitor having *in vivo* antitumor effects on mouse colorectal cancer xenograft models with good efficacy. A molecular probe of CL16, CL16-alkyne, was developed, which can function as a probe for RhoA to validate the specific binding of CL16 with RhoA. The results show that the RhoA inhibitors disclosed herein can be used for biological studies of RhoA functions and activities in cancers, and as drug candidates for targeting RhoA for cancer therapy.

### Materials and Methods

### Gel-based Competitive Activity-Based Protein Profiling (ABPP)

Recombinant human RhoA (0.1 µg; ab 101594) was pretreated with cysteine-reactive compounds or DMSO for 1 hour at room temperature, followed by incubation with iodoacetamide-rhodamine (IA-Rh; 1 µM) for 1 hour at room temperature. The reaction was then quenched by boiling in sampling buffer. Samples were subjected to SDS-PAGE to separate proteins bands. In-gel fluorescence intensity was measured with a Chemidoc using setting for Rhodamine. Gel was subsequently stained with silver stain (Pierce) for visualization of protein content and covalent modification. Band intensity was quantified using ImageJ.

### Covalent docking

Schrödinger Maestro 13.4 was used for covalent docking. Structure of GDP-bound RhoA (PDB:1CC0) was retrieved from Protein Database (PDB). The protein structure was prepared using the protein preparation wizard tool by adding missing side chain and loops, removing water protein, and adjusting hydrogen orientations at pH 7.0 using PROPKA. The protein was minimized by OPLS4 force field. CL16 ligand structure was prepared by LigPrep module, subjected to Epik at pH 7.0 (±2.0) then minimized by OPLS4 force field. Covalent docking was performed by CovDoc algorithm. Receptor had A: 16 (Cys16) residue chosen, with box size enabled docking of ligands of ≤20 Å length. Nucleophilic substitution was selected as reaction type. No constraints were imposed.

### Co-immunoprecipitation

10 µg of purified GST-RhoA was immobilized on Glutathione resin (Pierce) and incubated with CL16 in GTP-exchange buffer (20 mM HEPES, 150 mM NaCl, 5 mM MgCl₂, pH 7.4) for 1 hour. Then, the mixture was centrifuged at 700g for 2 min and supernatant was discarded. 120 µg of total cell lysate with overexpression of GEF (Arhgef1 p-115, Arhgef12 LARG DH-PH domain, Arhgef13 Akap Lbc) was added to the resin. The mixture was put on an end-to-end rotator and incubated at 4°C for 4 hours. The resin was then washed, eluted and subjected to immunoblotting.

### GDP-GTP exchange assay

Recombinant human RhoA protein (0.2 µg; ab101594) was incubated with GDP (50 nM) in 100 µL of GTP-exchange buffer (20 mM HEPES, 150 mM NaCl, 5 mM MgCl₂, pH 7.4) at room temperature for 1 h. CL16 or solvent control was then added to the solution mixture and incubated at room temperature for another hour. For self-exchange assay, BODIPY-GTP (100 nM) was added to the solution and the fluorescence from plate was measured every 30 s. For LARG-mediated GDP-to-GTP exchange, BODIPY-GTP (100 nM) and LARG DH-PH protein (30 nM) were added to the solution mixture and the fluorescence from plate was measured every 30 s. BODIPY-GTP was excited at 477±14 nm and its fluorescence was measured at 525±30 nm using a plate reader (Clariostar).

### RhoA GTP-rhotekin pull down

RhoA Pull-Down Activation Assay Biochem Kit (#BK036) was purchased from Cytoskeleton. According to the manufacturer's protocol, total cell lysate (0.5mg/mL; 300 µL) was added to 7.5 µL of Rhotekin-Rho Binding Domain beads in 0.6 mL tubes. The solution mixture was incubated at 4°C on an end-to-end rotator for 1 hour. Beads were washed with 500 µL of washing buffer once, then centrifuged at 5000g for 3 min and the supernatant was discarded. RhoA-GTP was then eluted in 2X laemmli buffer and resolved in 10% SDS-PAGE.

### Cell culture

Cell lines were cultured in a 37°C incubator with 5% CO₂. HT29 cells was cultured in DMEM (Gibco, with 10% FBS and 1% penicillin and streptomycin). SW620, SW480, LOVO, and HCT116 cells were cultured in RPMI (Gibco, with 10% FBS and 1% penicillin and streptomycin). CCD-18Co cells were cultured in MEM (Gibco, with 10% FBS and 1% penicillin and streptomycin). These cell lines were obtained from Prof. SY Leung and Dr. Helen Yan at the Department of Pathology, The University of Hong Kong.

### Gene Set Enrichment Analysis (GSEA)

Unstranded TPM values from open-access colorectal adenocarcinoma tumour tissue RNA-seq results obtained from TCGA were used for analysis and GSEA v4.3.3 Mac App was used for computation. In comparing stages 0-I and IV, the results were divided according to AJCC pathological stages and relevant samples were used for analysis. To compare high and low EMT scores, the EMT scores were computed for all samples using single sample gene set enrichment scores (ssGSEAs) through the hacksig R package. The scores were separated into two groups, relative to the median score.

### Immunoblotting

Anti-RhoA (#2117), anti-GAPDH (#2118), anti-phosphoMLC (#3671), anti-MLC (#3672), anti-FLAG (#8146), anti-GFP (#2956), and anti-GST (#2624) were purchased from Cell Signaling Technology. Cells were washed with PBS and then harvested using cell lysis buffer (1% Triton X100, 10 mM beta glycerol phosphate, 10mM sodium pyrophosphate, 40 mM HEPES, 4 mM EDTA, pH7.4) with 4% protease/phosphatase inhibitor cocktail (Pierce). Protein samples were quantified using BCA assays (Pierce), normalized and then boiled with 4X Laemmli sampling buffer in 95°C for 5mins. Samples were resolved in 10% polyacrylamide gel (Biorad) and then transferred onto PVDF membrane (Biorad) and blocked with blocking buffer (5% bovine serum albumin (BSA) in Tris-buffered saline with 0.1% Tween 20 (TBST)) for 1 h. Membrane was washed in TBST, followed by incubation with corresponding primary antibodies (diluted 1:1000 in 5% BSA/milk in TBST with 1% NaN₃) overnight at 4°C. After washing thrice with TBST, the membrane was incubated with HRP-conjugated secondary antibodies (#7074 or 7076) for one hour at room temperature. The membrane was then incubated with LumiGlo reagent (Cell Signaling Technology) and chemiluminescence was captured using a Chemidoc (Biorad).

### Rac1-GTP and Cdc42-GTP assay

The procedure is similar to that for RhoA-GTP assay, except that Rac1-GTP and Cdc42-GTP in CRC cells were first enriched by Rac1 Pull-Down Activation Assay Biochem Kit (Cytoskeleton #BK035) instead of using the RhoA kit. The enriched Rac1-GTP and Cdc42-GTP were then immunoblotted by Rac1/2/3 antibody (CST #2465) and Cdc42 antibody (CST #2462) respectively.

### MTT Assay

HCT116 cells was seeded at 2,500 cells/well; HT29, LOVO and SW620 cells were seeded at 4,000 cells/well; CCD-18Co cells were seeded at 10,000/well; and SW480 cells were seeded at 12,000 cells/well in 96 well plate in triplicates. Cells were treated with a concentration gradient of CL16 for 48 hours. Then, cells were incubated with 10% 3-(4,5-Dimethylthiazol-2-yl)-2,5-diphenyltetrazolium bromide (MTT) solution (50mg/mL) in 37°C for 4 hours. Cells were then lyzed in 10% SDS in 0.01M HCl. Absorbance of individual well was measured at 570nm. IC₅₀ values were analyzed by nonlinear best-fit regression using Prism Graphpad 9.

### Wound Healing andMatrigel Invasion Assays

For wound healing assay, HT29 cells were seeded in 90% confluency, a wound was scratched using a P-20 tip the next day. Cell debris was washed with PBS then aspirated. Then, complete DMEM medium with CL16 or Rhosin with corresponding concentration was added to the cells. Wound closure was recorded every 24 hours under light microscope with 10X objective and measurement of wound area was made by ImageJ.

For Matrigel invasion assay, SW620 cells (100,000 cells per well) in serum-free medium with CL16 or DMSO were seeded in a Boyden chamber. Full RPMI was loaded in lower chamber as chemoattractant. After 24 h, the bottom of Boyden chamber was fixed by 4% PFA for 10 min and then stained with crystal violet solution for another 10 min. Excess stain was washed out by tap water. Invaded cells were recorded under light microscope with 10X objective and areas with penetrated cells were measured by ImageJ.

### siRNA knockdown

A set of 4 siRNAs that target different *RHOA* sequences was purchased from Horizon (Horizon L-003560-00-0010). HT-29 (3×10⁶ cells per well) were transfected with 5 µM siRNA using Dharmafect reagent (Horizon) for 48 hours. Knockdown efficiency was quantified using immunoblotting.

### Spheroid formation assay

5,000 cells of HT29 and SW620 cells were inoculated in serum-free DMEM/F12 medium with 20 ng/mL epithelial growth factor (EGF) in 96-well round-bottom low-affinity plate (Nunc). Medium was supplemented with CL16 or Rhosin when spheroid has formed an opaque core. Morphology was recorded under a light microscope with 10X objective.

### Flow cytometry experiment on cell cycle of CRC cells treated with CL16

HCT116 cells were seeded onto 6-well plates (500,000 cells) and cultured overnight in complete medium at 37 °C with 5% CO₂. After treatment with DMSO control or CL16 at the indicated concentration for 24 h, cells were harvested by trypsin digestion, washed with PBS, and fixed in 70% cold ethanol on ice for 30 minutes. The cells were then centrifuged at 250g for 5 min and resuspended in staining buffer (PBS with 0.05% Triton X-100) containing propidium iodide (50 µg/mL) and RNase (100 µg/mL) for 30 min at 37°C in the dark. The cells were then filtered through a 40 µm strainer and analyzed by flow cytometry. Representative FACS plots were developed to illustrate the gating strategy in studying cell cycle. The NovoCyte Advanteon BVYG analyzer was used to acquire cell events, and the data were processed using NovoExpress software.

### Apoptosis assay by confocal fluorescence microscopy

HCT116 cells were seeded onto a poly-D-lysine coated Ibidi 8-well chamber slide (20,000 cells) and cultured overnight in complete medium at 37 °C with 5% CO₂. The cells were treated with DMSO control, Rhosin or CL16 at the indicated concentration along with 5µM of CellEvent^{™} Caspase-3/7 green in serum-free medium for 24 h. Prior to imaging, the medium was exchanged for Hanks' Balanced Salt Solution (HBSS). Confocal fluorescence microscopy was performed using a Zeiss laser scanning microscope 880 with a 20× water-immersion objective lens and ZEN 2.3 (Black Version) software (Carl Zeiss). CellEvent^{™} Caspase-3/7 green was excited by a 488 nm diode laser and emission was collected on a META detector between 490 and 525 nm. Image analysis was performed on ImageJ where the number of cells expressing fluorescence within the field was counted against the total number of cells to report the percentage of apoptotic cells. The average percentage was calculated from 3 separate images from the same well.

### Mouse xenograft model

7-week-old mice with weight around 20 g were inoculated subcutaneously with HCT116 (2×10⁶ cells per mouse) or HT29 (3×10⁶ cells per mouse) cells, in serum-free RMPI or Matrigel in PBS respectively. CL16 was dissolved in PET (60% PEG400, 30% Ethanol, 10% Tween80, v/v) with sonication until the solution turns clear. PET vehicle or CL16 in PET was then mixed with PBS in 1: 1 ratio (v/v) to prepare the solution for injection. CL16 was injected intra-peritoneally (200 µL) every 2 days after tumor has reached 100 mm³ in size, and until humane endpoint has reached. The tumor volume and body weight of the nude mice were measured throughout the treatment. The tumor volume was calculated by the equation: Volume (mm³) = l×w²/2, where l and w were the length and width of the tumor. At the end of the treatment, the mice were sacrificed and the tumor weight was measured. The tumor tissues were then processed into PEFF blocks.

### GelABPP

HCT116 and SW620 cells were plated onto 6-well plate at a density of 5×10⁵ cells per well overnight. Then the cells were treated with CL16 at indicated concentration for 2 hours, followed by treatment with CL16-alkyne (1 µM) for 2 hours. After treatment, the cells were washed with PBS and lysed by probe sonication. Cell debris were removed by centrifugation at 5,000g for 10 min at 4° C. 50 µL of normalized protein lysates (2mg/mL) were labeled with azide-fluor 545 by copper-catalyzed azide-alkyne cycloaddition at room temperature in dark for an hour. Then, the reaction was quenched by boiling in sampling buffer and samples were subjected to SDS-PAGE to separate proteins band for measuring in-gel fluorescence. After imaging, the gel was washed with MilliQ and incubated with Coomassie Blue stain with gentle shaking.

### Stable HT29 cells expressing RhoA C16A

HT29 cells with RhoA C16A knock-in were prepared by BrainVTA using the CRISPR-Cas9 technique. Briefly, HT29 cells were transfected with RhoA construct with mutated loci in CRISPR/Cas9 vector (U6-sa-sgRNA) using PEI. Cells were subsequently selected with 2 µg/mL puromycin and 800 µg/mL neomycin for 2 weeks. Monoclonal cells were then selected, propagated and subjected to Sanger sequencing to validate the homozygous C16A amino acid substitution (GCT to TGT) in RhoA gene.

### Physical Measurements and Instrumentation

¹H NMR and ¹³C{¹H} spectra were collected on Bruker AVANCE NEO 600 MHz spectrometer at the Department of Chemistry or Department of Microbiology at the University of Hong Kong. All chemical shifts are reported in the standard δ notation of parts per million relative to residual solvent peak as an internal reference. Splitting patterns are indicated as follows: br, broad; s, singlet; d, doublet; t, triplet; m, multiplet; dd, doublet of doublets; tt, triplet of triplets. UV spectra of chemicals were acquired by ACQUITY UPLC I-Class PLUS System (Waters) whereas high resolution mass spectrometry by TripleTOF^{™} 6600 (Sciex) offered by the Biomedical Technology Support Centre from Hong Kong Science Park. UV-Vis absorption and fluorescence from microplates were recorded on Perkin Elmer Victor 3 (Molecular Devices) or CLARIOstar Plus (BMG Labtech). In-gel fluorescence images were recorded on ChemiDoc MP Gel Imaging system (Bio-Rad). Confocal microscopic images were recorded on a Zeiss laser scanning microscope 880 with a 20× air objective lens using ZEN 2.3 (Black Version) software (Carl Zeiss) with 3× magnification zoom-in. Flow cytometry experiments were performed on NovoCyte Advanteon BVYG analyzer (Agilent).

### LC-MS/MS experiment on HCT116 cells treated with CL16

HCT116 cells were treated with DMSO or CL16 (25 or 50 µM) in complete medium for 4 hours, washed with PBS, harvested, and lysed in PBS by sonication. The protein amount of the samples was determined by BCA assay and normalized. The cell lysates in PBS were reduced by TCEP (1 mM) at 65 °C for 30 min, followed by alkylation with iodoacetamide (18 mM) at 37 °C for 30 min in the dark. The mixtures were added with prechilled acetone (600 µL) for protein precipitation at -20 °C overnight. The samples were centrifuged at 8000g for 10 min at 4 °C. The supernatant was discarded and the dried pellet was resuspended in 2M urea in PBS (100 µL). Sequencing grade trypsin (Promega #V5111) were added to the samples for overnight digestion at 37 °C with constant shaking. The solutions were then acidified with formic acid (FA; final concentration 5%), centrifuged at 13,200 rpm for 30 min at 4 °C. The supernatant was collected and stored at - 80 °C before MS analysis.

The peptide solutions were dried and desalted by C18 Stage tips, and the peptides were loaded onto an Aurora C18 UHPLC column (75 µm i.d. × 25 cm length × 1.6 µm particle size (IonOpticks Australia) coupled to timsTOF Pro mass spectrometer (Bruker). Chromatographic separation was carried out using buffer A (98:2 water:acetonitrile, 0.1% formic acid) and B (acetonitrile, 0.1% formic acid) with the gradient from 2% to 30% buffer B at a flow rate of 300 nL/min over 90 min, followed by an increase from 30% to 50% buffer B over 25 min, an isocratic gradient of 95% buffer B over 8.5 min, a decrease of buffer B to 2% in 0.5 min and then an isocratic gradient of 2% buffer B for 5.5 min. MS data was collected over a m/z range of 100 to 1700, and MS/MS range of 100 to 1700. During MS/MS data collection, each TIMS cycle was 1.1 s and included 1 MS + an average of 10 PASEF MS/MS scans.

Raw files were searched using MaxQuant (v2.0.3.0) with Uniprot human database (UP000005640). The search was specified to tryptic digestion (allowed up to 3 cleavages). Cysteine carbamidomethylation (+57.02146) was selected as fixed modification, while N-terminal acetylation (+42.01057), methionine oxidation (+15.99491) and CL16-cysteine adduct (+235.06333) were set as variable modifications. Peptide false discovery rate (FDR) was set to be 1%. Modified peptides with spectral count less than 2, PEP larger than 0.01, and lower spectral counts at 50 µM of CL16 than 25 µM were filtered out.

### LC-MS/MS for CL16 target identification using CL16-alkyne

HCT116 cells were treated with DMSO or CL16 (25 µM) in complete medium for 2 hours. The cells were washed with PBS, followed by incubation with CL16-alkyne (50 µM) for 2 hours. After treatment, the cells were washed with PBS and lysed by probe sonication in PBS. Cell debris were removed by centrifugation at 5,000g for 10 min at 4 °C. After conducting a protein assay using BCA and protein normalization, the cell lysates (4 mg/mL, 2 mL) were mixed with a master-mix solution for CuAAC reaction. The master-mix solution contained CuSO₄, TBTA, DTB-PEG-azide, and TECP in final concentrations of 1 mM, 100 µM, 100 µM, and 1 mM respectively. After incubating the samples with the master mix solution for 1 hour at room temperature with vortexing, prechilled acetone (12 mL) was added to the samples, and the proteins were allowed to precipitate at -20 °C overnight. The samples were centrifuged at 5,000g at 4 °C for 10 min, and the supernatant was discarded. The protein pellets were then re-dispersed in 1.2% SDS in PBS (w/v) and heated at 80 °C for 5 min. Any insoluble solids were discarded by centrifugation at 6,500g for 5 min, and the supernatant was transferred to a PBS solution containing Pierce^{™} Streptavidin Agarose beads (Thermo Scientific #20349) with a final concentration of 0.2% SDS (w/v). The samples and beads were incubated at 4 °C with rotation overnight. The beads were then washed with PBS and water, and re-dispersed in 6M urea in PBS. The samples were reduced by TCEP (1 mM) at 65 °C for 20 min, followed by alkylation with iodoacetamide (18 mM) at 37 °C for 30 min in the dark. The beads were then spun down by centrifugation at 1,400g for 2 min, washed with PBS, and re-suspended in 2M urea in PBS. The proteins on the beads were digested by sequencing grade trypsin (Promega) at 37 °C overnight. The solution mixtures were then transferred to a BioSpin column (Bio-rad), and the tryptic digested peptides were collected by centrifugation at 1,400g for 2 min. The beads were washed with PBS (100 µL) twice. The washing was collected by centrifugation at 1,400g for 2 min and combined with the tryptic digested peptide solution. The combined solution was dried and desalted by C18 Stage tips. Finally, the peptides were sent for LC-MS/MS analysis on timsTOF Pro mass spectrometer and analyzed using the aforementioned protocol for the LC-MS/MS experiment.

Raw files were searched using MSFragger (v3.7) with Uniprot human database (UP000005640). The search was specified to tryptic digestion (allowed up to 3 cleavages). Cysteine carbamidomethylation (+57.02146) was selected as fixed modification, while N-terminal acetylation (+42.01057) and methionine oxidation (+15.99491) were set as variable modifications. Peptide false discovery rate (FDR) was set to be 1%. For the proteins with zero LFQ intensity for all the 3 replicate runs in the control group and non-zero LFQ intensity for all the 3 runs in the treated group, they were assigned for LFQ ratio of 20. To eliminate false positives, proteins with average intensity smaller than 100,000, combined spectral counts less than 10, or protein probability less than 1 were filtered.

### Synthesis of exemplary RhoA inhibitors

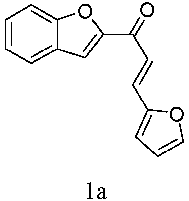

1a was synthesized according to the reported literature (Wang, X.-L. et al. European Journal of Medicinal Chemistry 45, 4631-4639 (2010)).

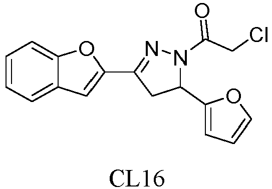

Hydrazine (2 equiv.) was added to 1a (1 equiv.) in ethanol and heat under reflux for 3-4 hours. Any organic volatile was evaporated under reduced pressure, then the residue was extracted with dichloromethane. The dichloromethane layer was dried over MgSO₄, filtered and evaporated to dryness. Then the intermediate was dissolved in dry dichloromethane. Chloroacetyl chloride (1.2 equiv.) and triethylamine (2 equiv.) were added dropwise slowly on ice. The reaction was allowed to proceed overnight. Any volatile organic solvent was evaporated under reduced pressure, and the crude product was purified by column chromatography on silica gel using hexane/ethyl acetate (80:20, v/v) as eluent to afford CL16. ¹H NMR (600 MHz, Chloroform-d) δ 7.66 - 7.54 (m, 2H), 7.44 - 7.39 (m, 1H), 7.34 - 7.27 (m, 2H), 7.16 (s, 1H), 6.45 - 6.29 (m, 2H), 5.73 (dd, J = 11.7, 4.8 Hz, 1H), 4.65 - 4.53 (m, 2H), 3.70 - 3.53 (m, 2H). ESI-MS (m/z): [M+H]⁺ 329.3. ¹³C NMR (151 MHz, Chloroform-d) δ 164.14, 155.63, 150.46, 147.68, 147.18, 142.37, 127.68, 126.71, 123.74, 121.87, 111.80, 110.73, 109.72, 108.73, 53.82, 42.35, 37.72. HRMS (ESI+): m/z calc for C₁₇H₁₄ClN₂O₃ [M+H]+ 329.0693; observed 329.0698.

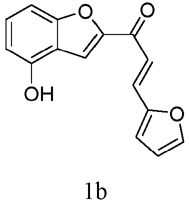

1b was synthesized according to the same procedure as 1a except using 2-acetyl-7-hydroxybenzofuran instead of 2-acetylbenzofuran. The crude product was purified by column chromatography on silica gel using hexane/ ethyl acetate (60:40, v/v) as eluent, yielding 1b as a yellow solid. ¹H NMR (600 MHz, Chloroform-*d*) δ 7.70 (d, *J =* 15.3 Hz, 1H), 7.65 (s, 1H), 7.57 (d, *J =* 1.8 Hz, 1H), 7.43 (d, *J =* 15.3 Hz, 1H), 7.28 (dd, *J =* 7.9, 1.0 Hz, 2H), 7.21 (t, *J =* 7.8 Hz, 1H), 7.03 (ddd, *J =* 7.0, 5.9, 1.1 Hz, 1H), 6.79 (d, *J =* 3.4 Hz, 1H), 6.55 (dd, *J =* 3.4, 1.8 Hz, 1H). ESI-MS (m/z): [M+H]⁺ 255.1.

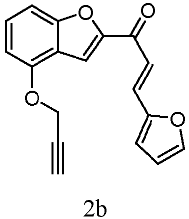

2b (1.0 equiv.) was dissolved in acetonitrile. Propargyl bromide (1.5 equiv.) and K₂CO₃ (2.5 equiv.) were added to the solution mixture and heated under reflux overnight. The reaction mixture was quenched by adding water and dried under reduced pressure, then the aqueous layer was extracted with dichloromethane. The organic layer was dried by MgSO₄, filtered and evaporated under reduced pressure. The crude product was purified by column chromatography on silica gel using hexane/ethyl acetate (80:20, v/v) as eluent to afford the compound 2b. ¹H NMR (600 MHz, Chloroform-*d*) δ 7.70 (d, *J =* 15.4 Hz, 1H), 7.62 (s, 1H), 7.57 (s, 1H), 7.52 (d, *J =* 15.4 Hz, 1H), 7.35 (d, *J =* 7.4 Hz, 1H), 7.22-7.27 (m, 1H), 7.11 (d, *J =* 7.9 Hz, 1H), 6.78 (d, *J =* 3.4 Hz, 1H), 6.53-6.54 (m, 1H), 4.97 (d, *J =* 2.4 Hz, 2H), 2.58 (t, *J =* 2.4 Hz, 1H). ESI-MS (m/z): [M+H]⁺ 293.3.

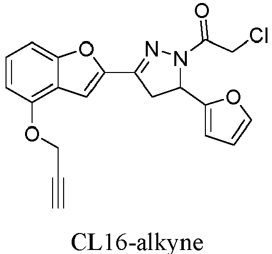

Hydrazine (2 equiv.) was added to 2b (1 equiv.) in ethanol and heat under reflux for 3-4 hours. Any organic volatile was evaporated under reduced pressure, then the residue was extracted with dichloromethane. The dichloromethane layer was dried over MgSO₄, filtered and evaporated to dryness. Then the intermediate was dissolved in dry dichloromethane. Chloroacetyl chloride (1.2 equiv.) and triethylamine (2 equiv.) were added dropwise slowly on ice. The reaction was allowed to proceed overnight. Any volatile organic solvent was evaporated under reduced pressure, and the crude product can be purified by column chromatography on silica gel using hexane/ethyl acetate (80:20, v/v) as eluent to afford CL16-alkyne. ¹H NMR (600 MHz, Chloroform-*d*) δ 7.30 (d, *J =* 1.8 Hz, 1H), 7.27 (d, *J =* 7.4 Hz, 1H), 7.21 (d, *J =* 7.9 Hz, 1H), 7.16 (d, *J =* 3.6 Hz, 1H), 7.04 (dd, *J =* 7.9, 1.1 Hz, 1H), 6.40 (d, *J =* 3.3 Hz, 1H), 6.32 (dd, *J =* 3.3, 1.8 Hz, 1H), 5.71 (dd, *J =* 11.6, 4.9 Hz, 1H), 4.93 (d, *J =* 2.4 Hz, 2H), 4.59 (q, *J =* 14.3 Hz, 2H), 3.66 (dd, *J =* 17.6, 11.7 Hz, 1H), 3.57 (dd, *J =* 17.6, 4.9 Hz, 1H), 2.57 (t, *J =* 2.4 Hz, 1H). ¹³C NMR (151 MHz, Chloroform-*d*) δ 171.21, 164.20, 164.18, 150.47, 148.04, 147.88, 147.26, 147.16, 145.18, 144.46, 143.23, 142.34, 132.89, 130.03, 129.72, 129.55, 124.36, 124.29, 118.35, 116.32, 114.98, 114.83, 114.13, 110.69, 110.42, 109.98, 109.54, 109.48, 109.40, 108.65, 108.62, 78.06, 76.31, 76.22, 69.94, 60.41, 56.76, 53.83, 42.35, 42.30, 37.85, 37.82, 29.69, 21.05, 14.19. HRMS (ESI+): m/z calc for C₂₀H₁₆ClN₂O₄ [M+H]+ 383.0799; observed 383.0796.

### Results

### Expressions of RhoA, GEFs and GAPs correlate with CRC development and/or patient survival

RhoA is known to be a molecular switch governing cytoskeleton organization and cell movement, thus closely associated with cancer invasion and metastasis. It exists in the active form when bound with GTP, allowing enhanced interactions with downstream effector proteins for signal transduction. On the other hand, it turns into the inactive form when bound with GDP. The equilibrium between the GDP-bound and GTP-bound states is mainly regulated by two protein families, guanine exchange factors (GEFs) and GTPase activating proteins (GAPs). GEFs facilitate the exchange of GDP to GTP, thereby activating RhoA. GAPs catalyze the hydrolysis of GTP on RhoA to GDP, leading to its inactivation (**Figure 15**). Therefore, the cellular activity of RhoA is not solely dependent on its expression level, but rather on the crucial level of the active GTP-bound form of RhoA.

Higher RhoA expressions were found in tumor tissues of patients with colon adenocarcinoma (COAD) and rectum adenocarcinoma (READ), as compared to the normal tissues, based on the analysis of the dataset in The Cancer Genome Atlas (TCGA) and Genotype-Tissue Expression (GTEx; **Figure 8A**). Although their was not a correlation between RhoA expression and patient survival (**Figure 8B**), higher expressions of two GEF proteins, ARHGEF25 and AKAP13, were found to associate with poor patient survival (**Figures 8C-8D**). On the other hand, lower GAP levels (ARHGAP26 and ARHGAP35) favored patient survival (**Figures 8E-8F**). Increases in ARHGEF23 and AKAP13 levels with more advanced tumor stage in the COAD and READ patients with statistical significance was observed (**Figures 8G-8H**). It is noteworthy that AKAP13 is a RhoA GTPase-specific GEF and has no guanine nucleotide exchange activity on Cdc42, Rac and Ras. In addition, CRC patients with stage IV tumors are more enriched in RhoA signaling pathway than patients with early-stage tumors (**Figures 8I-8J**), suggesting an elevation of RhoA activity in advanced-stage tumors. Together with the enrichment of Rho pathway in CRC patients with higher metastatic potential as indicated by high epithelial-mesenchymal transition (EMT) score (**Figures 8K-8L**), all these results indicate a correlation between RhoA activity and CRC development and aggressiveness.

*Gel-based ABPP experiments to identify CL16 as the lead compound with strong and specific binding with RhoA in vitro.*

CL16 was identified for its strong and specific binding with RhoA in covalent ligand screening experiment by ABPP. ABPP is known as a powerful platform for covalent ligand screening, particularly for the identification of lead compounds targeting new hotspots for therapy. Competitive gel-based activity-based protein profiling (ABPP) experiment was performed on purified RhoA protein for screening its interactions with a library of cysteine-reactive covalent ligands with acrylamide or chloroacetamide warheads. RhoA protein for the first screening and on Rac1 and Cdc42 for the second screening to identify lead compounds with specific binding onto RhoA Cys16 . RhoA protein was pre-treated with covalent ligands or DMSO control, followed by incubation with iodoacetamide-rhodamine for cysteine-labeling. The reaction mixture was then resolved by SDS-PAGE and in-gel fluorescence was imaged (**Figure 1A**). RhoA-binding leads were identified by abolition in fluorescent signal or shifts in protein bands in silver stain due to covalent modifications. Since Cys16 is the only unique cysteine on RhoA (**Figure. 9**) and RhoA, Rac1 and Cdc42 share similar protein structures, compounds showing bindings with RhoA but not Rac1 and Cdc42 should target RhoA Cys16. Dose-dependent experiments revealed strong binding affinities of CL16 toward RhoA, with notable IC₅₀ of the competitive binding down to 440 nM (**Figures 1B-1D**). There was no observable binding of CL16 with other GTPases, such as Rac1 and Cdc42. Therefore, CL16 was identified as the lead compound for its strong and specific binding with RhoA.

For the analysis of the 1^{st} screening result, covalent ligands that show lower than 30% of the intensity ratio and retain at least 70% intensity in the silver stain as compared to the DMSO control were considered the lead compounds.

In the first screening experiment with RhoA protein 6 lead compounds were found (**Figure 16**).

*LC-MS*/*MS experiment revealed covalent binding of CL16 onto Cysl6 of RhoA in HCT116 cells.*

A liquid chromatography coupled to tandem mass spectrometry (LC-MS/MS) experiment has been conducted on colorectal cancer HCT116 cells treated with CL16. The only covalent modification of RhoA by CL16 was found to be Cys16 (**Figure 2A**). Cys16 is a unique residue on RhoA, which is absent in Rac1 or Cdc42 (**Figure 2B**), thus can explain the high specificity of CL16 for RhoA over other Rho GTPases.

### Covalent docking showed that CL16 resided next to the nucleotide-binding pocket of RhoA.

Cys16 on RhoA is close to the nucleotide-binding pocket, which is important for the regulation of RhoA activity. Thus, the structural features of CL16 binding with RhoA was studied using covalent docking (**Figure 2C**). CL16 was found to reside next to the nucleotide-binding pocket, interacting with Phe30 and Lys118 as well as the Mg²⁺ cofactor ion. This showed that covalent targeting of RhoA by CL16 could interfere with the nucleotide binding onto RhoA.

*Covalent targeting of RhoA Cys16 by CL16 perturbed GDP-GTP exchange and RhoA-GEF interactions in vitro.*

Covalent docking of CL16 and RhoA protein (PDB: 1CC0) revealed π-π stacking, π-cation and hydrogen bonding interactions of CL16 with Thr19, Phe30 and Lys118 in the nucleotide binding pocket, as well as metal coordination and π-cation interactions with the Mg²⁺ cofactor ion (**Figure 2C**). Although Rho GTPases generally bind strongly with GTP, a covalent inhibitor has been reported to compete with GTP binding onto a genetically engineered KRAS GTPase through irreversible covalent binding, suggesting that covalent binding onto RhoA Cys16 from CL16 could interfere GTP loading and inhibit RhoA to some extent. This is supported by the fluorescence assay on MANT-GTP binding onto RhoA, where pre-incubation of RhoA with CL16 significantly slowed down the increase in fluorescence intensity, indicating that the CL16 binding prohibited GTP loading onto RhoA (**Figure 10A**). In addition, in the analysis of CL16 interactions with the co-crystal structure of RhoA and DH/PH domains of LARG (PDB: 1X86), it was found that CL16 interacts with both RhoA and LARG with the docking model of CL16 with covalent binding onto Cys16 of RhoA in the RhoA-LARG protein complex (PDB: 1X86). This suggests that CL16 binding could perturb RhoA-GEF interactions and hence GTP loading onto RhoA. Also, in line with this covalent docking model, fluorescence-based assay revealed a significant decrease in the kinetics of Dbs-mediated GTP binding onto RhoA with increasing concentrations of CL16 (**Figure 10B**). Co-immunoprecipitation experiments (i.e., a mixture of purified human GST-RhoA (10 µg) and HEK293T cell lysates containing ARHGEF1 or LARG-GFP (360 µg), incubated with CL16 or the reported RhoA inhibitor Rhosin) supported the disruption of RhoA interactions with ARHGEF1, LARG DH-PH domain, AKAP13 and ARHGAP4s by CL16 (**Figure 2D**).

*Prohibitions of RhoA activation in colorectal cancer cells incubated with CL16.*

GTP loading onto RhoA is critical for the protein activation and mediating downstream signals. In view of the inhibitions of GDP-to-GTP exchange of RhoA by CL16 *in vitro* as discussed above, effects of CL16 on RhoA biology in colorectal cancer cells were examined. In the serum starvation-re-stimulation experiment, HCT116 cells were grown in serum-free medium with CL16, Rhosin or solvent control for 24 h, followed by stimulation with 10% FBS for 10 min. The cells were then lysed and the RhoA activity was assayed by the level of RhoA-GTP, which is the active form of RhoA. In the solvent control, an increase in RhoA-GTP level was observed in the serum-stimulated cells, demonstrating an activation of RhoA signaling (**Figure 3A**). CL16 treatment led to much lower cellular RhoA-GTP levels after serum stimulation as compared to the solvent control, demonstrating a prohibition of RhoA activation by CL 16. Surprisingly, CL16 was found to be more effective than Rhosin on inhibition of RhoA activity, as shown by the lower RhoA-GTP level in cells treated with 10 µM of CL16 than those treated with 30 µM of Rhosin (**Figure 3A**). This demonstrated that CL16 is a more potent RhoA inhibitor than Rhosin in CRCs. A specific inhibition of RhoA activity in HT29 cells treated with CL16 with no significant changes in the activity of other Rho GTPases such as Rac1 and Cdc42 was observed, while Rhosin inhibited Cdc42 in HT29 cells at its working concentration for RhoA inhibition (30 µM). HT29 cells were incubated with solvent vehicle, CL16 or Rhosin in complete medium for 24 h. The cells were then washed, lysed and subjected to rhotekin pull-down for blotting RhoA-GTP, or PAK-PBD pull-down for blotting Rac1-GTP and Cdc42-GTP. Confocal fluorescence images from HCT116 and HT29 cells expressing FRET RhoA-biosensor in the serum starvation-restimulation experiment were taken.

The effects of CL16 on RhoA downstream signals in CRCs with serum starvation-restimulation were examined. HCT116 cells incubated with solvent control showed elevated levels of phosphorylated MLC, which is the downstream effector of RhoA, upon serum re-stimulation (**Figure 3B**). In contrast, CL16 treatment significantly eliminated the increases in phosphorylation of MLC.

To further validate the inhibitory effects of CL16 on RhoA, HCT116 and HT29 cells were transfected with a FRET-RhoA biosensor, where cells with elevated RhoA activity would show a higher FRET/mTFP1. Specifically, HCT116 cells transfected with FRET-RhoA biosensor were treated with CL16 or solvent in serum-free medium for 24 h, washed with PBS, re-stimulated with 10% FBS for 10 min, washed, and then imaged in HBSS by confocal fluorescence microscopy. CL16 treatment led to abolishment of the increase in FRET/mTFP1 from the transfected cells as found in the serum re-stimulation experiment (**Figure 3C**). In addition, HCT116 cells were serum-starved and treated with CL16 or Rhosin for 24 h, followed by 10% FBS re-stimulation for 10 min. The cells were then washed and fixed by 4% paraformaldehyde, washed with PBS and stained with Alexa Fluor 488-Phalloidin in PBS (1:20, v/v) at room temperature for 15 min. The cells were washed with PBS, incubated with Hoechst (8.2 µM) in PBS for 15 min, washed and then imaged in PBS by confocal fluorescence microscopy. The results showed a reduction of F-actin fibers in cells treated with CL16 was observed starting at 5 µM, as compared to the solvent control, in the serum re-stimulation experiment (imaging data not shown). Since RhoA regulates cytoskeletal dynamics and RhoA activation can induce the formation of contractile F-actin, the reduction of F-actin fibers by CL16 treatment supports its inhibitory roles on RhoA signaling.

### Anticancer and anti-metastatic effects of CL16 on CRC through RhoA inhibition.

As discussed above, overexpression/hyperactivity of RhoA associated with poor prognosis and aggressive behaviors of CRCs (**Figure 8A**). Thus, the anti-cancer and anti-metastatic properties of CL16 in CRCs were explored. First, it was confirmed that genetic knockdown of RhoA by siRNA slowed down colony formation and migration of CRCs by using staining (data not shown) and wound healing assay (**Figure 4A**). MTT assay was used to determine cell viability of CRCs and normal colon fibroblast CCD-18Co cells treated with **CL16** for 48 h (*n* = 3 different biological replicates/group). A much lower expression level of RhoA in normal colon fibroblast CCD-18Co cells was also determined, as compared to CRCs, by western blotting (data not shown). CL16 showed cytotoxicity effects in CRCs, and the IC₅₀ values were correlated with the RhoA level in the CRCs (**Figures 4B and 4C**). HT29 cells were transfected with siRNA (5 µM) using Dharmafect1 for 48 hours, and protein expressions were then investigated by immunoblotting. CL16 was much less toxic in normal CCD-18Co, which has lower RhoA level, demonstrating a good therapeutic window for using CL16 to treat CRCs. Further, CL16 showed good efficacy in reducing HT29 cell migration and invasion, as shown in the wound healing assay (**Figure 4D**) and transwell invasion assay (HT29 cells treated with **CL16** or Rhosin for 24 h; data not shown).

To further investigate the anticancer effects of CL16, HT29 and SW620 cells were inoculated as spheroids in low-affinity plate with 20 ng/mL EGF, and incubated with different concentrations of CL16 or Rhosin. CL16 treatment resulted in significant inhibitions in the growth of HT29 and SW620 spheroids, and was found to be more effective than Rhosin treatment as reflected by the lower working doses of CL16 to mediate similar anticancer effects (**Figures 4E** **and** **4F**). The low cytotoxic IC₅₀ values of CL16 in SW620 and HT29 spheroids (4.10 and 5.57 µM respectively; **Figures 5A** **and** **5B**) demonstrated good penetration of CL16 through tumor mass to mediate anticancer effects via RhoA inhibition, as well as its use in treating CRC *in vivo.*

The molecular mechanisms of anticancer effects from CL16 were investigated. Flow cytometry analysis on CL16-treated HCT116 cells revealed a significant increase in G₂ phase cell population (**Figure 11A**). Western blotting experiments showed an increase in p21 and p27 protein levels in HCT116, HT29 and SW620 cells after CL16 treatment, while no significant changes were observed in cyclin A2 and cyclin B1. Immunoblotting of cell cycle checkpoint proteins in HCT116 cells underwent CL16 treatment for 24 h. The effects of CL16 treatment on cell cycle checkpoint proteins were investigated with immunoblotting of the cell cycle checkpoint proteins in SW620 and HT29 cells after CL16 treatment for 24 h. CL16 treatment also mediated cell apoptosis, as found in the confocal fluorescence imaging experiment using Caspase-3/7 Green Detection Reagent (**Figure 11B**). Caspase-3/7 Green Detection Reagent was used to study apoptosis in HCT116 cells treated with Rhosin by confocal fluorescence imaging. Since p21 and p27 expression is regulated by Dial which is a downstream target of RhoA, the elevated levels of p21 and p27 are attributable to the downregulated RhoA-Dial signaling by CL16. This leads to inhibitory effects of p21 and p27 on CDK1-cyclin B complex, preventing CRC cells from progressing into mitosis and inducing G2 phase arrest that triggers apoptotic cell death (**Figure 17**).

### Molecular probe of CL16 and chemoproteomics experiments demonstrated RhoA Cysl6 as the primary target of CL16 in CRC.

To identify protein target and any off-targets of CL16 in CRCs, a molecular probe of CL16, CL16-alkyne (**Figure 6A**), was synthesized. CL16-alkyne contains an alkyne handle that allows functionalization through copper-catalyzed azide-alkyne cycloaddition (CuAAC) for studying CL16-protein binding. HCT116 cells transiently expressing FLAG-RhoA were pre-treated with solvent vehicle or CL16 for 2 h, followed by the incubation with CL16-alkyne in complete medium for another 2 h. The cells were then lysed and subjected to immunoprecipitation by anti-FLAG beads. The enriched proteins were reacted with rhodamine azide through copper-catalyzed azide-alkyne cycloaddition (CuAAC), boiled with sampling buffer and separated by SDS-PAGE. The protein bands were then visualized by their in-gel fluorescence or blotted for RhoA. The results confirmed the ability of CL16-alkyne to bind with RhoA by the incubation of the probe with FLAG-RhoA expressing HCT116 cells, as shown by the strong fluorescence from FLAG-RhoA protein after immunoprecipitation and CuAAC (**Figure 6B**). Notably, pre-incubation of the FLAG-RhoA expressing HCT116 cells with CL16 competed with the alkyne probe labeling (**Figure 6B**), supporting the target engagement of CL16 with RhoA. Next, CL16-alkyne was employed to examine any off-target binding from CL16 in CRCs. HCT116 cells were pre-treated with solvent vehicle or CL16 for 2 h, followed by an incubation with CL16-alkyne for another 2 h. The probe-labeled proteins were then installed with desthiobiotin through CuAAC, enriched, tryptic digested and profiled by LC-MS/MS (**Figures 12A** **and** **18**). Peptides with fold change (Control/CL16)>4 and p<0.05 (i.e. at least 75% occupancy and statistical significance) were considered as targets bound with CL16. RhoA was found to be one of the protein targets of CL16 (**Figure 12A**), and was the only protein identified in both the LC-MS/MS experiments on CL16-treated HCT116 cells and CL16-alkyne-probed HCT116 cells (**Figure 12B**), suggesting that RhoA is the primary target of CL16. Gel-based ABPP experiment also supported CL16 binding onto RhoA, with minimal off-target binding as other proteins showed much weaker fluorescence or no significant changes in fluorescence intensity in cells pretreated with CL16 as compared to the DMSO control (**Figure 6C**).The probe-labeled proteins were installed with rhodamine through CuAAC, and visualized by their in-gel fluorescence after SDS-PAGE. A protein band with strong fluorescence was found at ~22 kDa in the sample incubated with CL16-alkyne only, in line with the molecular weight of RhoA (**Figure 6C**). CL16 pre-treatment at 10 µM led to complete abolishment of the protein band, demonstrating strong binding of CL16 with this protein (**Figure 6C**). Other proteins showed much weaker fluorescence or no significant competitive binding from CL16 (**Figure 6C**), demonstrating minimal off-target binding of CL16 in HCT116 cells.

### CL16 exhibits biological activity in CRC through covalent targeting onto RhoA Cysl6.

The effects of CL16 in HT29 cells expressing RhoA WT protein and HT29 cells with RhoA C16A knock-in by CRISPR-Cas9 were investigated, respectively, to validate the molecular mechanism of RhoA inhibition and anti-cancer properties from CL16. CL16 treatment resulted in a significant decrease in the active RhoA-GTP level in the HT29 cells with RhoA WT, but not in the RhoA C16A knock-in cells. HT29 cells expressing RhoA WT or C16A protein were incubated with solvent vehicle, CL16 or Rhosin in complete medium for 24 h. The cells were then washed, lysed, subjected to rhotekin pull-down and immunoblotted. A decrease in cell migration in HT29 cells expressing RhoA WT after treatment with CL16 for 48 h was observed, while no inhibitory effects were observed in CL16-treated HT29 cells with C16A knock-in. Wound healing assay on HT29 cells expressing RhoA WT or C16A protein were incubated with solvent vehicle, CL16 or Rhosin in complete medium for 48 h (**Figures 19A** and **19B**). These results demonstrate that CL16 inhibited RhoA activity and mediated anti-cancer properties in CRC primarily through covalent targeting to RhoA Cys16.

### CL16 showed in vivo antitumor effects on CRC.

After confirming the anticancer properties of CL16 on CRC cells and RhoA Cys16 as its primary molecular target, the anticancer effect of CL16 *in vivo* was studied. In nude mice with CRC xenografts, CL16 significantly inhibited tumor growth of HCT116 xenograft (**Figure 7A**), HT29 xenograft (**Figure 7B**), and SW620 xenograft (**Figures 7D and 7E**), at dosage of 10mg/kg, as compared to vehicle control. Body weight of treated mouse was monitored throughout the experiment and there was no observable difference between treated and untreated groups, indicating minimal toxicity of CL16 (**Figures 7C** **and** **7F**). Moreover, immunohistochemistry staining of H&E and Ki67 on tumor tissues of the solvent- or CL16-treated mice showed reduction in Ki67 in treated group (**Figures 14A-14D**), demonstrating decrease in proliferating cancer cells upon CL16 treatment. The elevated cleaved caspase-3 level in the tumor tissues from CL16-treated mice suggested the induction of apoptosis in CRC cells, highlighting the effective killing of cancer cells *in vivo* by CL16. H&E staining revealed no observable damages on major organs in the mice treated with CL16, further supporting the low systemic toxicity associated with CL16 treatment.

Antitumor effects of CL16 in an immunocompetent, orthotopic mouse CRC model, which was established by the inoculation of mouse CRC cells, SL4, intracecally were examined (**Figure 20**). Before the mouse experiment, it was confirmed that Cys16 is conserved on mouse RhoA protein and CL16 treatment significantly decreased the viability of SL4 (**Figure 13A**), similar to the findings in human CRC cells. In this immunocompetent mouse CRC model, it was observed that a significantly lower tumor weight after CL16 treatment (**Figure 13B**). H&E staining on tumor tissues from CL16-treated mice revealed nuclear atypia such as nuclear condensation and fragmentation, indicating the induction of apoptosis. This is supported by the immunohistochemistry of cleaved caspase-3, which showed an elevated level in the tumor tissues from the treated mice (**Figures 13C-13H**). Immunohistochemistry staining of CD3, CD4, CD8, Ki67 was conducted, with cleaved caspase-3 and VEGFR2 on tumor tissues from the vehicle- or CL16-treated mice. The decrease in Ki67 in tumor tissues was in line with the promising antitumor effects of CL16 (**Figures 13C-13H**). On the other hand, no significant changes in the body weight of the treated mice (**Figure 13I**) and no organ damages in the kidney tissue were found in the treated mice, suggesting a minimal toxicity from CL16 treatment. In addition, it was observed that tumor metastasis to liver in the control mice, while CL16 treatment prohibited the tumor metastasis. Together with the decrease in VEGFR2 level in the immunohistochemistry experiment (**Figures 13C-13H**), this illustrates the anti-metastatic properties of CL16 in vivo. Interestingly, increases in CD3, CD4 and CD8 were found in the immunohistochemistry of the tumor tissues from the CL16-treated mice (**Figures 13C-13H**), suggesting an increase in infiltration of cytotoxic T cells into the tumor microenvironment after CL16 treatment. To the best of our knowledge, this is the first reported study showing an increase in immunity through RhoA inhibition in CRC cells by a small molecule compound.

### RhoA inhibitory effects and anticancer properties of CL16 through covalent targeting of Cys16 of RhoA.

RhoA serves as an invaluable therapeutic target due to its role in modulating cancer migration and invasion. Clinical data also demonstrated that upregulation of RhoA in melanoma, breast cancer, and colorectal cancer samples. K-Ras covalent inhibitors that target small Rho GTPases have gained interest. A covalent inhibitor of RhoA targeting Cys107, DC-Rhoin, was reported. In contrast, the RhoA covalent inhibitors disclosed herein, such as CL16, target another Cys on RhoA to mediate different modes of action in RhoA inhibition as compared to DC-Rhoin. Through covalent binding onto Cys16, which locates near the nucleotide-binding pocket, CL16 resided next to the nucleotide-binding pocket in RhoA as found in covalent docking. This leads to significant decreases in the GDP-to-GTP self-exchange, as well as the LARG-mediated exchange, which are critical for RhoA activation, as demonstrated using the fluorescence-based exchange assays. This unique mechanism of action allows the disclosed RhoA covalent inhibitors, such as CL16, to show better efficacy in inhibition of cellular RhoA activity in CRC cells than Rhosin, as demonstrated using western blotting, FRET-RhoA biosensor imaging and immunofluorescence experiments.

Cys16 is an ideal target site of RhoA to covalent inhibitor for achieving high specificity over other small GTPases, as compared to other cysteines residues. This may be accounted for the uniqueness of Cys16 on RhoA according to sequence homology analysis, while Cys107, which is the binding site of reported DC-Rhoin, is conserved in Rac1 and Cdc42 as well. Covalent targeting of Cys16 to achieve a high specificity for RhoA is important for cancer therapy with minimal toxic side effects, especially that Rac1 and Cdc42 govern many essential cellular processes including cell proliferation, polarity and migration. The RhoA inhibitory effects and good RhoA specificity over other Rho GTPases allow the disclosed RhoA covalent inhibitors, such as CL16, to exhibit high cytotoxicity in CRC cells, with correlation with the RhoA expression/activity in the cancer cells, while it is much less toxic for normal colon fibroblast which has lower RhoA level. In line with its excellent anticancer and anti-metastatic properties in 2D cell culture, CL16 inhibited the growth of CRC spheroids, with better potency than the reported RhoA inhibitor, Rhosin. More importantly, the exemplary compound CL16 is the first RhoA covalent inhibitor that shows anti-tumor effects on mouse CRC xenografts *in vivo.* No observable toxicity was found from the treated mice, as shown by the body weight measurement and immunohistochemistry on the tissue sections of different organs. This *in vivo* data highlights that covalent targeting of RhoA with good specificity is an effective strategy for treating CRCs.

### References:

KR1020170141542
KR102224059
WO2015028929A1
CN114028368A
WO2019122981A1
Shang, X. et al. Chem Biol. 19, 699-710 (2012)
Sun, Z. et al. Advanced Science 7, 2000098 (2020)
Liu, H. et al. Cancers (Basel) 12, 33 (2019).
Yang, N.-Y. et al. Journal of Biological Chemistry 281, 32574-32586 (2006). Bass, A. J. et al. Nature 513, 202-209 (2014).
Kakiuchi, M. et al. Nat. Genet. 46, 583-587 (2014).
Yan, H. H. N. et al. Cell Stem Cell 23, 882-897.e11 (2018).
Wang, K. et al. Nat. Genet. 46, 573-582 (2014).
Zhang, H. et al. Cancer Discov. 10, 288-305 (2020).
Zhang, S. et al., Molecular Cancer Research 7, 570-580 (2009).
Liu, S. et al. Cancer Res. 69, 8742-8751 (2009).
Wang, W. et al. Cancer Res. 64, 8585-8594 (2004).
Kalpana, G. et al. Sci. Rep. 9, 16351 (2019).
Rodrigues, P. et al. Nat. Commun. 5, 5458 (2014).
JEONG, D. et al. Int. J. Oncol. 48, 714-722 (2016).
Schwartz, M. J. Cell Sci. 117, 5457-5458 (2004).
Zandvakili, I. et al. Oncogene 36, 3213-3222 (2017).
O'Connor, K. & Chen, M. Small GTPases 4, 141-147 (2013).
Huang, B. et al. Sci. Rep. 4, 6449 (2014).
Eitaki, M. et al. BMC Cancer 12, 469 (2012).
Cardama G.A. et al. Int. J. Oncol. 51, 1025-1034 (2017).
Ellenbroek, S. I. J. & Collard, J. G. Clin. Exp. Metastasis 24, 657-672 (2007). Campbell, H. et al. Nat. Commun. 9, 254 (2018).
Shang, X. et al. Chem. Biol. 19, 699-710 (2012).
Sun, Z. et al. Advanced Science 7, 2000098 (2020).
Zhu, Y. et al. Molecular Cancer Research 17, 1910-1919 (2019).
Barcelo, J. et al. Trends Cancer 9, 250-263 (2023).
Ozyigit, G. et al. Strahlentherapie und Onkologie 195, 882-893 (2019).
Wang, Q. et al. Neural Regen. Res. 17, 2623 (2022).
Ido, K. et al. PLoS One 15, e0230953 (2020).
Funakoshi, H. et al. Annals of Clinical Epidemiology 2, 20-13 (2020). Limzerwala, J. F. et al. Nat. Cancer 1, 1010-1024 (2020).
Tsubaki, M. et al. Biomedicines 9, 35 (2021).
Janes, M. R. et al. Cell 172, 578-589.e17 (2018).
Kwan, A. et al. Journal of Experimental & Clinical Cancer Research 41, 27 (2022). Lanman, B. A. et al. J. Med. Chem. 63, 52-65 (2020).
Singh, J. et al. Nat Rev Drug Discov 10, 307-317 (2011).
Sutanto, F. et al. RSC Med Chem 11, 876-884 (2020).
Sato, M. et al. ACS Med Chem Lett 11, 1137-1144 (2020).
Hossam, M. et al. Arch Pharm (Weinheim) 349, 573-593 (2016).
Schwartz, P. A. et al. PNAS 111, 173-178 (2014).
Ridley, A. J. & Hall, A. Cell 116, S23-S27 (2004).
Cravatt, B. F. Isr. J. Chem. 63, e2023000 (2023).
Speers, A. E. & Cravatt, B. F. Curr. Protoc. Chem. Biol. 1, 29-41 (2009).
Cravatt, B. F. et al. Annu. Rev. Biochem. 77, 383-414 (2008).
Kuljanin, M. et al. Nat. Biotechnol. 39, 630-641 (2021).
Boike, L. et al. Cell Chem. Biol. 28, 4-13.e17 (2021).
Wang, S. et al. Front Pharmacol. 9, (2018).
Ward, C. C. et al. ACS Chem. Biol. 12, 1478-1483 (2017).
Grossman, E. A. et al. Cell Chem. Biol. 24, 1368-1376.e4 (2017).
Roberts, A. M. et al. Curr. Opin. Biotechnol. 43, 25-33 (2017).
Bateman, L. A. et al. Chemical Communications 53, 7234-7237 (2017). Nobis, M. et al. Small GTPases 11, 240-247 (2020).
Pertz, O. et al. Nature 440, 1069-1072 (2006).
Song, S. et al. Asian J. Pharm. Sci. 14, 30-39 (2019).
Wang, X.-L. et al. European J. of Medicinal Chemistry 45, 4631-4639 (2010).

## Claims

1. A compound having a structure of: 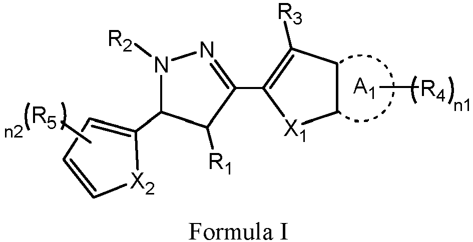 wherein:
(i) X₁ and X₂ are independently C(R₆)₂, O, S, or NR₆ and R₆ is hydrogen, a substituted or unsubstituted alkyl, a substituted or unsubstituted alkenyl, a substituted or unsubstituted alkynyl, an alkoxyl, or a carbonyl;
(ii) A₁ is a C₅-C₆ aromatic ring or a C₄-C₅ heteroaromatic ring;
(iii) n1 is an integer from 0 to 4;
(iv) n2 is an integer from 0 to 3;
(v) R₁, R₃, and R₅ are independently hydrogen, a substituted or unsubstituted alkyl, or a substituted or unsubstituted aryl;
(vi) R₂ is a cysteine-reactive group (such as haloacetamide, acrylamide, maleimide, vinyl sulfone and epoxide), which allows chemical reaction with cysteines on RhoA;
(vii) R₄ is hydrogen, an alkoxyl, a hydroxyl, a halogen, a substituted or unsubstituted alkynyl, a substituted or unsubstituted aryl, a carbonyl, an amino, or a haloalkyl; and
(viii) the substituents, when present, are independently unsubstituted alkyl, unsubstituted aryl, unsubstituted heteroaryl, unsubstituted polyaryl, unsubstituted heteropolyaryl, unsubstituted alkylaryl, unsubstituted cyclic, unsubstituted heterocyclic, halide, amino, amido, thiol, hydroxyl, cyano, nitro, carbonyl, or alkoxyl,
with the proviso that:
(i) the compound is not 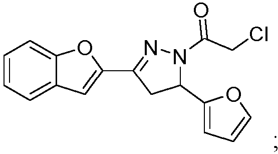 and
(ii) when X₁ is O, X₂ is S, R₁ and R₃-R₄ are hydrogen, and A₁ is a C₆ aromatic ring, then R₂ is not haloacetamide.

2. The compound of claim 1, having a structure of:

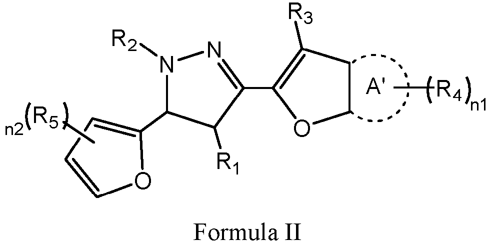

3. The compound of claim 1, having a structure of:

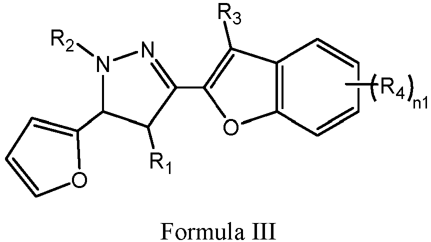

4. The compound of any one of claims 1-3, wherein n₁ is 0 or 1.

5. The compound of any one of claims 1-4, wherein R₁ is hydrogen.

6. The compound of any one of claims 1-5, wherein R₃ is hydrogen or a substituted or unsubstituted aryl, wherein the substituents, when present, are independently an unsubstituted alkyl, an unsubstituted aryl, or an unsubstituted alkyl aryl.

7. The compound of any one of claims 1-6, wherein R₂ is 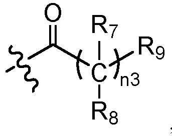 n3 is an integer from 0 to 6, R₇ and R₈ are independently hydrogen, an unsubstituted alkyl, or halogen, and R₉ is hydrogen or halogen.

8. The compound of any one of claims 1-7, wherein R₄ is hydrogen or 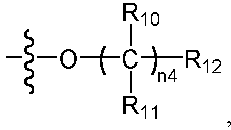 n4 is an integer from 0 to 6, R₁₀ and R₁₁ are independently hydrogen or halogen, and R₁₂ is hydrogen, halogen, amino, an unsubstituted alkynyl, aldehyde, or carboxylic acid;
optionally wherein R₁₀ and R₁₁ are hydrogen, and R₁₂ is an unsubstituted alkynyl.

9. The compound of any one of claims 1-8, having the structure of:

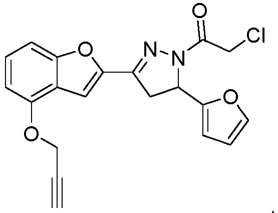

10. The compound of any one of claims 1-9, wherein the compound covalently binds onto RhoA, optionally wherein:
(i) the compound covalently binds onto Cys16 of RhoA; and/or
(ii) the compound inhibits RhoA activity in living cells.

11. A pharmaceutical composition for use in a method of treating a cancer in a subject, the pharmaceutical composition comprising a compound having a structure of: 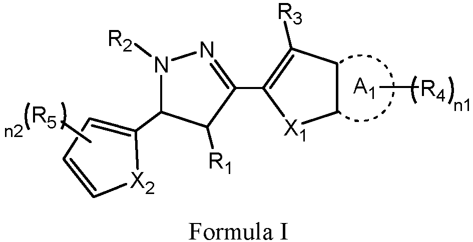 wherein:
(i) X₁ and X₂ are independently C(R₆)₂, O, S, or NR₆ and R₆ is hydrogen, a substituted or unsubstituted alkyl, a substituted or unsubstituted alkenyl, a substituted or unsubstituted alkynyl, an alkoxyl, or a carbonyl;
(ii) A₁ is a C₅-C₆ aromatic ring or a C₄-C₅ heteroaromatic ring;
(iii) n1 is an integer from 0 to 4;
(iv) n2 is an integer from 0 to 3;
(v) R₁, R₃, and R₅ are independently hydrogen, a substituted or unsubstituted alkyl, or a substituted or unsubstituted aryl;
(vi) R₂ is a cysteine-reactive group (such as haloacetamide, acrylamide, maleimide, vinyl sulfone and epoxide), which allows chemical reaction with cysteines on RhoA;
(vii) R₄ is hydrogen, an alkoxyl, a hydroxyl, a halogen, a substituted or unsubstituted alkynyl, a substituted or unsubstituted aryl, a carbonyl, an amino, or a haloalkyl; and
(viii) the substituents, when present, are independently unsubstituted alkyl, unsubstituted aryl, unsubstituted heteroaryl, unsubstituted polyaryl, unsubstituted heteropolyaryl, unsubstituted alkylaryl, unsubstituted cyclic, unsubstituted heterocyclic, halide, amino, amido, thiol, hydroxyl, cyano, nitro, carbonyl, or alkoxyl, and
optionally one or more pharmaceutically acceptable excipients,
wherein the compound is in an effective amount to ameliorate one or more symptoms associated with the cancer in the subject.

12. The pharmaceutical composition for use according to claim 11, the pharmaceutical composition further comprising a second active agent, optionally wherein the second active agent is an anticancer agent.

13. The pharmaceutical composition for use according to claim 11, wherein the compound in the pharmaceutical composition is in an amount ranging from about 0.1 µM to about 1 mM, from about 0.1 µM to about 500 µM, from about 0.1 µM to about 200 µM, from about 0.1 µM to about 100 µM, from about 0.1 µM to about 50 µM, or from about 0.1 µM to about 20 µM.

14. The pharmaceutical composition for use according to claim 11 the method comprising:
(i) administering the pharmaceutical composition to a subject in need thereof, wherein step (i) occurs one or more times.

15. The pharmaceutical composition for use according to claim 14 wherein:
(a) the pharmaceutical composition is administered by oral administration, intramuscular administration, intravenous administration, intraperitoneal administration, or subcutaneous administration, or a combination thereof;
(b) in step (i), the dosage of the compound administered is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject;
(c) the cancer is a solid cancer, optionally wherein following step (i) or all of step (i) (if step (i) occurs more than one time), the tumor volume and/or tumor weight is at least 10%, at least 20%, at least 30%, at least 40%, or at least 50% smaller/less than a control administered with the pharmaceutically acceptable excipient(s) only;
(d) the cancer is a colorectal cancer;
(e) following step (i) or all of step (i) (if step (i) occurs more than one time), there is no observable toxicity to the subject, as indicated by body vital measurements (e.g., body weight), standard hematology markers, and/or blood biochemical parameters compared to the control; and/or
(f) the method further comprising administering an active agent prior to, during, and/or subsequent to step (i), optionally wherein the active agent is an anticancer agent.
